# EUROPEAN PATENT APPLICATION

(11) **EP 4 342 378 A2**
(43) Date of publication of application: **27.03.2024**
(21) Application number: 23214855.1
(22) Date of filing: 22.12.2017
(51) Int. Cl.: A61B 5/157

(54) **SYSTEMS AND METHODS FOR PATIENT MONITORING USING AN HCP - SPECIFIC DEVICE**

(30) Priority: 27.12.2016 US 201662439342 P
(62) Divisional of application: 17889204.8
(71) Applicant: Dexcom, Inc., San Diego, CA 92121 (US)
(72) Inventor: BELLIVEAU, Scott M., San Diego, 92121 (US); BHAVARAJU, Naresh C., San Diego, 92121 (US); CHUM DEW, Darin Edward, San Diego, 92121 (US); COHEN, Eric, San Diego, 92121 (US); DAVIS, Anna Leigh, San Diego, 92121 (US); DERVAES, Mark, San Diego, 92121 (US); DUNN, Laura J., San Diego, 92121 (US); GRUCELA, Minda McDorman, San Diego, 92121 (US); HAMPAPURAM, Hari, San Diego, 92121 (US); JOHNSON, Matthew Lawrence, San Diego, 92121 (US); KAMATH, Apurv Ullas, San Diego, 92121 (US); KING, Steven David, San Diego, 92121 (US); KOEHLER, Katherine Yerre, San Diego, 92121 (US); MANDAPAKA, Aditya Sagar, San Diego, 92121 (US); MCDANIEL, Zebediah L., San Diego, 92121 (US); MIKAMI, Sumitaka, San Diego, 92121 (US); PAI, Subrai Girish, San Diego, 92121 (US); PELLOUCHOUD, Philip Mansiel, San Diego, 92121 (US); REICHERT, Stephen Alan, San Diego, 92121 (US); REIHMAN, Eli, San Diego, 92121 (US); SIMPSON, Peter C., San Diego, 92121 (US); SMITH, Brian Christopher, San Diego, 92121 (US); VANSLYKE, Stephen J., San Diego, 92121 (US); VAN TASSEL, Robert Patrick, San Diego, 92121 (US); WIGHTLIN, Matthew D., San Diego, 92121 (US); YANG, Richard C., San Diego, 92121 (US); AMIDEI, James Stephen, San Diego, 92121 (US); DERENZY, David, San Diego, 92121 (US); WEST, Benjamin Elrod, San Diego, 92121 (US); CRABTREE, Vincent, San Diego, 92121 (US); MOORE, Michael Levozier, San Diego, 92121 (US); BURNETTE, Douglas William, San Diego, 92121 (US); CONSTANTIN, Alexandra Elena, San Diego, 92121 (US); POLYTARIDIS, Nicholas, San Diego, 92121 (US); CAMBRA, Dana Charles, San Diego, 92121 (US); SHARMA, Abhishek, San Diego, 92121 (US); BRAUN, Kho, San Diego, 92121 (US); MCBRIDE, Patrick Wile, San Diego, 92121 (US)
(74) Representative: Dentons UK and Middle East LLP

(57) **Abstract**

Systems and methods disclosed provide ways for Health Care Professionals (HCPs) to be involved in initial patient system set up so that the data received is truly transformative, such that the patient not just understands what all the various numbers mean but also how the data can be used. For example, in one implementation, a CGM device is configured for use by a HCP, and includes a housing and a circuit configured to receive a signal from a transmitter coupled to an indwelling glucose sensor. A calibration module converts the received signal into clinical units. A user interface is provided that is configured to display a measured glucose concentration in the clinical units. The user interface is further configured to receive input data about a patient level, where the input data about the patient level causes the device to operate in a mode appropriate to the patient level.

## Description

### INCORPORATION BY REFERENCE TO RELATED APPLICATION

Any and all priority claims identified in the Application Data Sheet, or any correction thereto, are hereby incorporated by reference under 37 CFR 1.57. This application claims the benefit of U.S. Provisional Appl. No. 62/439,342 filed on December 27, 2016. The aforementioned application is incorporated by reference herein in its entirety, and is hereby expressly made a part of this specification.

### TECHNICAL FIELD

The present disclosure relates generally to continuous monitoring of analyte values received from an analyte sensor system. More particularly, the present disclosure is directed to systems, methods, apparatuses, and devices, for enabling participation of a health care provider in the set up and subsequent use by a patient of an analyte sensor system.

### BACKGROUND

Diabetes mellitus is a disorder in which the pancreas cannot create sufficient insulin (T-1 or insulin dependent) and/or in which insulin is not effective (Type 2 or non insulin dependent). In the diabetic state, the victim suffers from high blood sugar, which causes an array of physiological derangements (kidney failure, skin ulcers, or bleeding into the vitreous of the eye) associated with the deterioration of small blood vessels. A hypoglycemic reaction (low blood sugar) may be induced by an inadvertent overdose of insulin, or after a normal dose of insulin or glucose-lowering agent accompanied by extraordinary exercise or insufficient food intake.

Conventionally, a diabetic person carries a self-monitoring blood glucose (SMBG) monitor, which typically requires uncomfortable finger pricking methods. Due to the lack of comfort and convenience, a diabetic will normally only measure his or her glucose level two to four times per day. Unfortunately, these time intervals are spread so far apart that the diabetic will likely be alerted to a hyperglycemic or hypoglycemic condition too late, sometimes incurring dangerous side effects as a result. In fact, it is not only unlikely that a diabetic will take a timely SMBG value, but will not know if his blood glucose value is going up (higher) or down (lower), due to limitations of conventional methods.

Consequently, a variety of non-invasive, transdermal (e.g., transcutaneous) and/or implantable electrochemical sensors are being developed for continuously detecting and/or quantifying blood glucose values. Continuous glucose monitors have been increasing in popularity as an easy way to monitor glucose levels. In the past, patients sample their blood glucose levels several times throughout a day, such as in the morning, around lunch, and in the evening. The levels can be measured by taking a small blood sample of the patient and measuring the glucose levels with a test strip or glucose meter. This technique, however, has drawbacks because patients would prefer to not have to take a blood sample, and users do not know what their blood glucose levels are throughout the day between the samples.

One potentially dangerous timeframe is at night because a patient's glucose levels can fall dangerously low during sleep. As a result, continuous glucose monitors have gained popularity by providing a sensor that continuously measures glucose levels of a patient and transmits the measured glucose levels wirelessly to a display. This allows the patient or patient's caregiver to monitor the patient's glucose levels throughout the day and even set alarms for when glucose levels reach a predefined level or experience a defined change.

There are about 30 million diabetic patients in the U.S. 86 million people have prediabetes. However, 9 out 10 do not know they have prediabetes. Currently 1 out 3 people will develop Type-2 (T-2) diabetes in their lifetime. While T-2 can be prevented, however, once developed, patients need to manage the disease. According to Center for Disease Control (CDC), one way to manage any form of diabetes is to work very closely with a health care professional (HCP).

Patients and HCP's agree that diabetes is a difficult disease, managed with varying degrees of success. It is often hard to manage on a daily basis, and remaining vigilant in the face of disease can be physically and emotionally exhausting. In many cases success is dependent on the patient's motivation to incorporate lifestyle changes into daily routines.

This Background is provided to introduce a brief context for the Summary and Detailed Description that follow. This Background is not intended to be an aid in determining the scope of the claimed subject matter nor be viewed as limiting the claimed subject matter to implementations that solve any or all of the disadvantages or problems presented above.

### SUMMARY

Systems and methods according to present principles address many of the issues above, according to implementation. In more detail, systems and methods provide ways for HCPs to be involved in initial patient system set up so that the data received is truly transformative. The patient not just understands what all the various numbers mean but also how the data can be used. Better educated patients lead to significantly better health outcomes, and significantly lower healthcare costs.

In this regard it is noted initially that continuous glucose monitors wirelessly transmitted data relating to glucose levels to a dedicated display. The dedicated display is a medical device designed to display glucose levels, trending patterns, and other information for a user. However, with the increasing popularity of smart phones and software applications (apps) executing on smart phones, some users prefer to avoid having to carry a dedicated display. Instead, some users prefer to monitor their glucose levels using a dedicated software app executing on their mobile computing device, such as a smart phone, tablet or wearable device like a smartwatch or smartglasses.

T-2 patients could greatly benefit from CGM technology, but this market is difficult to address as it is highly heterogeneous in both patients as well as caregivers. For example, a person newly diagnosed is very different than a person who has attempted many prior therapeutic efforts, e.g., medications. New users are very different from older users. Newly-diagnosed patients may be highly motivated to control their disease, while patients who have tried and failed at control may be frustrated. In some cases, patients are poorly controlled for years before medications are advanced, and such patients often have a myriad of comorbidities and complications. Patients also have a wide range of levels of compliance to therapies.

Attitudes of patients and clinicians vary widely and their goals may be disparate. In many cases, there is a significant disconnect between physicians and patients with uncontrolled T-2 diabetes in their perceptions of diabetes control, including how they define control, obstacles to control, in the impact of uncontrolled T-2 diabetes. Compared to patients with uncontrolled T-2 diabetes, physicians generally have a more focused and clinical perspective of diabetes control, e.g., focusing on factors such as HbA1c value, frequency and severity of hypoglycemia, and medical complications due to diabetes. Patients with uncontrolled T-2 diabetes often have a broader perspective, considering day-to-day factors such as energy levels and how much they have to think about diabetes, in addition to clinical measures.

A first aspect is directed toward a continuous glucose monitoring device configured for use by a Health Care Professional (HCP), including: a housing; a circuit configured to receive a signal from a transmitter coupled to an indwelling glucose sensor; a calibration module configured to convert the received signal into clinical units; a user interface configured to display a measured glucose concentration in the clinical units; where the user interface is further configured to receive input data about a patient level; and where the input data about the patient level causes the device to operate in a mode appropriate to the patient level.

Implementations of the aspects and embodiments may include one or more of the following. The patient level may correspond to a user's technical skill level or to a user's type of diabetes. The user interface may be further configured to prompt an HCP to enter data about whether the user is a T-I diabetic, a T-II diabetic, or prediabetic. The device may further include a memory to store glucose concentration values in clinical units and further configured to store the input data. The device may further include an output circuit configured to transmit the stored glucose concentration values. The transmission may be configured to occur over a duration of less than five seconds. The transmission may be configured to occur using near field communications or Bluetooth low-energy. The transmitter may be configured to store measured glucose concentration values. The device may further include an output circuit configured to transmit the stored glucose concentration values. The transmission may be configured to occur using near field communications or Bluetooth low-energy.

A second aspect is directed toward a method of configuring a continuous glucose monitoring device, including: displaying a user interface on an HCP device; displaying a prompt on the user interface for an HCP to enter data about a patient; and causing a continuous glucose monitoring device, including an indwelling sensor and a signally-coupled transmitter, the transmitter in signal communication with the HCP device, to operate in a mode based on the entered data.

Implementations of the aspects and embodiments may include one or more of the following. The data may be about whether a patient is a T-1 diabetic, a T-II diabetic, or is prediabetic, or may also be about a user's technical skill level. The method may further include storing glucose concentration values in clinical units and the input data. The method may further include transmitting the stored glucose concentration values from the transmitter to the HCP device upon receipt of or triggering by an interrogation signal. The interrogation signal may be received by the transmitter from a near field communications device or a Bluetooth low-energy device coupled to the HCP device. The continuous glucose monitoring device may be configured to download an app, the app configured to control the continuous glucose monitoring.

The mode may be a blinded mode, such that the app running on the continuous glucose monitoring device is configured to receive and store but not display glucose concentration data. The app may be further configured to receive entered data corresponding to event data, where in the event data corresponds to medication data, meal data, or exercise data. The mode may be an unblinded mode, such that the continuous glucose monitoring device is configured to receive and store and display glucose concentration data. The mode may be configured to begin in a blinded mode and to switch to an unblinded mode at a predetermined time after a sensor session has begun. The mode may be configured to begin in a blinded mode and to switch to an unblinded mode upon the occurrence of a trigger event. The trigger event may correspond to a patient parameter meeting a predetermined threshold criterion. The mode may be configured to begin in a blinded mode and to switch to an unblinded mode upon the receiving of a trigger signal from an external device. The patient data may include a transmitter serial number, the transmitter serial number having a plurality of extensions selectable by the HCP, the extension selected to be appropriate for the patient, where the serial number extension identifies the mode in which the continuous glucose monitoring device is to operate. The mode may be a real-time blinded mode, such that the app running on the continuous glucose monitoring device is configured to receive and store but not display real time glucose concentration data, but is configured to display non-real-time historic glucose concentration data. The method may further include operating a diagnostic application on the HCP device, the diagnostic application allowing HCP's to view and set CGM parameters without changing the course of therapy. The parameters may include the sensor time remaining, sensor status, and current time in the session. The mode may be a blinded mode, and the transmitter may be configured to store data measured by the indwelling sensor.

A third aspect is directed toward a method of configuring a continuous glucose monitoring device for use by a patient, the configuring performed by an HCP, including: establishing a communication session associated with an HCP account between an HCP client device and a server; on a user interface associated with the HCP client device, prompting an HCP to enter patient data; also on the user interface associated with the client device, prompting an HCP to enter identifying data corresponding to a transmitter and/or sensor, the transmitter and/or sensor associated with the continuous glucose monitoring device; receiving the entered patient data and the sensor identifying data, and transmitting the entered patient data and the identifying data to the server for storage and association with a patient account.

Implementations of the aspects and embodiments may include one or more of the following. The identifying data may be identifying data about a transmitter. The method may further include receiving an output from the server in response to the transmitting entered patient data and the identifying data to the server, where the received output includes a code configured for use by a patient smart device to download an app for use in continuous glucose monitoring. The code may be received by way of email or text the method may further include transmitting set up information to the continuous glucose monitoring device. The transmitting may occur by way of near field communications or Bluetooth low-energy.

A fourth aspect is directed toward a method of configuring a continuous glucose monitoring device for use by a patient, the configuring performed by an HCP, including: establishing a first communication session associated with an HCP account between an HCP client device and a server; on a user interface associated with the HCP client device, prompting an HCP to enter patient data; establishing a second communication session between the HCP client device and a transmitter associated with the glucose monitoring device, whereby the transmitter and/or a sensor associated with the continuous glucose monitoring device may be identified to the HCP client device; receiving the entered patient data and the identifying data, and transmitting the entered patient data and the identifying data to the server for storage and association with a patient account.

Implementations of the aspects and embodiments may include one or more of the following. The second communication session may transmit transmitter identification data to the HCP client device.

The method may further include receiving an output from the server in response to the transmitting entered patient data and the sensor identifying data to the server, where the received output includes a code configured for use by a patient smart device to download an app for use in continuous glucose monitoring. The code may be received by way of email or text the method may further include transmitting set up information to the continuous glucose monitoring device. The transmitting may occur by way of near field communications or Bluetooth low-energy.

A fifth aspect is directed toward a reader device configured for use by an HCP, including: a housing; a first circuit configured to receive a first signal from a transmitter associated with an indwelling glucose sensor; a second circuit configured to transmit a second signal to a computing environment.

Implementations of the aspects and embodiments may include one or more of the following. The device may be configured to receive a first signal is configured to receive the first signal using a wired or wireless communications protocol. The device may be configured to receive a first signal is activated by an interrogation signal from the transmitter. The device may be configured to receive a first signal activates the transmitter using an interrogation signal. Following activation, the circuit may extract stored glucose concentration data from the transmitter. The communications protocol may be wireless and may be selected from the group consisting of: Bluetooth low-energy or near field communications. The device may further include a third circuit configured to measure one or more operating parameters of a transmitter and to provide an output based on the measured one or more operating parameters, whereby a status of operation of the transmitter may be determined.

A sixth aspect is directed toward a reader device configured for use by an HCP, the reader device configured to determine proper functioning of a transmitter associated with a continuous glucose monitor, including a circuit configured to measure one or more operating parameters of a transmitter and to provide an output based on the measured one or more operating parameters, whereby a status of operation of the transmitter is determined.

A seventh aspect is directed toward a method of determining proper startup of a transmitter, the transmitter associated with a continuous glucose monitor, including: on a transmitter, detecting insertion of a sensor; upon the detecting, causing the transmitter to transition from an inactive state to an active state, where in the active state the transmitter transmits signals encoded with data corresponding to readings received from the sensor.

Implementations of the aspects and embodiments may include one or more of the following. In the active state, the transmitter may transmit signals using Bluetooth low energy. The method may further include receiving the transmitted signals on an HCP device the method may further include receiving the transmitted signals on a patient device. The patient device may be a smart phone or a smart watch. The method may further include, upon the receipt of an acknowledgment signal by the transmitter from the patient device, transmitting a signal from the transmitter to an HCP device, whereby the HCP device is provided verification that a patient device is properly working with a patient sensor and transmitter. The method may further include collecting data from the transmitter over a duration of time from the transmitter as measured by the sensor. The extracting may include interrogating the transmitter using the HCP device the interrogating may be performed using near field communications or Bluetooth low-energy. The method may further include transmitting a signal from the transmitter, the signal encoded with data indicating that sensor insertion has occurred.

An eighth aspect is directed toward a method of activating a transmitter in a rapid fashion, the transmitter having been stored for a duration of time, the transmitter configured for physical engagement with an indwelling glucose sensor, the transmitter configured for transmitting signals representative of measured glucose concentration values to a mobile device, including: transmitting a wake-up command from an HCP device to the transmitter, where the wake-up command causes the transmitter to transition from an inactive state to an active state.

Implementations of the aspects and embodiments may include one or more of the following. The transmitting a wake-up command may be performed using near field communications or Bluetooth low-energy. The transmitting a wake-up command may be performed by communicating a signal to a wake-up pin on a processor at least in part operating the transmitter. The transmitting a wake-up command may be performed in response to the detection of a signal measured by the indwelling glucose sensor.

A ninth aspect is directed toward a method of activating a transmitter in a rapid fashion, the transmitter having been stored for a duration of time, the transmitter configured for physical engagement with an indwelling glucose sensor, the transmitter configured for transmitting signals representative of measured glucose concentration values to a mobile device, including: on the transmitter, detecting a signal from a connected sensor, if the detected signal is determined to have an amplitude that exceeds a predetermined threshold, causing the transmitter to transition from an inactive state to an active state.

A tenth aspect is directed toward a method of activating a transmitter in a rapid fashion, the transmitter having been stored for a duration of time, the transmitter configured for physical engagement with an indwelling glucose sensor, the transmitter configured for transmitting signals representative of measured glucose concentration values to a mobile device, including: causing a transmitter to be activated on a periodic basis, the transmitter when activated configured to receive signals from a glucose sensor; such that if the transmitter is activated and does not receive signals from a glucose sensor within a predetermined period of time, the transmitter is caused to be inactivated; and such that if the transmitter is activated and does receive signals from a glucose sensor within a predetermined period of time, the transmitter is caused to be permanently activated, whereby the transmitter may be periodically woken up to determine if the connection has been made to the indwelling glucose sensor.

In one implementation, the periodic basis may be between every five minutes and every fifteen minutes.

An eleventh aspect is directed toward a method of activating a transmitter in a rapid fashion, the transmitter configured for physical engagement with an indwelling glucose sensor, the transmitter configured for transmitting signals representative of measured glucose concentration values to a mobile device, including, upon activation of a transmitter, where a transmitter is coupled to a sensor and adhered to a patient by a patch, the transmitter receiving measurement signals from the sensor, the activation causing the transmitter to emit signals representative of and based on the measurement signals, rendering an indication of the activation on the transmitter, the sensor, or the patch, whereby a user may be notified of the activation without having to use other devices.

In an implementation, the rendered indication may be in a visual or audible form.

A twelfth aspect is directed toward a method of activating a transmitter in a rapid fashion, the transmitter configured for physical engagement with an indwelling glucose sensor, the transmitter configured for transmitting signals representative of measured glucose concentration values to a mobile device, including, upon activation of a transmitter, where a transmitter is coupled to a sensor and adhered to a patient by a patch, the transmitter receiving measurement signals from the sensor, the activation causing the transmitter to emit signals representative of and based on the measurement signals, transmitting a signal from the transmitter to an external device, the external device rendering an indication of the activation, whereby a user may be notified of the activation.

A thirteenth aspect is directed toward a method of activating a CGM system in a rapid fashion, the CGM system including a transmitter configured for physical engagement with an indwelling glucose sensor, the transmitter configured for transmitting signals representative of measured glucose concentration values to a patient mobile device, including: upon a wireless engagement of a patient mobile device and a transmitter, causing an app to download to the patient mobile device; and further upon the wireless engagement, and the download of the app, establishing a communication session between the patient mobile device and a server, the communication session associated with a user account.

Implementations of the aspects and embodiments may include one or more of the following. The wireless engagement may include an engagement using near field communications or Bluetooth low-energy. The app may be downloaded to the patient mobile device from the transmitter or from a server. The method may further include causing the transmitter to begin transmitting signals from the sensor, the signals indicative of measured glucose values measured by the sensor.

A fourteenth aspect is directed toward a system for activating a transmitter in a rapid fashion, the transmitter configured for physical engagement with an indwelling glucose sensor, the transmitter configured for transmitting signals representative of measured glucose concentration values to a mobile device, including: an applicator configured to install an indwelling sensor; where the applicator is further configured to install a transmitter in physical engagement with the indwelling sensor, where the transmitter has a switch which when activated causes the transmitter to enter an active state, where in the active state the transmitter receives signals from the sensor and transmits signals representative of measured glucose concentration values to a mobile device; and such that the switch is configured to be activated when the transmitter is caused to physically engage with the indwelling sensor.

A fifteenth aspect is directed toward a transmitter configured to be activated in a rapid fashion, the transmitter further configured for physical engagement with an indwelling glucose sensor, the transmitter configured for transmitting signals representative of measured glucose concentration values to a mobile device, including: a housing, the housing including means to physically engage a glucose sensor, the glucose sensor configured to at least partially indwell within a patient; a light sensor disposed within the housing, the light sensor optically exposed to an exterior of the housing through a window; a cover applied to the window, the cover configured to block exposure of the light sensor to light prior to use of the transmitter, such that, before use, a user removes the cover to expose the light sensor to light, the light sensor when activated causing the transmitter to enter an active state, whereby the transmitter may be woken up by removal of the cover.

In one implementation, the cover may adhere to the window.

A sixteenth aspect is directed toward a transmitter configured for physical engagement with an indwelling glucose sensor, the transmitter configured for transmitting signals representative of measured glucose concentration values to a mobile device, including: a housing, the housing including means to physically engage a glucose sensor, the glucose sensor configured to at least partially indwell within a patient; a circuit configured to receive timestamp information from an external source; and a memory configured to store the timestamp information from the external source.

Implementations of the aspects and embodiments may include one or more of the following. The memory may be further configured to associate the timestamp information with one or more received data packets, the received data packets associated with signals from the sensor. The circuit may be a near field communication circuit or a Bluetooth low-energy circuit. The circuit may be a Bluetooth low-energy circuit, and the circuit may be configured to periodically poll nearby Bluetooth devices for timestamp information. The circuit may be configured to receive timestamp information from an HCP device. The circuit may be configured to receive timestamp information from a patient mobile device. The circuit may further include a processor in signal communication with the memory, the processor configured to compensate for time drifts or time lags. The processor may be configured to compensate for time drifts or time lags by causing a periodic or occasional synchronization.

A seventeenth aspect is directed toward a kit for pairing a patient mobile device including a continuous glucose monitoring app with a transmitter, the transmitter configured for transmitting signals representative of measured glucose concentration values to a mobile device, including: a transmitter, the transmitter including means to physically engage a glucose sensor; and an identifying component including flexible electronics, the identifying component configured to be scanned by a patient mobile device, the patient mobile device receiving identification information about the transmitter, the identification information stored on the flexible electronics; such that upon receipt of the identification information the patient mobile device is configured to be paired with the transmitter, where the pairing allows the patient mobile device to receive signals transmitted by the transmitter, the signals representative of measured glucose concentration values.

Implementations of the aspects and embodiments may include one or more of the following. The identifying component may include a sticker and may further include calibration information. The pairing may be further between the glucose monitoring app and the transmitter, and may be performed on the basis of identified RSSI signal strength. The identifying component may be integrated as part of a sensor adhesive patch the app may be configured to verify that the sensor is within a proper operating range by determining whether received counts are within a predetermined range over a predetermined period of time.

An eighteenth aspect is directed toward a method for pairing a patient mobile device including a continuous glucose monitoring app with a transmitter, the transmitter configured for transmitting signals representative of measured glucose concentration values to a mobile device, including: receiving input on a user interface of a mobile device a desire an indication that the mobile device should be paired with a transmitter; detecting on an accelerometer within the mobile device a motion artifact; upon the detecting, transmitting a signal to cause the transmitter to be placed in a pairing mode; pairing the transmitter with the mobile device.

Implementations of the aspects and embodiments may include one or more of the following. The method may further include indicating on the user-interface a desired motion artifact, such as where the motion artifact includes a predetermined number of taps or a shaking motion for a predetermined threshold period of time.

A nineteenth aspect is directed toward a method for pairing a patient mobile device including a continuous glucose monitoring app with a transmitter, the transmitter configured for transmitting signals representative of measured glucose concentration values to a mobile device, including: receiving input on a user interface of a mobile device an indication that the mobile device is to be paired with a transmitter; detecting a signal from the transmitter; determining if an RSSI measurement of the detected signal exceeds a predetermined threshold; and causing a pairing to occur between the mobile device and the transmitter based on the determination that detected signal exceeded the predetermined threshold.

In one implementation, the determining step may include confirming an identity or availability of a user-identified transmitter based on selection criteria including ongoing detection of at least one of information related to the strength of the signal detected from the selected transmitter for a predetermined period of time and information related to the quality of the signal detected from the selected transmitter for a predetermined period of time.

A twentieth aspect is directed toward a reader device configured for use by an HCP, the reader device configured to set up a transmitter associated with a continuous glucose monitor, including a circuit configured to receive and transmit one or more operating parameters of a transmitter for display on a user interface of an HCP device, the reader device further configured to receive modified transmitter parameters from the user interface and to store the modified transmitter parameters on the transmitter.

A twenty-first aspect is directed toward a method of activating a transmitter, the transmitter having been employed in a sensor session, the transmitter configured for physical engagement with an indwelling glucose sensor, the transmitter configured for transmitting signals representative of measured glucose concentration values to a mobile device, the transmitter configured to, following a predetermined duration of a sensor session, transition from an active state to an inactive state, including: transmitting a wake-up command from an external device to the transmitter, where the wake-up command causes the transmitter to transition from an inactive state to an active state.

Implementations of the aspects and embodiments may include one or more of the following. The transmitting a wake-up command may be performed using near field communications or Bluetooth low-energy. The method may further include downloading data stored in the transmitter to the external device. The wake-up command may be transmitted using a near field communications protocol. The downloading may be performed using a near field communications protocol or a Bluetooth low-energy protocol. The external device may be the HCP device, and the HCP device may be an HCP reader or controller or an HCP smart phone. The external device may be a patient device such as the patient's smart phone.

A twenty-second aspect is directed toward a method of reducing noise in the signal transmissions between a transmitter and a mobile device, the transmitter configured for physical engagement with an indwelling glucose sensor, the transmitter configured for transmitting signals representative of measured glucose concentration values to the mobile device using a near field communications protocol, including: upon detection of a signal with energy greater than a predetermined threshold level on a near field communication circuit within a transmitter, setting a first flag and associating the first flag with corresponding data; on the detection of near field communications signaling between the transmitter and an external device, setting a second flag and associating the second flag with corresponding data; transmitting glucose concentration data from the transmitter to an external device; on the external device, storing the transmitted data; in calculations involving the transmitted data, adjusting, compensating for, or disregarding data for which both the first and second flags are set.

A twenty-third aspect is directed toward a method of reducing noise in the signal transmissions between a transmitter and a mobile device, the transmitter configured for physical engagement with an indwelling glucose sensor, the transmitter configured for transmitting signals representative of measured glucose concentration values to the mobile device using a near field communications protocol, including: upon detection of a signal with energy greater than a predetermined threshold level on a near field communication circuit with then a transmitter, setting a first flag and associating the first flag with corresponding data; on the detection of near field communications signaling between the transmitter and an external device, setting a second flag and associating the second flag with corresponding data, transmitting glucose concentration data from the transmitter to an external device; on the external device, storing the transmitted data; in calculations involving the transmitted data, associating data for which both the first and second flags are set with a lower weighting than data for which neither flag is set.

A twenty-fourth aspect is directed toward a method of reducing noise in the signal transmissions between a transmitter and a mobile device, the transmitter configured for physical engagement with an indwelling glucose sensor, the transmitter configured for transmitting signals representative of measured glucose concentration values to the mobile device using a near field communications protocol, including: upon detection of a signal with energy greater than a predetermined threshold level on a near field communication circuit with then a transmitter, setting a first flag and associating the first flag with corresponding data; on the detection of near field communications signaling between the transmitter and an external device, setting a second flag and associating the second flag with corresponding data; transmitting glucose concentration data from the transmitter to an external device; on the external device, storing the transmitted data; in calculations involving the transmitted data, disregarding data for which the first flag is set.

A twenty-fifth aspect is directed toward a transmitter configured for reducing noise in signal transmissions to a mobile device, including: a housing, the housing including means to physically engage a glucose sensor, the glucose sensor configured to at least partially indwell within a patient; a near field communication circuit disposed within the housing, the near field communication circuit configured for transmitting signals to an external device; a threshold detector coupled to the near field communication circuit, the threshold detector configured to detect a signal energy of energy captured by the near field communications circuit, such that if the threshold detector detects a signal of energy greater than a predetermined threshold, the threshold detector causes the near field communication circuit to become inactive.

In an implementation, the threshold detector may cause an antenna associated with the near field communication circuit to be disabled.

A twenty-sixth aspect is directed toward a transmitter configured for reducing noise in signal transmissions to a mobile device, including: a housing, the housing including means to physically engage a glucose sensor, the glucose sensor configured to at least partially indwell within a patient; a wireless communication circuit disposed within the housing, the wireless communication circuit configured for transmitting signals to an external device; a detector coupled to the wireless communication circuit, the detector configured to detect if a signal corresponding to a wake-up command corresponding to a wake-up process is being captured by the wireless communications circuit, such that if the detector detects a wake-up signal, the detector causes the wireless communication circuit to become inactive following completion of the wake-up process.

In an implementation, the wireless communication circuit may be a near field communications circuit.

A twenty-seventh aspect is directed toward a transmitter configured for enhanced battery life, the transmitter further configured for signal transmission to a mobile device, including: a housing, the housing including means to physically engage a glucose sensor, the glucose sensor configured to at least partially indwell within a patient; a wireless communication circuit disposed within the housing, the wireless communication circuit configured for transmitting signals to an external device; a battery, the battery configured to power the wireless communication circuit and to enable measurement by the glucose sensor; a power down circuit configured to cause the transmitter to transition from an active state to an inactive state following completion of a sensor session; a wake-up circuit configured to cause the transmitter to transition from an inactive state to an active state following completion of the sensor session such that data stored on the transmitter may be transmitted to the external device, the wake-up circuit in part activated by a wake-up pin, the wake-up circuit further configured to connect the battery to the wireless communications circuit in the active state, and such that the wake-up circuit is configured to, when transitioning to the inactive state, disable the wake-up pin.

A twenty-eighth aspect is directed toward a transmitter configured for enhanced battery life, the transmitter further configured for signal transmission to a mobile device, including: a housing, the housing including means to physically engage a glucose sensor, the glucose sensor configured to at least partially indwell within a patient; a wireless communication circuit disposed within the housing, the wireless communication circuit configured for transmitting signals to an external device; a battery, the battery configured to power the wireless communication circuit and to enable measurement by the glucose sensor; a power down circuit configured to cause the transmitter to transition from an active state to an inactive state following completion of a sensor session; a wake-up circuit configured to cause the transmitter to transition from an inactive state to an active state following completion of the sensor session such that data stored on the transmitter may be transmitted to the external device, the wake-up circuit in part activated by a wake-up pin, the wake-up circuit further configured to connect the battery to the wireless communications circuit in the active state, and such that the wake-up pin is hardened against EMI.

Implementations of the aspects and embodiments may include one or more of the following. The hardening may be by use of a strong pull up/down resistor, and/or further by use of a capacitor. The hardening may also be by way of mechanically shorting the wake-up pin to a disabled polarity.

A twenty-ninth aspect is directed toward a transmitter configured for enhanced battery life, the transmitter further configured for signal transmission to a mobile device, including: a housing, the housing including means to physically engage a glucose sensor, the glucose sensor configured to at least partially indwell within a patient; a wireless communication circuit disposed within the housing, the wireless communication circuit configured for transmitting signals to an external device; a battery, the battery configured to power the wireless communication circuit and to enable measurement by the glucose sensor; a power down circuit configured to cause the transmitter to transition from an active state to an inactive state following completion of a sensor session; a wake-up circuit configured to cause the transmitter to transition from an inactive state to an active state following completion of the sensor session such that data stored on the transmitter may be transmitted to the external device, the wake-up circuit in part activated by a wake-up pin, the wake-up circuit further configured to connect the battery to the wireless communications circuit in the active state, and such that the wake-up circuit is configured to, when transitioning to the inactive state, disable the wake-up circuit.

A thirtieth aspect is directed toward a transmitter configured for enhanced data storage, the transmitter further configured for signal transmission to a mobile device, including: a housing, the housing including means to physically engage a glucose sensor, the glucose sensor configured to at least partially indwell within a patient; a wireless communication circuit disposed within the housing, the wireless communication circuit configured for transmitting signals to an external device; a battery, the battery configured to power the wireless communication circuit and to enable measurement by the glucose sensor; a memory, the memory configured to store data representative of glucose values measured by the glucose sensor; and a processor configured to perform data processing on data stored in the memory, where the processor is configured to periodically compress the data stored in the memory, such that the data occupies less memory than before the compressing.

Implementations of the aspects and embodiments may include one or more of the following. The processor may be configured to compress the data to a minimum number of data points necessary to accurately illustrate a glycemic exposure of the patient. The data points may include those corresponding to maximums, minimums, and inflection points, along with corresponding abscissa time values. The processor may be configured to compress the data by eliminating data points representing stable values, where stable values are those within a range of stability. The processor may be configured to compress the data using lossy or lossless compression techniques. The lossy or lossless compression techniques may include one or more selected from the group consisting of Lempel-Ziv compression, Huffman encoding, or algorithmic encoding. The processor may be configured to compress the data by only storing data that change is more than a defined amount, where data in between such points is interpolated. The processor may be configured to compress the data by removing artifacts.

A thirty-first aspect is directed toward a method of operating a transmitter for enhanced data storage, the transmitter configured for transmitting signals from an indwelling glucose sensor to a mobile device, including: receiving signals over time from an indwelling glucose sensor; storing data representing the received signals in a memory; performing data processing on the stored data, where the data processing includes periodically or aperiodically compressing the data stored in the memory, such that the data occupies less memory than before the compressing.

Implementations of the aspects and embodiments may include one or more of the following. The compressing may compress the data to a minimum number of data points necessary to accurately illustrate a glycemic exposure of the patient. The data points may include those corresponding to maximums, minimums, and inflection points, along with corresponding abscissa time values. The compressing may compress the data by eliminating data points representing stable values, where stable values are those within a range of stability. The compressing may compress the data using lossy or lossless compression techniques the lossy or lossless compression techniques may include one or more selected from the group consisting of Lempel-Ziv compression, Huffman encoding, or algorithmic encoding. The compressing may compress the data by only storing data that change is more than a defined amount, where data in between such points is interpolated. The compressing may compress the data by removing artifacts.

A thirty-second aspect is directed toward a method of operating a transmitter for enhanced data accuracy, the transmitter configured for transmitting signals from an indwelling glucose sensor to a mobile device, including: receiving signals over time from an indwelling glucose sensor; storing data representing the received signals in a memory; performing data processing on the stored data, where the data processing includes periodically or aperiodically post-processing the data stored in the memory, such that the accuracy of the data, or accuracy of calculations based on the data, is enhanced.

Implementations of the aspects and embodiments may include one or more of the following. The periodically post -processing the data may include post -processing the data every twenty-four hours or every forty-eight hours. The periodically post -processing the data may include periodically smoothing the data.

A thirty-third aspect is directed toward a method of operating a continuous glucose monitor, including: receiving signals at a transmitter from an indwelling glucose sensor; receiving external signals at the transmitter from an external sensor, where the sensor signals and the external signals are stored based on an absolute or relative time of receipt.

Implementations of the aspects and embodiments may include one or more of the following. The external sensor may be an ambient noise sensor the method may further conclude calculating a sleep or exercise event based on the signals from the noise sensor. The external sensor may be an accelerometer the method may further conclude calculating a sleep or exercise event based on the signals from the accelerometer. The external sensor may also be a GPS receiver the method may further include calculating a sleep or exercise or meal event based on the signals from the GPS receiver.

A thirty-fourth aspect is directed toward a method of operating a continuous glucose monitor, including: receiving signals at a mobile device from a transmitter as measured by an indwelling glucose sensor; receiving external signals at the mobile device from an external sensor, and storing the sensor signals and the external signals at the mobile device based on an absolute or relative time of receipt.

A thirty-fifth aspect is directed toward a transmitter configured for physical engagement with an indwelling glucose sensor and transmitting signals representative of measured glucose concentration values to a mobile device, including: a housing configured to physically engage a glucose sensor, the glucose sensor configured to at least partially indwell within a patient; a first communication circuit configured to communicate with the mobile device using a short-range wireless protocol; a second communication circuit configured to communicate with the mobile device using an encrypted wireless protocol; and a memory for storing information received from the glucose sensor.

Implementations of the aspects and embodiments may include one or more of the following: the first communication circuit may be a near field communication (NFC) circuit; the second communication circuit may be a Bluetooth low energy circuit the mobile device may be an HCP device; the transmitter may further include a feedback indicator that facilitates physical alignment by a user between an antenna of het NFC circuit and an antenna of the mobile device.

A thirty-sixth aspect is directed toward a method for communication between a mobile device and a transmitter configured for physical engagement with a continuous glucose sensor and for transmitting signals representative of measured glucose concentration values to the mobile device, including: receiving, at the transmitter, a data extraction command from the mobile device using a short-range wireless protocol; responsive to the data extraction command, causing the transmitter to be placed in a data extraction mode of operation; transmitting an advertising message in accordance with an encrypted wireless protocol to initiate a connection with the mobile device over the encrypted wireless protocol; and upon establishing a connection with the mobile device over the encrypted wireless protocol, transmitting estimated glucose values to the mobile device over the connection.

Implementations of the aspects and embodiments may include one or more of the following: prior to receiving the data extraction command, receiving, at the transmitter, a query command using the short-range wireless protocol and, responsive to the query command, transmitting an encrypted identifier of the transmitter using the short-range wireless protocol; the query command may be issued after the glucose sensor is inserted in the patient; the query command may be issued while in an HCP office; responsive to the query command, transmitting operational state information specifying one or more operational states of the transmitter.

A thirty-seventh aspect is directed toward a continuous glucose monitoring device configured for use by a Health Care Professional (HCP), including: a housing; a first communication circuit configured to communicate with a continuous glucose monitoring device that includes a transmitter coupled to an indwelling glucose sensor using a short-range wireless protocol; a second communication circuit configured to communicate with the transmitter using an encrypted wireless protocol; and a user interface for entering an operational mode in which the continuous glucose monitoring device is to operate.

A thirty-eighth aspect is directed toward a method for pairing a patient mobile device including a continuous glucose monitoring app with a transmitter, the transmitter configured for transmitting signals representative of measured glucose concentration values to the patient mobile device, including: detecting insertion of the transmitter in a glucose sensor housing; responsive to the detecting, automatically causing the transmitter to transition from an inactive state to an active state in which the transmitter is able to transmit signals encoded with data corresponding to readings received from the sensor, broadcasting an advertisement message in accordance with a first wireless protocol; responsive to the broadcasting, opening an app on the patient mobile device; causing a security code to be entered on the patient mobile device to pair the transmitter with the patient mobile device using the first wireless protocol; pairing the transmitter with the patient mobile device using the first wireless protocol if the security code is correct; and responsive to the pairing, causing a sensor session to begin.

A thirty-ninth aspect is directed toward a method of activating a transmitter in a rapid fashion, the transmitter having been stored for a duration of time, the transmitter configured for physical engagement with an indwelling glucose sensor, the transmitter configured for transmitting signals representative of measured glucose concentration values to a mobile device including; on a transmitter that is stored in packaging, detecting a signal indicative of an acceleration event; and if the detected signal is determined to have indicate that the acceleration event resulted from acceleration within a specified range, causing the transmitter to transition from an inactive state to an active state.

A fortieth aspect is directed toward a method for communication between a mobile device and a transmitter configured for physical engagement with an indwelling glucose sensor, the transmitter configured for transmitting signals representative of measured glucose concentration values to the mobile device, including: receiving, at the transmitter, a data extraction command from the mobile device using a short-range wireless protocol; responsive to the data extraction command, causing the transmitter to be placed in a data extraction mode of operation; transmitting an advertising message in accordance with an encrypted wireless protocol to initiate a connection with the mobile device over the encrypted wireless protocol; and upon establishing a connection with the mobile device over the encrypted wireless protocol, transmitting operational state information specifying at least one operational state of the transmitter, the at least one operational state of the transmitter including a sensor session completion state indicating that a session is complete and the data is available for downloading.

A forty-first aspect is directed toward a method for communication between an HCP reader and a transmitter configured for physical engagement with an indwelling glucose sensor, the transmitter configured for transmitting signals representative of measured glucose concentration values to the HCP reader, including: receiving, at the transmitter, a data extraction command from the HCP reader using a short-range wireless protocol, the data extraction command including an encrypted version of an identifier of the transmitter; responsive to the data extraction command, causing the transmitter to be placed in a data extraction mode of operation if the transmitter determines that the identifier included in the data extraction command is correct; transmitting an advertising message in accordance with an encrypted wireless protocol to initiate a connection with the HCP reader over the encrypted wireless protocol, the advertising message including information that causes the HCP reader to have priority of connection over other devices; and upon establishing a connection with the HCP reader over the encrypted wireless protocol, transmitting sensor data to the HCP reader over the connection.

In further aspects and embodiments, the above method features of the various aspects are formulated in terms of a system as in various aspects, configured to carry out the method features. Any of the features of an embodiment of any of the aspects, including but not limited to any embodiments of any of the first through thirty-fourth aspects referred to above, is applicable to all other aspects and embodiments identified herein, including but not limited to any embodiments of any of the first through thirty-fourth aspects referred to above. Moreover, any of the features of an embodiment of the various aspects, including but not limited to any embodiments of any of the first through thirty-fourth aspects referred to above, is independently combinable, partly or wholly with other embodiments described herein in any way, e.g., one, two, or three or more embodiments may be combinable in whole or in part. Further, any of the features of an embodiment of the various aspects, including but not limited to any embodiments of any of the first through thirty-fourth aspects referred to above, may be made optional to other aspects or embodiments. Any aspect or embodiment of a method can be performed by a system or apparatus of another aspect or embodiment, and any aspect or embodiment of a system or apparatus can be configured to perform a method of another aspect or embodiment, including but not limited to any embodiments of any of the first through thirty-fourth aspects referred to above.

Advantages of the aspects may include, in certain embodiments, one or more of the following. Systems and methods according to present principles can provide a valuable educational tool that increases communications between HCP's and patients, and allows greater education of the user about their disease, and the treatment thereof. The HCP and patient are provided more useful information in making decisions, therapies, and behavioral decision-making. The HCP's are enabled to have more meaningful discussions with their patients. HCP's are advantageously provided downloadable data ahead of time, prior to their meeting with the patient, making patient visits more useful and informative. Other advantages will be understood from the description that follows, including the figures and claims.

This Summary is provided to introduce a selection of concepts in a simplified form. The concepts are further described in the Detailed Description section. Elements or steps other than those described in this Summary are possible, and no element or step is necessarily required. This Summary is not intended to identify key features or essential features of the claimed subject matter, nor is it intended for use as an aid in determining the scope of the claimed subject matter. The claimed subject matter is not limited to implementations that solve any or all disadvantages noted in any part of this disclosure.

### BRIEF DESCRIPTION OF THE DRAWINGS

Further aspects of the present disclosure will be more readily appreciated upon review of the detailed description of the various disclosed embodiments, described below, when taken in conjunction with the accompanying figures.
FIG. 1A illustrates aspects of an example system that may be used in connection with implementing embodiments of the disclosure.
FIG. 1B illustrates aspects of an example system that may be used in connection with implementing embodiments of the disclosure.
FIG. 2A is a perspective view of an example enclosure that may be used in connection with implementing embodiments of an analyte sensor system.
FIG. 2B is a side view of an example enclosure that may be used in connection with implementing embodiments of an analyte sensor system.
FIG. 3A illustrates aspects of an example system that may be used in connection with implementing embodiments of the disclosure.
FIG. 3B illustrates aspects of an example system that may be used in connection with implementing embodiments of the disclosure.
FIG. 4 illustrates a system within the context of an HCP office, the system incorporating various elements for use by the HCP and/or a user.
FIG. 5 illustrates a logical arrangement of various parts of a system according to present principles.
FIG. 6 illustrates a flow chart of a method according to an implementation of present principles.
FIG. 7 illustrates a flow chart of a method according to another implementation of present principles.
FIG. 8 illustrates a flow chart of a method according to another implementation of present principles.
FIG. 9 illustrates a flow chart of a method according to another implementation of present principles.
FIG. 10 illustrates a flow chart of a method according to another implementation of present principles.
FIG. 11 illustrates a flow chart of a method according to another implementation of present principles.
FIG. 12 illustrates a flow chart of a method according to another implementation of present principles.
FIG. 13 illustrates a flow chart of a method according to another implementation of present principles.
FIG. 14 illustrates a flow chart of a method according to another implementation of present principles.
FIG. 15 illustrates a flow chart of a method according to another implementation of present principles.
FIG. 16 illustrates a flow chart of a method according to another implementation of present principles.
FIG. 17 illustrates a flow chart of a method according to another implementation of present principles.
FIGs. 18 and 19 illustrates a transmitter according to an implementation of present principles.
FIG. 20 illustrates a flow chart of a method according to another implementation of present principles.
FIG. 21 illustrates a flow chart of a method according to another implementation of present principles.
FIG. 22 illustrates a flow chart of a method according to another implementation of present principles.
FIG. 23 illustrates a flow chart of a method according to another implementation of present principles.
FIG. 24 illustrates a flow chart of a method according to another implementation of present principles.
FIG. 25 illustrates a flow chart of a method according to another implementation of present principles.
FIG. 26A illustrates a flow chart of a method according to another implementation of present principles.
FIG. 26B illustrates a chart of power use versus time in a mode configured for particularly low battery consumption.
FIG. 27 illustrates a flow chart of a method according to another implementation of present principles.
FIG. 28 illustrates a flow chart of a method according to another implementation of present principles.
FIG. 29 illustrates a logical diagram of an arrangement according to present principles.
FIG. 30 illustrates a flow chart of a method according to another implementation of present principles.
FIG. 31 illustrates a flow chart of a method according to another implementation of present principles.
FIG. 32 illustrates a logical diagram of an arrangement according to present principles.
FIG. 33 illustrates a logical diagram of an arrangement according to present principles.
FIG. 34 illustrates a flow chart of a method according to another implementation of present principles.
FIG. 35 illustrates a flow chart of a method according to another implementation of present principles.
FIG. 36A illustrates a flow chart of a method according to another implementation of present principles.
FIG. 36B illustrates a schematic of a transmitter chip including a wake-up pin.
FIG. 37 illustrates a flow chart of a method according to another implementation of present principles.
FIG. 38 illustrates a flow chart of a method according to another implementation of present principles.
FIG. 39 illustrates a flow chart of a method according to another implementation of present principles.
FIG. 40 illustrates a logical diagram of an arrangement according to present principles.
FIG. 41 illustrates a flow chart of a method according to another implementation of present principles.

The figures are described in greater detail in the description and examples below, are provided for purposes of illustration only, and merely depict typical or example embodiments of the disclosure. The figures are not intended to be exhaustive or to limit the disclosure to the precise form disclosed. It should also be understood that the disclosure may be practiced with modification or alteration, and that the disclosure may be limited only by the claims and the equivalents thereof.

### DETAILED DESCRIPTION

Embodiments of the present disclosure are directed to systems, methods, and devices for HCP set up of analyte monitoring systems, as well as ancillary technology and functionality. In various implementations and arrangements described herein, the analyte data is glucose data generated by an analyte sensor system configured to connect to receivers such as display devices and the like. Implementing aspects of the present disclosure, as described in detail herein, may include communication protocols and ways of not just setting up analyte monitoring systems but also downloading data therefrom. Moreover, implementing aspects of the present disclosure may also relate to systems and methods for conserving battery life in such systems, as such systems constitute wearable devices, and battery capacity, as well as circuitry real estate, are at a premium.

The details of some example embodiments of the systems, methods, and devices of the present disclosure are set forth in this description and in some cases, in other portions of the disclosure. Other features, objects, and advantages of the disclosure will be apparent to one of skill in the art upon examination of the present disclosure, description, figures, examples, and claims. It is intended that all such additional systems, methods, devices, features, and advantages be included within this description (whether explicitly or by reference), be within the scope of the present disclosure, and be protected by one or more of the accompanying claims.

### Overview

In some embodiments, a system is provided for continuous measurement of an analyte in a host. The system may include: a continuous analyte sensor configured to continuously measure a concentration of the analyte in the host, and a sensor electronics module physically connected to the continuous analyte sensor during sensor use. In certain embodiments, the sensor electronics module includes electronics configured to process a data stream associated with an analyte concentration measured by the continuous analyte sensor, in order to generate sensor information that includes raw sensor data, transformed sensor data, and/or any other sensor data, for example. The sensor electronics module may further be configured to generate sensor information that is customized for respective display devices, such that different display devices may receive different sensor information.

For ease of explanation and illustration, in some instances the detailed description describes exemplary systems and methods in terms of a continuous glucose monitoring environment, however it should be understood that the scope of the invention is not limited to that particular environment, and that one skilled in the art will appreciate that the systems and methods described herein can be embodied in various forms. Accordingly any structural and/or functional details disclosed herein are not to be interpreted as limiting the systems and methods, but rather are provided as attributes of a representative embodiment and/or arrangement for teaching one skilled in the art one or more ways to implement the systems and methods, which may be advantageous in other contexts.

For example, and without limitation, described monitoring systems and methods may include sensors that measure the concentration of one or more analytes (for instance glucose, lactate, potassium, pH, cholesterol, isoprene, and/or hemoglobin) and/or other blood or bodily fluid constituents of or relevant to a host and/or another party.

By way of example, and without limitation, monitoring system and method embodiments described herein may include finger-stick blood sampling, blood analyte test strips, non-invasive sensors, wearable monitors (e.g. smart bracelets, smart watches, smart rings, smart necklaces or pendants, workout monitors, fitness monitors, health and/or medical monitors, clipon monitors, and the like), adhesive sensors, smart textiles and/or clothing incorporating sensors, shoe inserts and/or insoles that include sensors, transdermal (i.e. transcutaneous) sensors, and/or swallowed, inhaled or implantable sensors.

In some embodiments, and without limitation, monitoring systems and methods may comprise other sensors instead of or in additional to the sensors described herein, such as inertial measurement units including accelerometers, gyroscopes, magnetometers and/or barometers; motion, altitude, position, and/or location sensors; biometric sensors; optical sensors including for instance optical heart rate monitors, photoplethysmogram (PPG)/pulse oximeters, fluorescence monitors, and cameras; wearable electrodes; electrocardiogram (EKG or ECG), electroencephalography (EEG), and/or electroinyography (EMG) sensors; chemical sensors; flexible sensors for instance for measuring stretch, displacement, pressure, weight, or impact; galvanometric sensors, capacitive sensors, electric field sensors, temperature/thermal sensors, microphones, vibration sensors, ultrasound sensors, piezoelectric/piezoresistive sensors, and/or transducers for measuring information of or relevant to a host and/or another party.

The term "analyte" as used herein is a broad term and is to be given its ordinary and customary meaning to a person of ordinary skill in the art (and is not to be limited to a special or customized meaning), and furthermore refers without limitation to a substance or chemical constituent in a biological fluid (for example, blood, interstitial fluid, cerebral spinal fluid, lymph fluid, urine, sweat, saliva, etc.) that can be analyzed. Analytes can include naturally occurring substances, artificial substances, metabolites, and/or reaction products. In some implementations, the analyte for measurement by the methods or devices is glucose. However, other analytes are contemplated as well, including but not limited to: acarboxyprothrombin; acetoacetic acid; acetone; Acetyl CoA; acylcarnitine; adenine phosphoribosyl transferase; adenosine deaminase; albumin; alpha-fetoprotein; amino acid profiles (arginine (Krebs cycle), histidine/urocanic acid, homocysteine, phenylalanine/tyrosine, tryptophan); andrenostenedione; antipyrine; arabinitol enantiomers; arginase; benzoylecgonine (cocaine); biotinidase; biopterin; c-reactive protein; carnitine; carnosinase; CD4; ceruloplasmin; chenodeoxycholic acid; chloroquine; cholesterol; cholinesterase; conjugated 1-β hydroxy-cholic acid; cortisol; creatine kinase; creatine kinase MM isoenzyme; cyclosporin A; d-penicillamine; de-ethylchloroquine; dehydroepiandrosterone sulfate; DNA (acetylator polymorphism, alcohol dehydrogenase, alpha 1-antitrypsin, cystic fibrosis, Duchenne/Becker muscular dystrophy, glucose-6-phosphate dehydrogenase, hemoglobin A, hemoglobin S, hemoglobin C, hemoglobin D, hemoglobin E, hemoglobin F, D-Punjab, betathalassemia, hepatitis B virus, HCMV, HIV-1, HTLV-1, Leber hereditary optic neuropathy, MCAD, RNA, PKU, Plasmodium vivax, sexual differentiation, 21-deoxycortisol); desbutylhalofantrine; dihydropteridine reductase; diptheria/tetanus antitoxin; erythrocyte arginase; erythrocyte protoporphyrin; esterase D; fatty acids/acylglycines; triglycerides; glycerol; free β-human chorionic gonadotropin; free erythrocyte porphyrin; free thyroxine (FT4); free tri-iodothyronine (FT3); fumarylacetoacetase; galactose/gal-1-phosphate; galactose-1-phosphate uridyltransferase; gentamicin; glucose-6-phosphate dehydrogenase; glutathione; glutathione perioxidase; glycocholic acid; glycosylated hemoglobin; halofantrine; hemoglobin variants; hexosaminidase A; human erythrocyte carbonic anhydrase I; 17-alpha-hydroxyprogesterone; hypoxanthine phosphoribosyl transferase; immunoreactive trypsin; ketone bodies; lactate; lead; lipoproteins ((a), B/A-1, β); lysozyme; mefloquine; netilmicin; phenobarbitone; phenytoin; phytanic/pristanic acid; progesterone; prolactin; prolidase; purine nucleoside phosphorylase; quinine; reverse tri-iodothyronine (rT3); selenium; serum pancreatic lipase; sissomicin; somatomedin C; specific antibodies (adenovirus, anti-nuclear antibody, anti-zeta antibody, arbovirus, Aujeszky's disease virus, Dracunculus medinensis, Echinococcus granulosus, Entamoeba histolytica, enterovirus, Giardia duodenalisa, Helicobacter pylori, hepatitis B virus, herpes virus, HIV-1, IgE (atopic disease), influenza virus, isoprene (2-methyl-1,3-butadiene), Leishmania donovani, leptospira, measles/mumps/rubella, Mycobacterium leprae, Mycoplasma pneumoniae, Myoglobin, Onchocerca volvulus, parainfluenza virus, Plasmodium falciparum, poliovirus, Pseudomonas aeruginosa, respiratory syncytial vims, rickettsia (scrub typhus), Schistosoma mansoni, Toxoplasma gondii, Trepenoma pallidium, Trypanosoma cruzi/rangeli, vesicular stomatis virus, Wuchereria bancrofti, Flavivirus (for example deer tick, dengue fever, Powassan, West Nile, yellow fever, or Zika virus); specific antigens (hepatitis B virus, HIV-1); succinylacetone; sulfadoxine; theophylline; thyrotropin (TSH); thyroxine (T4); thyroxine-binding globulin; trace elements; transferrin; UDP-galactose-4-epimerase; urea; uroporphyrinogen I synthase; vitamin A; white blood cells; and zinc protoporphyrin. Salts, sugar, protein, fat, vitamins, and hormones naturally occurring in blood or interstitial fluids can also constitute analytes in certain implementations. The analyte can be naturally present in the biological fluid, for example, a metabolic product, a hormone, an antigen, an antibody, and the like. Alternatively, the analyte can be introduced into the body or exogenous, for example, a contrast agent for imaging, a radioisotope, a chemical agent, a fluorocarbon-based synthetic blood, or a drug or pharmaceutical composition, including but not limited to insulin; glucagon, ethanol; cannabis (marijuana, tetrahydrocannabinol, hashish); inhalants (nitrous oxide, amyl nitrite, butyl nitrite, chlorohydrocarbons, hydrocarbons); cocaine (crack cocaine); stimulants (amphetainines, methamphetamines, Ritalin, Cylert, Preludin, Didrex, PreState, Voranil, Sandrex, Plegine); depressants (barbiturates, methaqualone, tranquilizers such as Valium, Librium, Miltown, Serax, Equanil, Tranxene); hallucinogens (phencyclidine, lysergic acid, mescaline, peyote, psilocybin); narcotics (heroin, codeine, morphine, opium, meperidine, Percocet, Percodan, Tussionex, Fentanyl, Darvon, Talwin, Lomotil); designer drugs (analogs of fentanyl, meperidine, amphetamines, methamphetamines, and phencyclidine, for example, Ecstasy); anabolic steroids; and nicotine. The metabolic products of drugs and pharmaceutical compositions are also contemplated analytes. Analytes such as neurochemicals and other chemicals generated within the body can also be analyzed, such as, for example, ascorbic acid, uric acid, dopamine, noradrenaline, 3-methoxytyramine (3MT), 3,4-Dihydroxyphenylacetic acid (DOPAC), Homovanillic acid (HVA), 5-Hydroxytryptamine (5HT), and 5-Hydroxyindoleacetic acid (FHIAA), and intermediaries in the Citric Acid Cycle.

### Alerts

In certain embodiments, one or more alerts are associated with a sensor electronics module. For example, each alert may include one or more alert conditions that indicate when the respective alert has been triggered. For example, a hypoglycemic alert may include alert conditions indicating a minimum glucose level. The alert conditions may also be based on transformed sensor data, such as trending data, and/or sensor data from multiple different sensors (e.g. an alert may be based on sensor data from both a glucose sensor and a temperature sensor). For example, a hypoglycemic alert may include alert conditions indicating a minimum required trend in the host's glucose level that must be present before triggering the alert. The term "trend," as used herein refers generally to data indicating some attribute of data that is acquired over time, e.g., such as calibrated or filtered data from a continuous glucose sensor. A trend may indicate amplitude, rate of change, acceleration, direction, etc., of data, such as sensor data, including transformed or raw sensor data.

In certain embodiments, each of the alerts is associated with one or more actions that are to be performed in response to triggering of the alert. Alert actions may include, for example, activating an alarm, such as displaying information on a display of the sensor electronics module or activating an audible or vibratory alarm coupled to the sensor electronics module, and/or transmitting data to one or more display devices external to the sensor electronics module. For any delivery action that is associated with a triggered alert, one or more delivery options define the content and/or format of the data to be transmitted, the device to which the data is to be transmitted, when the data is to be transmitted, and/or a communication protocol for delivery of the data.

In certain embodiments, multiple delivery actions (each having respective delivery options) may be associated with a single alert such that displayable sensor information having different content and formatting, for example, is transmitted to respective display devices in response to triggering of a single alert. For example, a mobile telephone may receive a data package including minimal displayable sensor information (that may be formatted specifically for display on the mobile telephone), while a desktop computer may receive a data package including most (or all) of the displayable sensor information that is generated by the sensor electronics module in response to triggering of a common alert. Advantageously, the sensor electronics module is not tied to a single display device, rather it is configured to communicate with a plurality of different display devices directly, systematically, simultaneously (e.g., *via* broadcasting), regularly, periodically, randomly, on-demand, in response to a query, based on alerts or alarms, and/or the like.

In some embodiments, clinical risk alerts are provided that include alert conditions that combine intelligent and dynamic estimative algorithms that estimate present or predicted danger with greater accuracy, more timeliness in pending danger, avoidance of false alarms, and less annoyance for the patient. In general, clinical risk alerts include dynamic and intelligent estimative algorithms based on analyte value, rate of change, acceleration, clinical risk, statistical probabilities, known physiological constraints, and/or individual physiological patterns, thereby providing more appropriate, clinically safe, and patient-friendly alarms. U.S. Patent Publication No. 2007/0208246, which is incorporated herein by reference in its entirety, describes some systems and methods associated with the clinical risk alerts (or alarms) described herein. In some embodiments, clinical risk alerts can be triggered for a predetermined time period to allow for the user to attend to his/her condition. Additionally, the clinical risk alerts can be de-activated when leaving a clinical risk zone so as not to annoy the patient by repeated clinical alarms (e.g., visual, audible or vibratory), when the patient's condition is improving. In some embodiments, dynamic and intelligent estimation determines a possibility of the patient avoiding clinical risk, based on the analyte concentration, the rate of change, and other aspects of the dynamic and intelligent estimative algorithms. If there is minimal or no possibility of avoiding the clinical risk, a clinical risk alert will be triggered. However, if there is a possibility of avoiding the clinical risk, the system is configured to wait a predetermined amount of time and re-analyze the possibility of avoiding the clinical risk. In some embodiments, when there is a possibility of avoiding the clinical risk, the system is further configured to provide targets, therapy recommendations, or other information that can aid the patient in proactively avoiding the clinical risk.

In some embodiments, the sensor electronics module is configured to search for one or more display devices within communication range of the sensor electronics module and to wirelessly communicate sensor information (e.g., a data package including displayable sensor information, one or more alarm conditions, and/or other alarm information) thereto. Accordingly, the display device is configured to display at least some of the sensor information and/or alarm the host (and/or care taker), wherein the alarm mechanism is located on the display device.

In some embodiments, the sensor electronics module is configured to provide one or a plurality of different alarms *via* the sensor electronics module and/or via transmission of a data package indicating an alarm should be initiated by one or a plurality of display devices (e.g., sequentially and/or simultaneously). In certain embodiments, the sensor electronics module merely provides a data field indicating that an alarm conditions exists and the display device, upon reading the data field indicating the existence of the alarm condition, may decide to trigger an alarm. In some embodiments, the sensor electronics module determines which of the one or more alarms to trigger based on one or more alerts that are triggered. For example, when an alert trigger indicates severe hypoglycemia, the sensor electronics module can perform multiple actions, such as activating an alarm on the sensor electronics module, transmitting a data package to a monitoring device indicating activation of an alarm on the display, and transmitting a data package as a text message to a care provider. As an example, a text message can appear on a custom monitoring device, cell phone, pager device, and/or the like, including displayable sensor information that indicates the host's condition (e.g., "severe hypoglycemia").

In some embodiments, the sensor electronics module is configured to wait a time period for the host to respond to a triggered alert (e.g., by pressing or selecting a snooze and/or off function and/or button on the sensor electronics module and/or a display device), after which additional alerts are triggered (e.g., in an escalating manner) until one or more alerts are responded to. In some embodiments, the sensor electronics module is configured to send control signals (e.g., a stop signal) to a medical device associated with an alarm condition (e.g., hypoglycemia), such as an insulin pump, wherein the stop alert triggers a stop of insulin delivery *via* the pump.

In some embodiments, the sensor electronics module is configured to directly, systematically, simultaneously (e.g., *via* broadcasting), regularly, periodically, randomly, ondemand, in response to a query (from the display device), based on alerts or alarms, and/or the like transmit alarm information. In some embodiments, the system further includes a repeater such that the wireless communication distance of the sensor electronics module can be increased, for example, to 10, 20, 30, 50 75, 100, 150, or 200 meters or more, wherein the repeater is configured to repeat a wireless communication from the sensor electronics module to the display device located remotely from the sensor electronics module. A repeater can be useful to families having children with diabetes. For example, to allow a parent to carry, or place in a stationary position, a display device, such as in a large house wherein the parents sleep at a distance from the child.

### Display Devices

In some embodiments, the sensor electronics module is configured to search for and/or attempt wireless communication with a display device from a list of display devices. In some embodiments, the sensor electronics module is configured to search for and/or attempt wireless communication with a list of display devices in a predetermined and/or programmable order (e.g., grading and/or escalating), for example, wherein a failed attempt at communication with and/or alarming with a first display device triggers an attempt at communication with and/or alarming with a second display device, and so on. In one example embodiment, the sensor electronics module is configured to search for and attempt to alarm a host or care provider sequentially using a list of display devices, such as: (1) a default display device or a custom analyte monitoring device; (2) a mobile phone via auditory and/or visual methods, such as, text message to the host and/or care provider, voice message to the host and/or care provider, and/or 911); (3) a tablet; (4) a smart watch or bracelet; and/or (5) smart glasses or other wearable display device.

Depending on the embodiment, one or more display devices that receive data packages from the sensor electronics module are "dummy displays", wherein they display the displayable sensor information received from the sensor electronics module without additional processing (e.g., prospective algorithmic processing necessary for real-time display of sensor information). In some embodiments, the displayable sensor information comprises transformed sensor data that does not require processing by the display device prior to display of the displayable sensor information. Some display devices may include software including display instructions (software programming comprising instructions configured to display the displayable sensor information and optionally query the sensor electronics module to obtain the displayable sensor information) configured to enable display of the displayable sensor information thereon. In some embodiments, the display device is programmed with the display instructions at the manufacturer and can include security and/or authentication to avoid plagiarism of the display device. In some embodiments, a display device is configured to display the displayable sensor information *via* a downloadable program (for example, a downloadable Java Script via the internet), such that any display device that supports downloading of a program (for example, any display device that supports Java applets) therefore can be configured to display displayable sensor information (e.g., mobile phones, tablets, PDAs, PCs and the like).

In some embodiments, certain display devices may be in direct wireless communication with the sensor electronics module, but intermediate network hardware, firmware, and/or software can be included within the direct wireless communication. In some embodiments, a repeater (e.g., a Bluetooth repeater) can be used to re-transmit the transmitted displayable sensor information to a location farther away than the immediate range of the telemetry module of the sensor electronics module, wherein the repeater enables direct wireless communication when substantive processing of the displayable sensor information does not occur. In some embodiments, a receiver (e.g., Bluetooth receiver) can be used to re-transmit the transmitted displayable sensor information, possibly in a different format, such as in a text message onto a TV screen, wherein the receiver enables direct wireless communication when substantive processing of the sensor information does not occur. In certain embodiments, the sensor electronics module directly wirelessly transmits displayable sensor information to one or a plurality of display devices, such that the displayable sensor information transmitted from the sensor electronics module is received by the display device without intermediate processing of the displayable sensor information.

In certain embodiments, one or more display devices include built-in authentication mechanisms, wherein authentication is required for communication between the sensor electronics module and the display device. In some embodiments, to authenticate the data communication between the sensor electronics module and display devices, a challenge-response protocol, such as a password authentication is provided, where the challenge is a request for the password and the valid response is the correct password, such that pairing of the sensor electronics module with the display devices can be accomplished by the user and/or manufacturer *via* the password. This may be referred to in some cases as two-way authentication. In some embodiments, biometric authentication may also be employed.

In some embodiments, one or more display devices are configured to query the sensor electronics module for displayable sensor information, wherein the display device acts as a master device requesting sensor information from the sensor electronics module (e.g., a slave device) on-demand, for example, in response to a query. In some embodiments, the sensor electronics module is configured for periodic, systematic, regular, and/or periodic transmission of sensor information to one or more display devices (for example, every 1, 2, 5, or 10 minutes or more). In some embodiments, the sensor electronics module is configured to transmit data packages associated with a triggered alert (e.g., triggered by one or more alert conditions). However, any combination of the above described statuses of data transmission can be implemented with any combination of paired sensor electronics module and display device(s). For example, one or more display devices can be configured for querying the sensor electronics module database and for receiving alarm information triggered by one or more alarm conditions being met. Additionally, the sensor electronics module can be configured for periodic transmission of sensor information to one or more display devices (the same or different display devices as described in the previous example), whereby a system can include display devices that function differently with regard to how sensor information is obtained.

In some embodiments, a display device is configured to query the data storage memory in the sensor electronics module for certain types of data content, including direct queries into a database in the sensor electronics module's memory and/or requests for configured or configurable packages of data content therefrom; namely, the data stored in the sensor electronics module is configurable, queryable, predetermined, and/or pre-packaged, based on the display device with which the sensor electronics module is communicating. In some additional or alternative embodiments, the sensor electronics module generates the displayable sensor information based on its knowledge of which display device is to receive a particular transmission. Additionally, some display devices are capable of obtaining calibration information and wirelessly transmitting the calibration information to the sensor electronics module, such as through manual entry of the calibration information, automatic delivery of the calibration information, and/or an integral reference analyte monitor incorporated into the display device. U.S. Patent Publication Nos. 2006/0222566, 2007/0203966, 2007/0208245, and 2005/0154271, all of which are incorporated herein by reference in their entirety, describe systems and methods for providing an integral reference analyte monitor incorporated into a display device and/or other calibration methods that can be implemented with embodiments disclosed herein.

In general, a plurality of display devices (e.g., a custom analyte monitoring device (which may also be referred to as an analyte display device), a mobile phone, a tablet, a smart watch, a reference analyte monitor, a drug delivery device, a medical device and a personal computer) may be configured to wirelessly communicate with the sensor electronics module. The plurality of display devices may be configured to display at least some of the displayable sensor information wirelessly communicated from the sensor electronics module. The displayable sensor information may include sensor data, such as raw data and/or transformed sensor data, such as analyte concentration values, rate of change information, trend information, alert information, sensor diagnostic information and/or calibration information, for example.

### Analyte Sensor

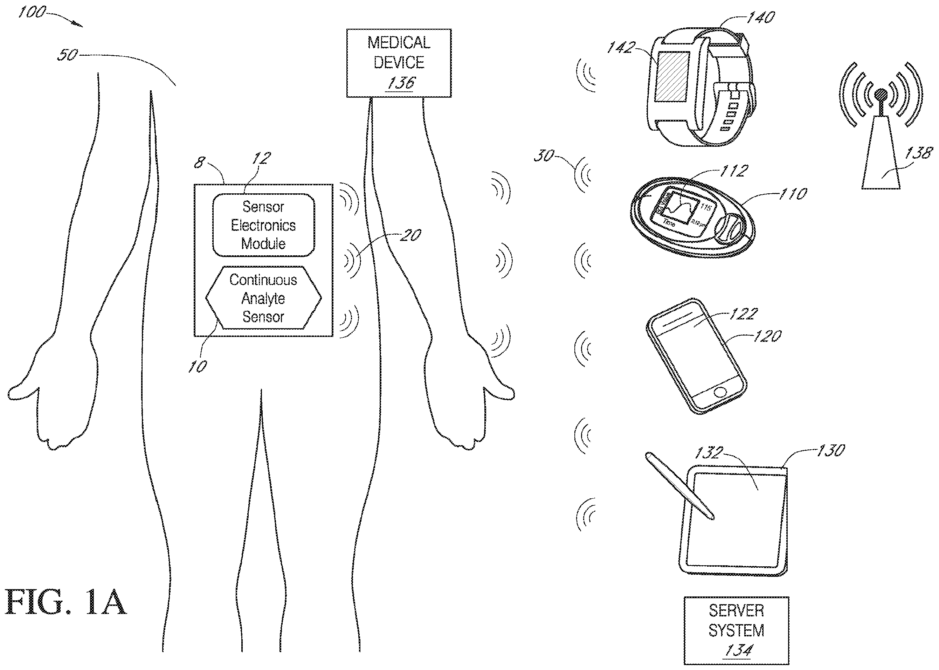

With reference to FIG. 1A, in some embodiments, analyte sensor 10 includes a continuous analyte sensor, for example, a subcutaneous, transdermal (e.g., transcutaneous), or intravascular device. In some embodiments, such a sensor or device can analyze a plurality of intermittent blood samples. While the present disclosure includes embodiments of glucose sensors, such embodiments may be used for other analytes as well. The glucose sensor can use any method of glucose-measurement, including enzymatic, chemical, physical, electrochemical, spectrophotometric, polarimetric, calorimetric, iontophoretic, radiometric, immunochemical, and the like.

A glucose sensor can use any known method, including invasive, minimally invasive, and non-invasive sensing techniques (e.g., fluorescent monitoring), to provide a data stream indicative of the concentration of glucose in a host. The data stream is typically a raw data signal, which is converted into a calibrated and/or filtered data stream that is used to provide a useful value of glucose to a user, such as a patient or a caretaker (e.g., a parent, a relative, a guardian, a teacher, a doctor, a nurse, or any other individual that has an interest in the wellbeing of the host).

A glucose sensor can be any device capable of measuring the concentration of glucose. According to one example embodiment described below, an implantable glucose sensor may be used. However, it should be understood that the devices and methods described herein can be applied to any device capable of detecting a concentration of glucose and providing an output signal that represents the concentration of glucose (e.g., as a form of analyte data).

In certain embodiments, analyte sensor 10 is an implantable glucose sensor, such as described with reference to U.S. Patent 6,001,067 and U.S. Patent Publication No. US-2005-0027463-A1. In embodiments, analyte sensor 10 is a transcutaneous glucose sensor, such as described with reference to U.S. Patent Publication No. US-2006-0020187-A1. In embodiments, analyte sensor 10 is configured to be implanted in a host vessel or extracorporeally, such as is described in U.S. Patent Publication No. US-2007-0027385-A1, co-pending U.S. Patent Publication No. US-2008-0119703-A1 filed October 4, 2006, U.S. Patent Publication No. US-2008-0108942-A1 filed on March 26, 2007, and U.S. Patent Application No. US-2007-0197890-A1 filed on February 14, 2007. In embodiments, the continuous glucose sensor includes a transcutaneous sensor such as described in U.S. Patent 6,565,509 to Say et al., for example. In embodiments, analyte sensor 10 is a continuous glucose sensor that includes a subcutaneous sensor such as described with reference to U.S. Patent 6,579,690 to Bonnecaze et al. or U.S. Patent 6,484,046 to Say et al., for example. In embodiments, the continuous glucose sensor includes a refillable subcutaneous sensor such as described with reference to U.S. Patent 6,512,939 to Colvin et al., for example. The continuous glucose sensor may include an intravascular sensor such as described with reference to U.S. Patent 6,477,395 to Schulman et al., for example. The continuous glucose sensor may include an intravascular sensor such as described with reference to U.S. Patent 6,424,847 to Mastrototaro et al., for example.

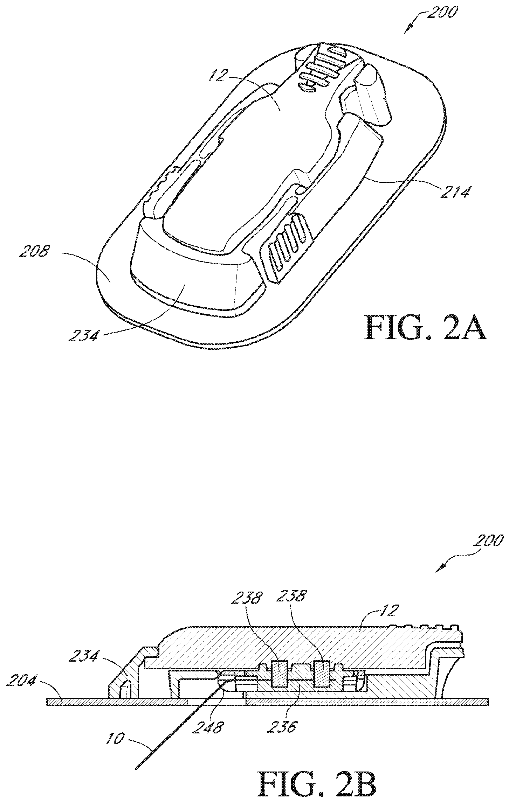

FIGS. 2A and 2B are perspective and side views of enclosure 200 that may be used in connection with implementing embodiments of analyte sensor system 8, according certain aspects of the present disclosure. Enclosure 200 includes mounting unit 214 and sensor electronics module 12 attached thereto in certain embodiments. Enclosure 200 is shown in a functional position, including mounting unit 214 and sensor electronics module 12 matingly engaged therein. In some embodiments, mounting unit 214, also referred to as a housing or sensor pod, includes base 234 adapted for fastening to a host's or user's skin. Base 234 can be formed from a variety of hard or soft materials, and can include a low profile for minimizing protrusion of the device from the host during use. In some embodiments, base 234 is formed at least partially from a flexible material, which may provide numerous advantages over other transcutaneous sensors, which, unfortunately, can suffer from motion-related artifacts associated with the host's movement when the host is using the device. Mounting unit 214 and/or sensor electronics module 12 can be located over the sensor insertion site to protect the site and/or provide a minimal footprint (utilization of surface area of the host's skin).

In some embodiments, a detachable connection between mounting unit 214 and sensor electronics module 12 is provided, which enables improved manufacturability, namely, the potentially relatively inexpensive mounting unit 214 can be disposed of when refurbishing or maintaining analyte sensor system 8, while the relatively more expensive sensor electronics module 12 can be reusable with multiple sensor systems. In some embodiments, sensor electronics module 12 is configured with signal processing (programming), for example, configured to filter, calibrate, and/or execute other algorithms useful for calibration and/or display of sensor information. However, an integral (non-detachable) sensor electronics module can be configured.

In some embodiments, contacts 238 are mounted on or in a subassembly hereinafter referred to as contact subassembly 236 configured to fit within base 234 of mounting unit 214 and hinge 248 that allows contact subassembly 236 to pivot between a first position (for insertion) and a second position (for use) relative to mounting unit 214. The term "hinge" as used herein is a broad term and is used in its ordinary sense, including, without limitation, to refer to any of a variety of pivoting, articulating, and/or hinging mechanisms, such as an adhesive hinge, a sliding joint, and the like; the term hinge does not necessarily imply a fulcrum or fixed point about which the articulation occurs. In some embodiments, contacts 238 are formed from a conductive elastomeric material, such as a carbon black elastomer, through which sensor 10 extends.

With further reference to FIGS. 2A and 213, in certain embodiments, mounting unit 214 is provided with adhesive pad 208, disposed on the mounting unit's back surface and includes a releasable backing layer. Thus, removing the backing layer and pressing at last a portion of base 234 of mounting unit 214 onto the host's skin adheres mounting unit 214 to the host's skin. Additionally or alternatively, an adhesive pad can be placed over some or all of analyte sensor system 8 and/or sensor 10 after sensor insertion is complete to ensure adhesion, and optionally to ensure an airtight seal or watertight seal around the wound exit-site (or sensor insertion site) (not shown). Appropriate adhesive pads can be chosen and designed to stretch, elongate, conform to, and/or aerate the region (*e.g*., host's skin). The embodiments described with reference to FIGS. 2A and 2B are described in more detail with reference to U.S. Patent No. 7,310,544, which is incorporated herein by reference in its entirety. Configurations and arrangements can provide water resistant, waterproof, and/or hermetically sealed properties associated with the mounting unit/sensor electronics module embodiments described herein.

Various methods and devices that are suitable for use in conjunction with aspects of some embodiments are disclosed in U.S. Patent Publication No. US-2009-0240120-A1, which is incorporated herein by reference in its entirety for all purposes.

### Example Configurations

Referring again to FIG. 1A, system 100 that may be used in connection with implementing aspects of an analyte sensor system is depicted. In some cases, system 100 may be used to implement various systems described herein. System 100 in embodiments includes analyte sensor system 8 and display devices 110, 120, 130, and 140, according to certain aspects of the present disclosure. Analyte sensor system 8 in the illustrated embodiment includes sensor electronics module 12 and continuous analyte sensor 10 associated with the sensor electronics module 12. Sensor electronics module 12 may be in wireless communication (e.g., directly or indirectly) with one or more of display devices 110, 120, 130, and 140. In embodiments, system 100 also includes medical device 136 and server system 134. Sensor electronics module 12 may also be in wireless communication (e.g., directly or indirectly) with medical device 136 and server system 134. in some examples, display devices 110-140 may also be in wireless communication with the server system 134 and/or the medical device 136.

In certain embodiments, sensor electronics module 12 includes electronic circuitry associated with measuring and processing the continuous analyte sensor data, including prospective algorithms associated with processing and calibration of the sensor data. Sensor electronics module 12 can be physically connected to continuous analyte sensor 10 and can be integral with (non-releasably attached to) or releasably attachable to continuous analyte sensor 10. Sensor electronics module 12 may include hardware, firmware, and/or software that enables measurement of levels of the analyte *via* a glucose sensor. For example, sensor electronics module 12 can include a potentiostat, a power source for providing power to the sensor, other components useful for signal processing and data storage, and a telemetry module for transmitting data from the sensor electronics module to one or more display devices. Electronics can be affixed to a printed circuit board (PCB), or the like, and can take a variety of forms. For example, the electronics can take the form of an integrated circuit (IC), such as an Application-Specific Integrated Circuit (ASIC), a microcontroller, and/or a processor.

Sensor electronics module 12 may include sensor electronics that are configured to process sensor information, such as sensor data, and generate transformed sensor data and displayable sensor information. Examples of systems and methods for processing sensor analyte data are described in more detail herein and in U.S. Patent Nos. 7,310,544 and 6,931,327 and U.S. Patent Publication Nos. 2005/0043598, 2007/0032706, 2007/0016381, 2008/0033254, 2005/0203360, 2005/0154271, 2005/0192557, 2006/0222566, 2007/0203966 and 2007/0208245, all of which are incorporated herein by reference in their entirety for all purposes.

Referring again to FIG. 1A, display devices 110, 120, 130, and/or 140 are configured for displaying (and/or alarming) the displayable sensor information that may be transmitted by sensor electronics module 12 (e.g., in a customized data package that is transmitted to the display devices based on their respective preferences). Each of display devices 110, 120, 130, or 140 can include a display such as a touchscreen display 112, 122, 132, /or 142 for displaying sensor information and/or analyte data to a user and/or receiving inputs from the user. For example, a graphical user interface may be presented to the user for such purposes. In some embodiments, the display devices may include other types of user interfaces such as voice user interface instead of or in addition to a touchscreen display for communicating sensor information to the user of the display device and/or receiving user inputs. In some embodiments, one, some, or all of the display devices is configured to display or otherwise communicate the sensor information as it is communicated from the sensor electronics module (e.g., in a data package that is transmitted to respective display devices), without any additional prospective processing required for calibration and real-time display of the sensor data.

It is noted here that in some cases the use of wires or wireless RF telemetry to communicate with sensors and user devices can lead to complications, particularly with regard to breakage, infection, and electrical noise. In some cases, the conductive properties of the body can be used to allow wireless communications with implanted devices. Such methods include, e.g., capacitive interbody communications which allows surface based communication between transmitters and receivers placed on or near the skin. Galvanic interbody communications may also be employed which have particular advantages of low power requirements. In one particular implementation, galvanic coupling may be employed to transmit signals from an implanted device to electrodes on the skin. Galvanic coupling may also be employed to communicate between devices mounted on the skin.

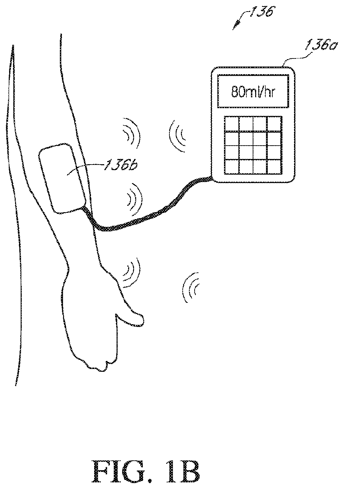

Returning to the discussion of FIG. 1A, the medical device 136 may be a passive device in example embodiments of the disclosure. For example, medical device 136 may be an insulin pump for administering insulin to a user, as shown in FIG. 1B. For a variety of reasons, it may be desirable for such an insulin pump to receive and track glucose values transmitted from analyte sensor system 8. One reason is to provide the insulin pump a capability to suspend or activate insulin administration when a glucose value falls below a threshold value. One solution that allows a passive device (e.g., medical device 136) to receive analyte data (e.g., glucose values) without being bonded to analyte sensor system 8 is to include the analyte data in the advertisement messages transmitted from analyte sensor system 8. The data included in the advertisement messages can be encoded so that only a device that has the identification information associated with analyte sensor system 8 can decode the analyte data. In some embodiments, the medical device 136 includes a sensor apparatus 136b, e.g., attachable or wearable by the user, in wired or wireless communication with a dedicated monitor or display apparatus 136a to process sensor data and/or display data from the sensor apparatus 136a and/or receive input for operation of the sensor apparatus and/or data processing.

With further reference to FIG. 1A, the plurality of display devices may include a custom display device specially designed for displaying certain types of displayable sensor information associated with analyte data received from sensor electronics module 12 (e.g., a numerical value and an arrow, in some embodiments). Analyte display device 110 is an example of such a custom device. In some embodiments, one of the plurality of display devices is smartphone, such as mobile phone 120 based on an Android, iOS or other operating system, and configured to display a graphical representation of the continuous sensor data (e.g., including current and historic data). Other display devices can include other hand-held devices, such as tablet 130, smart watch 140, medical device 136 (e.g., an insulin delivery device or a blood glucose meter), and/or a desktop or laptop computer.

Because different display devices provide different user interfaces, content of the data packages (e.g., amount, format, and/or type of data to be displayed, alarms, and the like) can be customized (e.g., programmed differently by the manufacture and/or by an end user) for each particular display device. Accordingly, in the embodiment of FIG. 1A, a plurality of different display devices can be in direct wireless communication with a sensor electronics module (e.g., such as an on-skin sensor electronics module 12 that is physically connected to the continuous analyte sensor 10) during a sensor session to enable a plurality of different types and/or levels of display and/or functionality associated with the displayable sensor information, which is described in more detail elsewhere herein.

As further illustrated in FIG. 1A, system 100 may also include wireless access point (WAP) 138 that may be used to couple one or more of analyte sensor system 8, the plurality display devices, server system 134, and medical device 136 to one another. For example, WAP 138 may provide Wi-Fi and/or cellular connectivity within system 100. Near Field Communication (NFC) may also be used among devices of system 100. Server system 134 may be used to collect analyte data from analyte sensor system 8 and/or the plurality of display devices, for example, to perform analytics thereon, generate universal or individualized models for glucose levels and profiles, and so on.

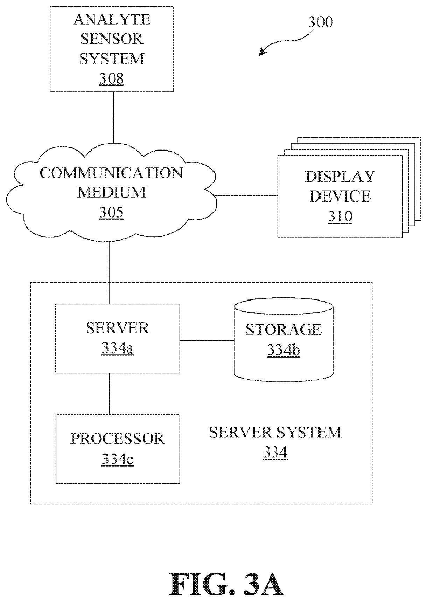

Referring now to FIG. 3A, system 300 is depicted. System 300 may be used in connection with implementing embodiments of the disclosed systems, methods, and devices. By way of example, the various below-described components of FIG. 3A may be used to provide wireless communication of glucose data, for example between an analyte sensor system and a plurality of display devices, medical devices, servers and so on, such as those shown in FIG. 1A.

As shown in FIG. 3A, system 300 may include analyte sensor system 308 and one or more display devices 310. Additionally, in the illustrated embodiment, system 300 includes server system 334, which in turn includes server 334a coupled to processor 334c and storage 334b. Analyte sensor system 308 may be coupled to display devices 310 and/or server system 334 via communication medium 305.

As will be described in detail herein, analyte sensor system 308 and display devices 310 may exchange messaging via communication medium 305, and communication medium 305 may also be used to deliver analyte data to display devices 310 and/or server system 334. As alluded to above, display devices 310 may include a variety of electronic computing devices, such as, for example, a smartphone, tablet, laptop, wearable device, etc. Display devices 310 may also include analyte display device 110 and medical device 136. Here, it will be noted that a GUI of display device 310 may perform such functions as accepting user input and displaying menus as well as information derived from analyte data. The GUI may be provided by various operating systems known in the art, such as, for example, iOS, Android, Windows Mobile, Windows, Mac OS, Chrome OS, Linux, Unix, a gaming platform OS (e.g., Xbox, Play Station, Wii), etc. In various embodiments, communication medium 305 may be based on one or more wireless communication protocols such as Bluetooth, Bluetooth Low Energy (BLE), ZigBee, Wi-Fi, 802.11 protocols, Infrared (IR), Radio Frequency (RF), 2G, 3G, 4G, etc., and/or wired protocols and media.

In various embodiments, the elements of system 300 may be used to perform various processes described herein and/or may be used to execute various operations described herein with regard to one or more disclosed systems and methods. Upon studying the present disclosure, one of skill in the art will appreciate that system 300 may include multiple analyte sensor systems, communication media 305, and/or server systems 334.

As mentioned, communication medium 305 may be used to connect or communicatively couple analyte sensor system 308, display devices 310, and/or server system 334 to one another or to a network, and communication medium 305 may be implemented in a variety of forms. For example, communication medium 305 may include an Internet connection, such as a local area network (LAN), a wide area network (WAN), a fiber optic network, internet over power lines, a hard-wired connection (e.g., a bus), and the like, or any other kind of network connection. Communication medium 305 may be implemented using any combination of routers, cables, modems, switches, fiber optics, wires, radio (e.g., microwave/RF links), and the like. Further, communication medium 305 may be implemented using various wireless standards, such as Bluetooth^{®}, BLE, Wi-Fi, 3GPP standards (e.g., 2G GSM/GPRS/EDGE, 3G UMTS/CDMA2000, or 4G LTE/LTE-U), etc. Upon reading the present disclosure, one of skill in the art will recognize other ways to implement communication medium 305 for communications purposes.

Server 334a may receive, collect, or monitor information, including analyte data and related information, from analyte sensor system 308 and/or display device 310, such as input responsive to the analyte data or input received in connection with an analyte monitoring application running on analyte sensor system or display device 310. In such cases, server 334a may be configured to receive such information via communication medium 305. This information may be stored in storage 334b and may be processed by processor 334c. For example, processor 334c may include an analytics engine capable of performing analytics on information that server 334a has collected, received, etc. via communication medium 305. In embodiments, server 334a, storage 334b, and/or processor 334c may be implemented as a distributed computing network, such as a Hadoop^{®} network, or as a relational database or the like.

Server 334a may include, for example, an Internet server, a router, a desktop or laptop computer, a smartphone, a tablet, a processor, a module, or the like, and may be implemented in various forms, including, for example, an integrated circuit or collection thereof, a printed circuit board or collection thereof, or in a discrete housing/package/rack or multiple of the same. In embodiments, server 334a at least partially directs communications made over communication medium 305. Such communications include the delivery and/or messaging (e.g., advertisement, command, or other messaging) and analyte data. For example, server 334a may process and exchange messages between analyte sensor system 308 and display devices 310 related to frequency bands, timing of transmissions, security, alarms, and so on. Server 334a may update information stored on analyte sensor system 308 and/or display devices 310, for example, by delivering applications thereto. Server 334a may send/receive information to/from analyte sensor system 308 and/or display devices 310 in real time or sporadically. Further, server 334a may implement cloud computing capabilities for analyte sensor system 308 and/or display devices 310.

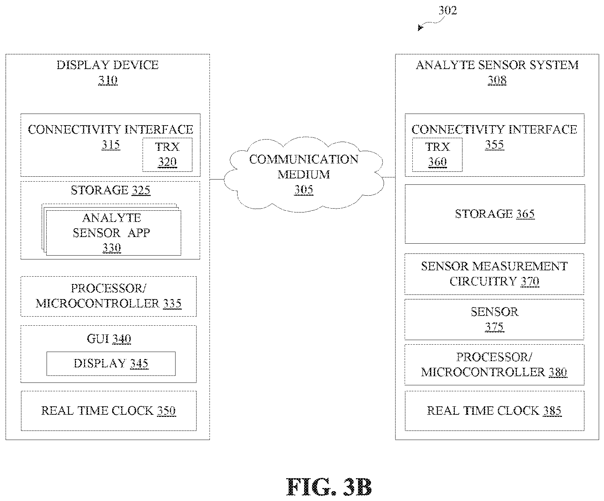

FIG. 3B depicts system 302, which includes examples of additional aspects of the present disclosure that may be used in connection implementing an analyte sensor system. As illustrated, system 302 may include analyte sensor system 308. As shown, analyte sensor system 308 may include analyte sensor 375 (e.g., which may also be designated with the numeral 10 in FIG. 1A) coupled to sensor measurement circuitry 370 for processing and managing sensor data. Sensor measurement circuitry 370 may be coupled to processor/microprocessor 380 (e.g., which may be part of item 12 in FIG. 1A). In some embodiments, processor 380 may perform part or all of the functions of the sensor measurement circuitry 370 for obtaining and processing sensor measurement values from sensor 375. Processor 380 may be further coupled to a radio unit or transceiver 320 (e.g., which may be part of item 12 in FIG. 1A) for sending sensor data and receiving requests and commands from an external device, such as display device 310, which may be used to display or otherwise provide the sensor data (or analyte data) to a user. As used herein, the terms "radio unit" and "transceiver" are used interchangeably and generally refer to a device that can wirelessly transmit and receive data. Analyte sensor system 308 may further include storage 365 (e.g., which may be part of item 12 in FIG. 1A) and real time clock (RTC) 380 (e.g., which may be part of item 12 in FIG. 1A) for storing and tracking sensor data.

As alluded to above, wireless communication protocols may be used to transmit and receive data between analyte sensor system 308 and the display device 310 via communication medium 305. Such wireless protocols may be designed for use in a wireless network that is optimized for periodic and small data transmissions (that may be transmitted at low rates if necessary) to and from multiple devices in a close range (e.g., a personal area network (PAN)). For example, one such protocol may be optimized for periodic data transfers where transceivers may be configured to transmit data for short intervals and then enter low power modes for long intervals. The protocol may have low overhead requirements both for normal data transmissions and for initially setting up communication channels (e.g., by reducing overhead) to reduce power consumption. In some embodiments, burst broadcasting schemes (e.g., one way communication) may be used. This may eliminate overhead required for acknowledgement signals and allow for periodic transmissions that consume little power.

The protocols may further be configured to establish communication channels with multiple devices while implementing interference avoidance schemes. In some embodiments, the protocol may make use of adaptive isochronous network topologies that define various time slots and frequency bands for communication with several devices. The protocol may thus modify transmission windows and frequencies in response to interference and to support communication with multiple devices. Accordingly, the wireless protocol may use time and frequency division multiplexing (TDMA) based schemes. The wireless protocol may also employ direct sequence spread spectrum (DSSS) and frequency-hopping spread spectrum schemes. Various network topologies may be used to support short-distance and/or low-power wireless communication such as peer-to-peer, start, tree, or mesh network topologies such as Wi-Fi, Bluetooth and Bluetooth Low Energy (BLE). The wireless protocol may operate in various frequency bands such as an open ISM band such as 2.4 GHz. Furthermore, to reduce power usage, the wireless protocol may adaptively configure data rates according to power consumption.

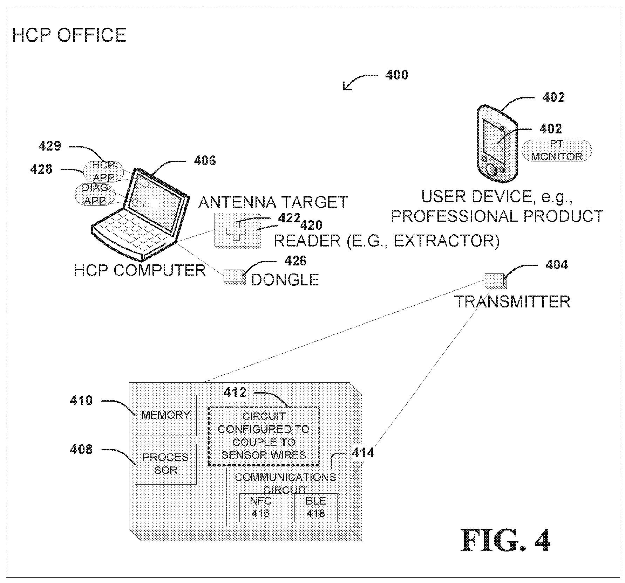

With further reference to FIG. 3B, system 302 may include display device 310 communicatively coupled to analyte sensor system 308 via communication medium 305. In the illustrated embodiment, display device 310 includes connectivity interface 315 (which in turn includes transceiver 320), storage 325 (which in turn stores analyte sensor application 330 and/or additional applications), processor/microprocessor 335, graphical user interface (GUI) 340 that may be presented using display 345 of display device 310, and real time clock (RTC) 350. A bus (not shown here) may be used to interconnect the various elements of display device 310 and transfer data between these elements. Wireless communications between elements may also be employed, e.g., wireless communications between the display device 310 and the analyte sensor 308. In a particular implementation, NFC may be employed as such a wireless communication scheme. Additional details of communications techniques between the sensor electronics and the receiver or display devices are described below in the context of Fig. 4 et seq.

Display device 310 may be used for alerting and providing sensor information or analyte data to a user, and may include a processor/microprocessor 335 for processing and managing sensor data. Display device 310 may include display 345, storage 325, analyte sensor application 330, and real time clock 350 for displaying, storing, and tracking sensor data. Display device 310 may further include a radio unit or transceiver 320 coupled to other elements of display device 310 via connectivity interface 315 and/or a bus. Transceiver 320 may be used for receiving sensor data and for sending requests, instructions, and/or data to analyte sensor system 308. Transceiver 320 may further employ a communication protocol. Storage 325 may also be used for storing an operating system for display device 310 and/or a custom (e.g., proprietary) application designed for wireless data communication between a transceiver and display device 310. Storage 325 may be a single memory device or multiple memory devices and may be a volatile or non-volatile memory for storing data and/or instructions for software programs and applications. The instructions may be executed by processor 335 to control and manage transceiver 320.

In some embodiments, when a standardized communication protocol is used, commercially available transceiver circuits may be utilized that incorporate processing circuitry to handle low level data communication functions such as the management of data encoding, transmission frequencies, handshake protocols, and the like. In these embodiments, processor 335, 380 does not need to manage these activities, but rather provides desired data values for transmission, and manages high level functions such as power up or down, set a rate at which messages are transmitted, and the like. Instructions and data values for performing these high level functions can be provided to the transceiver circuits via a data bus and transfer protocol established by the manufacturer of the transceiver 320, 360.

Components of analyte sensor system 308 may require replacement periodically. For example, analyte sensor system 308 may include an implantable sensor 375 that may be attached to a sensor electronics module that includes sensor measurement circuitry 370, processor 380, storage 365, and transceiver 360, and a battery (not shown). Sensor 375 may require periodic replacement (e.g., every 7 to 30 days). The sensor electronics module may be configured to be powered and active for much longer than sensor 375 (e.g., for three to six months or more) until the battery needs replacement. Replacing these components may be difficult and require the assistance of trained personnel. Reducing the need to replace such components, particularly the battery, significantly improves the convenience and cost of using analyte sensor system 308, including to the user. In some embodiments, when a sensor electronic module is used for the first time (or reactivated once a battery has been replaced in some cases), it may be connected to sensor 375 and a sensor session may be established. As will be further described below, there may be a process for initially establishing communication between display device 310 and the sensor electronics module when the module is first used or re-activated (e.g., the battery is replaced). Once display device 310 and sensor electronics module have established communication, display device 310 and the sensor electronics module may periodically and/or continuously be in communication over the life of several sensors 375 until, for example, the battery needs to be replaced. Each time sensor 375 is replaced, a new sensor session may be established. The new sensor session may be initiated through a process completed using display device 310 and the process may be triggered by notifications of a new sensor via the communication between the sensor electronics module and display device 310 that may be persistent across sensor sessions.

Analyte sensor system 308 typically gathers analyte data from sensor 375 and transmits the same to display device 310. Data points regarding analyte values may be gathered and transmitted over the life of sensor 375 (e.g., in the range of 1 to 30 days or more). New measurements may be transmitted often enough to adequately monitor glucose levels. Rather than having the transmission and receiving circuitry of each of analyte sensor system 308 and display device 310 continuously communicating, analyte sensor system 308 and display device 310 may regularly and/or periodically establish a communication channel between them. Thus, analyte sensor system 308 can in some cases communicate via wireless transmission with display device 310 (e.g., a hand-held computing device, medical device, or proprietary device) at predetermined time intervals. The duration of the predetermined time interval can be selected to be long enough so that analyte sensor system 308 does not consume too much power by transmitting data more frequently than needed, yet frequent enough to provide substantially real-time sensor information (e.g., measured glucose values or analyte data) to display device 310 for output (e.g., via display 345) to a user. While the predetermined time interval is every five minutes in some embodiments, it is appreciated that this time interval can be varied to be any desired length of time.

With continued reference to FIG. 3B, as shown, connectivity interface 315 interfaces display device 310 to communication medium 305, such that display device 310 may be communicatively coupled to analyte sensor system 308 via communication medium 305. Transceiver 320 of connectivity interface 315 may include multiple transceiver modules operable on different wireless standards. Transceiver 320 may be used to receive analyte data and associated commands and messages from analyte sensor system 308. Additionally, connectivity interface 315 may in some cases include additional components for controlling radio and/or wired connections, such as baseband and/or Ethernet modems, audio/video codecs, and so on.

Storage 325 may include volatile memory (e.g. RAM) and/or non-volatile memory (e.g. flash storage), may include any of EPROM, EEPROM, cache, or may include some combination/variation thereof. In various embodiments, storage 325 may store user input data and/or other data collected by display device 310 (e.g., input from other users gathered via analyte sensor application 330). Storage 325 may also be used to store volumes of analyte data received from analyte sensor system 308 for later retrieval and use, e.g., for determining trends and triggering alerts. Additionally, storage 325 may store analyte sensor application 330 that, when executed using processor 335, for example, receives input (e.g., by a conventional hard/soft key or a touch screen, voice detection, or other input mechanism), and allows a user to interact with the analyte data and related content via GUI 340, as will be described in further detail herein.

In various embodiments, a user may interact with analyte sensor application 330 via GUI 340, which may be provided by display 345 of display device 310. By way of example, display 345 may be a touchscreen display that accepts various hand gestures as inputs. Application 330 may process and/or present analyte-related data received by display device 310, according to various operations described herein, and present such data via display 345. Additionally, application 330 may be used to obtain, access, display, control, and/or interface with analyte data and related messaging and processes associated with analyte sensor system 308, as is described in further detail herein.

Application 330 may be downloaded, installed, and initially configured/setup on display device 310. For example, display device 310 may obtain application 330 from server system 334, or from another source accessed via a communication medium (e.g., communication medium 305), such as an application store or the like. Following installation and setup, application 330 may be used to access and/or interface with analyte data (e.g., whether stored on server system 334, locally from storage 325, or from analyte sensor system 308). By way of illustration, application 330 may present a menu that includes various controls or commands that may be executed in connection with the operating of analyte sensor system 308 and one or more display devices 310. Application 330 may also be used to interface with or control other display devices 310, for example, to deliver or make available thereto analyte data, including for example by receiving/sending analyte data directly to the other display device 310 and/or by sending an instruction for analyte sensor system 308 and the other display device 310 to be connected, etc., as will be described herein. In some implementations, application 330 may interact with other application(s) of the display device to retrieve or provide relevant data, e.g., such as other health data.

Analyte sensor application 330 may include various code/functional modules, such as, for example, a display module, a menu module, a list module, and so on as will become clear in light of the description of various functionalities herein (e.g., in connection with disclosed methods). These modules may be implemented separately or in combination. Each module may include computer-readable media and have computer-executable code stored thereon, such that the code may be operatively coupled to and/or executed by processor 335 (which, e.g., may include a circuitry for such execution) to perform specific functions (e.g., as described herein with regard to various operations and flow charts etc.) with respect to interfacing with analyte data and performing tasks related thereto. As will be further described below, a display module may present (e.g., via display 345) various screens to a user, with the screens containing graphical representations of information provided by application 330. In further embodiments, application 330 may be used to display to the user an environment for viewing and interacting with various display devices that may be connectable to analyte sensor system 308, as well as with analyte sensor system 308 itself. Sensor application 330 may include a native application modified with a software design kit (e.g., depending on the operating system) in order to carry out the functionalities/features described herein.

Referring again to FIG. 3B, display device 310 also includes processor 335. Processor 335 may include processor sub-modules, including, by way of example, an applications processor that interfaces with and/or controls other elements of display device 310 (e.g., connectivity interface 315, application 330, GUI 340, display 345, RTC 350, etc.). Processor 335 may include a controller and/or microcontroller that provides various controls (e.g., interfaces with buttons and switches) related to device management, such as, for example, lists of available or previously paired devices, information related to measurement values, information related to network conditions (e.g., link quality and the like), information related to the timing, type, and/or structure of messaging exchanged between analyte sensor system 308 and display device 310, and so on. Additionally, the controller may include various controls related to the gathering of user input, such as, for example, a user's finger print (e.g., to authorize the user's access to data or to be used for authorization/encryption of data, including analyte data), as well as analyte data.

Processor 335 may include circuitry such as logic circuits, memory, a battery and power circuitry, and other circuitry drivers for periphery components and audio components. Processor 335 and any sub-processors thereof may include logic circuits for receiving, processing, and/or storing data received and/or input to display device 310, and data to be transmitted or delivered by display device 310. Processor 335 may be coupled by a bus to display 345 as well as connectivity interface 315 and storage 325 (including application 330). Hence, processor 335 may receive and process electrical signals generated by these respective elements and thus perform various functions. By way of example, processor 335 may access stored content from storage 325 at the direction of application 330, and process the stored content for display and/or output by display 345. Additionally, processor 335 may process the stored content for transmission via connectivity interface 315 and communication medium 305 to other display devices 310, analyte sensor system 308, or server system 334. Display device 310 may include other peripheral components not shown in detail in FIG. 3B.

In further embodiments, processor 335 may further obtain, detect, calculate, and/or store data input by a user via display 345 or GUI 340, or data received from analyte sensor system 308 (e.g., analyte sensor data or related messaging), over a period of time. Processor 335 may use this input to gauge the user's physical and/or mental response to the data and/or other factors (e.g., time of day, location, etc.). In various embodiments, the user's response or other factors may indicate preferences with respect to the use of certain display devices 310 under certain conditions, and/or the use of certain connection/transmission schemes under various conditions, as will be described in further detail herein.

It should be noted at this juncture that like-named elements as between display device 310 and analyte sensor system 308 may include similar features, structures, and/or capabilities. Therefore, with respect to such elements, the description of display device 310 above may in some cases be applied to analyte sensor system 308.

Monitoring and gathering patients' diabetes information by a HCP, and at the same time helping patients to manage their disease, can be difficult, because of lack of user-friendly (for both HCP and patient), and cost-effective products. Even where such devices have been provided, it is generally desired to have significant involvement of a HCP in the set up thereof, particularly for patients unaccustomed to such procedures. These goals are complicated by the proliferation of user mobile devices, which provide a great degree of convenience for users/patients, but it is difficult for an HCP to have mastery of all such products, and all such apps that may be installed on such products.

In one implementation, and referring to the system 400 of Fig. 4, which is intended to show an arrangement of devices within an HCP office, a low cost, programmable, and easy to use product may be provided, which is termed herein a "professional product" 402. This product may then be provided on loan to the patient, who may return it at the end of the sensor session. The professional product 402 may include similar functionality as other mobile devices, e.g., smart phones, and includes functionality of communicating with a transmitter 404 via an appropriate communications protocol. The professional product 402, being provided to the HCP, will be familiar to the HCP, who thus need not learn an entirely new device. The professional product 402 may also be embodied in one implementation by an HCP device such as an HCP computer 406 operating an HCP app 429 or a mobile device (such as smartphone) configured to operate HCP apps. In this implementation, the patient is not provided with a device to provide data display during their session, or alternatively they use their smart phone for such where appropriate communications have been enabled (see below). In some examples, the HCP device/HCP smartphone or the HCP professional product may be a locked-down smart phone (e.g., a dedicated smartphone) provided by a diabetes management/medical device company to the HCP and operated by the HCP or one or more personnel in the HCP office. In such examples, the HCP device may have dedicated applications pre-installed in the HCP device that may be operated by the HCP to perform certain operations, such as performing transmitter and/or sensor verification, user or patient account setup, downloading data from the transmitter, etc. as described herein. Alternatively, HCP may download the dedicated app from a server. The app may provide guidance to the HCP as to how to operate the HCP device to carry out the operations (e.g., setting up a user/patient account, sensor and/or transmitter verification, data download from the transmitter, etc.). As further described herein, The HCP device may be equipped with the NFC and BLE wireless technologies to carry out the operations.

To enable communications between the transmitter and the professional product 402, any desired wireless scheme may be employed, but it may be convenient to employ technologies such as near field communications (NFC), Bluetooth^{®} including Bluetooth^{®} low energy (BLE) and the like. It will be understood, however, that other communications modes are possible, including the use of cellular technologies, Wi-Fi technologies, and so on, so long as appropriate security measures are taken for secure communications, including encryption.

Where a separate professional product mobile device is employed, the HCP device 406 may employ one or more devices enabling communications with the professional product 402, e.g., a reader 420, which may in some cases be embodied in a dongle 426. As technologies such as NFC require particularly close proximity between a reader and the professional product, a target 422 may be displayed on the reader 420 in order to indicate the location of the antenna. A similar target may be placed on the professional product 402, or in the case where an HCP uses their own smart phone or other mobile device, the HCP may be aware, or may become aware of, where the NFC antenna is located. In addition, in some implementations, the dongle or the reader may include both NFC and BLE radios.

Use of such a reader 420 in lieu of e.g., an HCP smart phone, provides various benefits. The same is generally lower cost, it is wired to the HCP device, so no charging is required, and it may have a larger coil for NFC pairing. In many cases it is preferred by the HCP as a device that is not likely to be easily lost, it has a longer lifespan, it is conveniently configurable to provide confirmation of sensor function, and is wired to the HCP device and thus does not require a separate pairing action.

The use of an HCP reader may provide other benefits as well. For example, where a user patient lacks a smart phone, or lacks a smart phone that is otherwise compatible with the analyte monitoring system, the HCP reader 420 or the HCP user device 402 may be employed to set up the transmitter 404 in order to initiate or activate the patient's device, as well as to download data from the user's device at the end of the session.

The HCP device 406 or the HCP user device 402 may access an HCP portal 429, which may be a web app, and which is in this case generally a portal in communication with a server on which the user has a user account associated with their disease management. The HCP device 406 or the HCP user device 402 may also run a stand-alone application (HCP app) in network communication with the server, and which communicates with a mobile phone of the user and/or the transmitter 404. In this specification the term "HCP app" is used to describe both an HCP portal, which can be a web app, as well as a standalone app.

The HCP app may be employed to enter data in fields for the set up of a user account, and may further be employed for diagnostic functionality including to confirm that the sensor is working, e.g., that the sensor and transmitter are delivering counts to a professional product (e.g., HCP professional product 402) or other device, or that the sensor and transmitter are receiving a predetermined number of counts over a predetermined time. Details of such functionality are provided below. The HCP app can further confirm the sensor is working by way of a visual indicator on the app and/or an audible beep, e.g., within several minutes of application on a patient. The HCP app may further be employed to extract data from the sensor. For example, the HCP app can work with an HCP device (e.g., the HCP professional product 402) or an HCP reader to pair with the sensor/transmitter, and to extract the data within a few seconds or minutes' time. A visual indicator and/or an audible beep may be provided when the data transfer is complete. The HCP app may then upload data to the server, and provide an indication of successful data uploading. The HCP app as a further function may cause the deletion of patient data from the transmitter once the same is securely stored on the HCP device or on a server. The HCP app may store data for multiple patients at one time. In some cases, the HCP app may incorporate a limited viewing functionality of patient data; a primary purpose may be to collect data, upload the same, and delete the data following upload. The HCP app may have various primary icons or pages, relating to accounts set up, sensor confirmation of operation, data extraction, and list of patient data statuses.

The transmitter 404 has been described above, but for purposes of this discussion, it will be noted that the same generally includes a processor 408 as well as a memory (or other storage) 410. The transmitter 404 includes a circuit 412 configured to couple to sensor wires. The circuit 412 generally includes contacts which, when the transmitter is inserted in the sensor housing, couple electrically to the two wires forming the sensor. In one implementation, these two wires are coaxial. In another, they are side-by-side. The transmitter further includes a communications circuit 414 through which the transmitter can communicate, e.g., to the HCP device 406, to the professional product 402, or to other devices, including other user devices such as personal smart phones or the like. The transmission circuit 414 may include provisions such as circuitry or antennas for NFC communications 416, as well as circuitry or antennas for BLE communications 418. It will be understood that circuitry including antennas may be provided for other modes of communication as well, both wired and wireless.

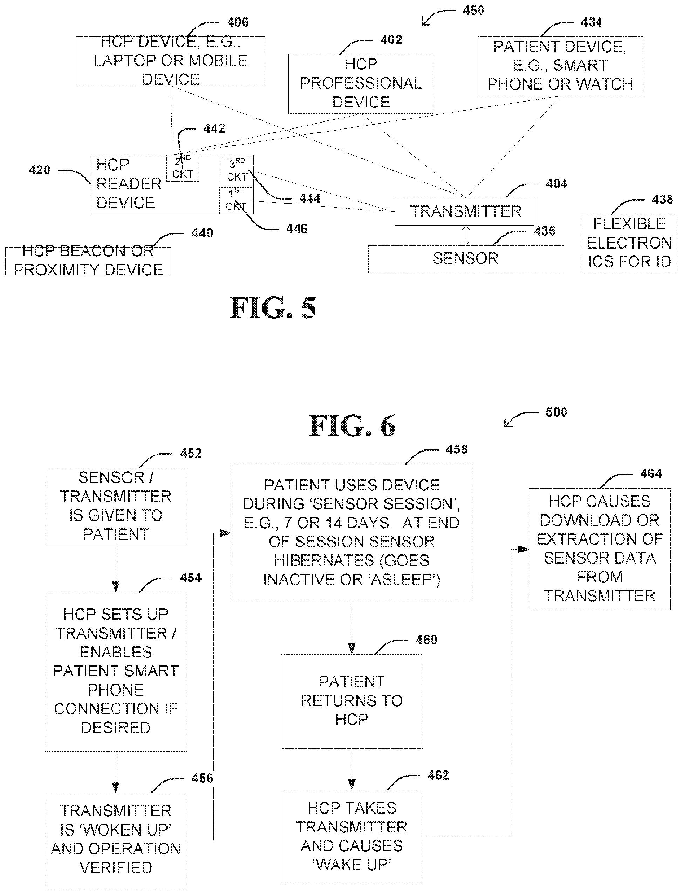

The system 450 of Fig. 5 illustrates another view of the devices often within the HCP office, including communication paths. This figure also shows other devices that may be employed in the system, including a patient personal device 434 such as a smart phone. Fig. 5 shows the transmitter 404 in communication with the indwelling sensor 436 within the patient, as well as a flexible electronics component 438. The flexible electronics component may be provided, e.g., as a sticker provided in (or with) the sensor and/or transmitter package, and the same may be conveniently employed for providing an identification element for the transmitter. Such identification elements may include sensor ID or may also include additional manufacturing information. The flexible electronics component 438 may be scanned by the reader 420 and/or the professional product 402 or patient device 434, such that the transmitter identification for that session may be associated with the patient user account. It will be understood that in other implementations, passive codes may be employed which may then subsequently be read by the reader 420 and/or the professional product 402 or patient device 434, e.g., barcodes, QR codes, or the like.

The flexible electronics component 438 may be conveniently employed as noted to simplify a transmitter pairing process, e.g., transmitter pairing with a professional product or a patient device, e.g., via a wireless link such as NFC or BLE, described in greater detail below.

Where the flexible electronics is provided as a sticker, the same may be preprogrammed with the transmitter pairing ID in placed in or on the transmitter packaging. Upon receipt, the patient may touch or tap their receiver or phone to the package, and the transmitter pairing ID would be read and automatically populated into the pairing device. Such could also allow for background capture of patient data, including, e.g., the date of transmitter activation. A similar concept may be implemented within the context of the sensor patch. For example, each patch may contain the NFC electronics, may be pre-populated with information such as lot or expiration date, and upon insertion into the housing, the transmitter could verify that the sensor is within its "safe to use" window, and may transmit this information to the cloud, e.g., through one of the connected devices, e.g., the reader 420, the professional product 402, the patient device 434, or even the HCP device 406. Such information would allow technical support functionality to access the information, streamlining troubleshooting, and allow the monitoring of field failures by lot for use in process optimization.

The HCP device 406 may include, e.g., a laptop or desktop computer employed by the HCP for patient treatment, but may also include a mobile device employed by the HCP for such purposes. Generally the HCP device 406 may include some way of communicating with a transmitter 404.

The HCP reader device 420 may include various circuits 442 - 446 to allow communications with various types of devices, and more particularly with devices employing various types of communications schemes. For example, a first circuit may be a wireless USB circuit, the second circuit may be an NFC circuit, and a third circuit may be a BLE circuit.

Fig. 5 also shows an HCP beacon/proximity device 440 disposed in communication with the various devices. The device 440 may include a wireless device that is disposed in a fixed location like an HCP office and which broadcasts a fixed piece of data via BLE. When the patient arrives at the HCP office for a review, e.g., a post-CGM 14-day review, the transmitter 404 or the professional product 402 or the patient device 434 could detect this beacon signal and trigger an automatic download of accumulated stored analyte concentration data for access by the HCP through the HCP app 429. The HCP app 429 may also be included in the HCP professional device 402. In such a scenario, where required, the HCP beacon for 40 may be paired with the transmitter during the initial set up stage. Moreover, to initiate download, the beacon device 440 may also need to be in communication with the HCP device 406 (or 402), and may provide notification to this device upon detecting that the transmitter has arrived back in the HCP office.
Figure 6 is a flowchart 500 illustrating an overall method in which implementations of the present arrangements may be situated. This figure describes in a summary fashion what will be described in greater detail throughout the remainder of the specification, and with reference to the elements of Fig. 5.

In a first step, a sensor/transmitter is given to a patient/user (step 452). Such is generally given as a kit, and in many cases a transmitter will be reusable, and thus it is only the sensor that is provided. As noted above, however, a flexible electronic circuit may be provided by way of a sticker or the like to provide easily readable identifying information about the sensor or the transmitter or both. Such information may also be provided as part of a barcode, QR code, or the like.

In a next step, if the user is unfamiliar with set up, an HCP may set up the transmitter and may further configure or otherwise enable a connection between the transmitter and the professional product, or smart phone if the user desires to use the same (step 454). Details of this configuration are provided below in connection with Fig. 4 - 20 and accompanying text.

The transmitter is then "woken up" or otherwise caused to enter an active state from an inactive state, if the transmitter is currently in an inactive or "asleep" state (step 456). This step is optional but is often taken because transmitters, to conserve battery power, are often shipped in an inactive state. Details of how transmitters or cause to be woken up or rendered in an active state are described below in connection with Fig. 4 - 20, 28 - 31, and 33 - 36 and accompanying text.

The patient then uses the device during a sensor session (step 458). The sensor session may be, e.g., 7 days, 14 days, or as otherwise prescribed by the HCP. Following the end of this period, the transmitter may be caused to enter an inactive state. The transmitter may be so caused by expiration of the time period, removal from the sensor housing, removal of the entire sensor patch from the patient, and via other triggers.

At the time of the next visit with the HCP, the patient returns (step 460), and the HCP may take an action that causes the transmitter to "wake up" if such has entered an inactive state (step 462). The HCP may then cause download or extraction of sensor data from the transmitter (step 464). In some cases, the data may have been intermittently or continuously transmitted to a server, or to a professional product or user device, depending on the nature of the set up.

Details of each of these above steps are now described throughout the remainder of the specification.

Initially the HCP set up steps are described.

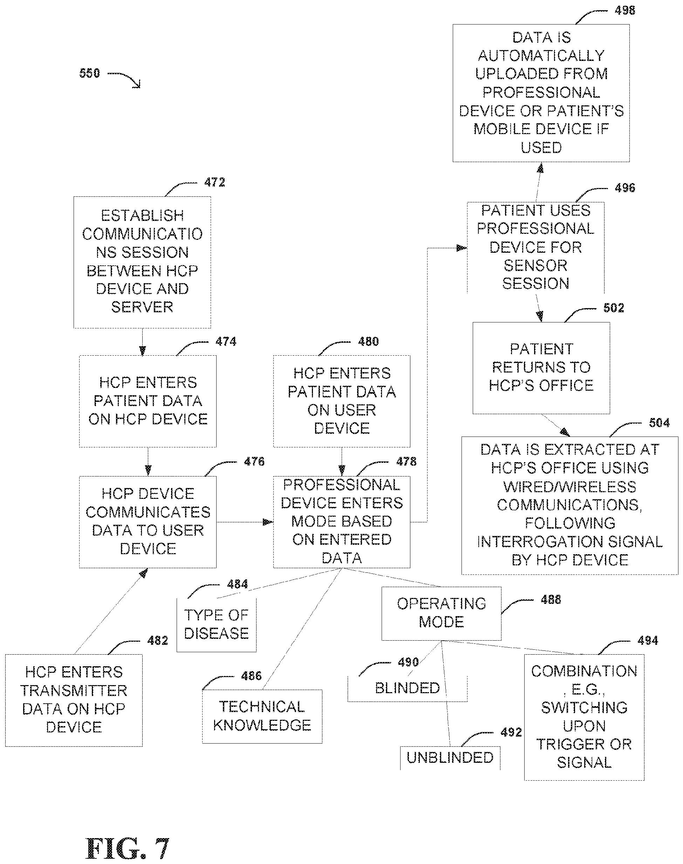

Referring to the flowchart 550 of Fig. 7, in a first step, a communication session is established between the HCP device and a server (step 472). Such may be done in a typical fashion, in which an HCP provides a username and password as well as in some implementations additional identifying information. The HCP may then enter patient information on the HCP device (step 474) or via the professional device. In some cases, particularly if a new transmitter is being employed, the HCP may enter transmitter data on the HCP device (step 482). This step may be employed using, e.g., the flexible electronic circuit noted above, barcodes, QR codes, or the like.

The data may then be communicated from the HCP device to the user device (step 476). The user device may then enter a given mode depending on the entered data (step 478).

In an alternative implementation, the HCP may enter the patient data directly on the user's mobile device (step 480).

Returning to step 478, the professional product may enter one or more different modes depending on the entered data. Such modes may be based on entries including, e.g., the type of disease (step 484), technical knowledge of the user (step 486), and operating mode (step 488). For example, the professional product may have a mode, typically varying by way of user interface, that is different as between a T-1 patient and a T-2 patient. The mode may also vary depending on the technical knowledge or savviness of the user. By programming the product according to the user's technical skill level or according to the disease type (T-1 or T-2 or prediabetic), the HCP can be confident that a proper UI will be presented to the user about their measured glucose level.

The mode may further vary depending on operating mode. While details are given below, here it is noted that in many cases, to avoid having user actions "contaminate" initial glucose concentration data, an HCP may desire that a user start in a blinded mode (step 490), so that the user is not tempted to take actions to affect their glucose data reflexively. While such actions are eventually desired, many HCP's want initially to see "where the user is at" in terms of glucose control, and a blinded mode is particularly useful for such endeavors. The mode may also be unblinded (step 492) in a different implementation, such that a user may see their current data and act on the same. Again, such is generally desired after the user has an idea of their current level of glycemic control. In some cases, a combination mode may be useful (step 494), such as where a user starts in a blinded mode, and upon the occurrence of a trigger event or the expiration of a time period, the system is configured to switch to an unblinded mode. For example, an HCP may desire that a user spends seven days in a blinded mode and seven days in an unblinded mode. Alternatively, the system may automatically switch to an unblinded mode once a user attains a current level of glycemic control, e.g., is able to control their glucose concentration to within a predetermined target range. In any case, such triggers and duration-based switching may occur automatically, and the system may be configured to provide such functionality.

The patient then uses the sensor, transmitter, and the professional product (or their own smart device) for the duration of the sensor session (step 496). The transmitter stores several weeks of data. In some cases, data may be automatically uploaded from the professional product or the patient's device (step 498) to a server associated with the patient's care or, e.g., an Electronic Medical Record (EMR).

In many cases, however, the patient must return to the HCP's office for extraction and analysis of the data (step 502). The data can generally be extracted in a very short time period, e.g., less than 5 or 10 seconds. The data may be extracted using wired or wireless communications, generally following some sort of interrogation signal by an HCP device (step 504). As part of this procedure, the transmitter may be woken up from an inactive state. Details of such wake-up techniques are provided below.

As part of the initial set up by the HCP, or as part of the data extraction, or even as part of a sensor session, various diagnostic routines can be run by the HCP device to confirm proper operation of the sensor and transmitter. In one implementation, and referring back to Fig. 4, such may be performed by a diagnostic application 428 on the HCP device. Moreover, the HCP may set up the user account as part of the initial set up or during the data extraction interview.

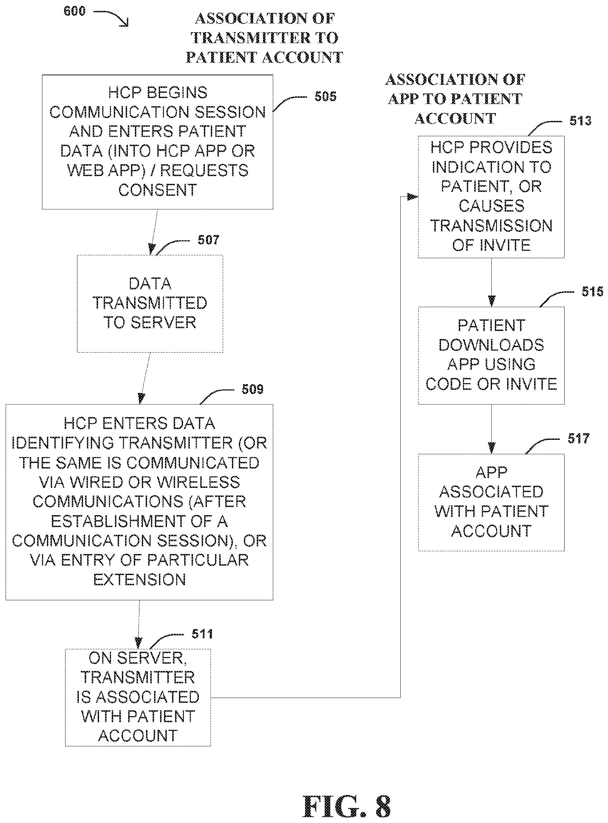

As noted above, the patient user may obtain the product at the HCP's office, but in many cases is unaware of how to obtain an account or set up the product. In one implementation, and referring to the flowchart 600 of Fig. 8, the HCP sets up the user account and the product for the user. The patient enters the HCP office, and the HCP provides, e.g., a transmitter package as noted above. Such a product includes a transmitter, a transmitter ID, e.g., using flexible electronics, or on the box or other portion of the product, and or optionally a professional product. The HCP begins a communication session and inquires about patient information, including, e.g., name, date of birth, email address, and so on, in order to enter the same into an HCP app (step 505). The HCP may verbally ask for patient consent for entering or using the patient's email address.

The HCP then enters patient information into the app or server portal, e.g., HCP portal, e.g., which may be a web app providing access to a server. The server is generally a cloud server, and in many cases, is also the recipient of patient data upon the patient's return, following the sensor session. The HCP app may also provide functionality including verification of sensor operation, data extraction, providing timestamp data to the transmitter, or the like. Whether by an HCP app or an HCP portal, the data is transmitted to a server (step 507). In some cases, the entered data may indicate a mode in which the transmitter is to be placed, and this mode may propagate through to the professional product or patient's smart device.

As part of the above steps, the HCP may enter data identifying the transmitter (step 509). Such may also be communicated via wired or wireless communications after establishment of a suitable pairing relationship and communication session.

In more detail, the HCP may manually enter transmitter information along with the patient information. The entered transmitter information may include a transmitter ID. By entering such information, the user account may be tied with the particular transmitter. If the HCP does not desire to enter such information, or does not desire to enter it manually, the same may be entered in the manner noted above, e.g., by use of a flexible electronic circuit which is scanned by an HCP receiver device, a QR code, a barcode, or the like. The HCP app, running on the HCP device, may also receive the transmitter ID by other means, and may communicate with the transmitter during the setup stage, e.g., via BLE or NFC. In any case, the HCP app retrieves the transmitter ID, e.g., serial number, and may cause the transmission of the same to the server.

On the server end, the transmitter is then associated with a patient account (or one is created) using the transmitter identification information (step 511). Once the account is created, the system may generate a unique code or token for the patient through which the patient may receive an app for their smart device or for their particular professional product. In more detail, the HCP may then provide an indication to the patient (step 513), e.g., a document or displayable file, the same including a code or token. The HCP may further provide instructions on how to download a monitoring or other associated app. The code may be scanned (or entered manually) and thereby configure an association between the patient and the downloaded app. That is, the code, after input into the smart device or professional product, may provide data such that the app is automatically associated with the patient, without the need for the patient to be burdened with additional data entry. In an alternative implementation, the HCP may cause the transmission of an invitation to the email address provided by the patient. It will be understood that such an invitation may also be sent via text message or the like. The patient may then download an application using the code (step 515). The application may be automatically associated with the patient account using the code (step 5 17). Alternatively, the app may be downloaded with the code entered after installation. In any case, the transmitter is linked to the user's account.

The HCP portal then links the patient information with the patient downloaded app. The HCP portal may further automatically generate another account for the patient, e.g., an account available for future purchase of sensors and transmitter products or the like. The opening of this other account may involve another email or text invite to the patient.

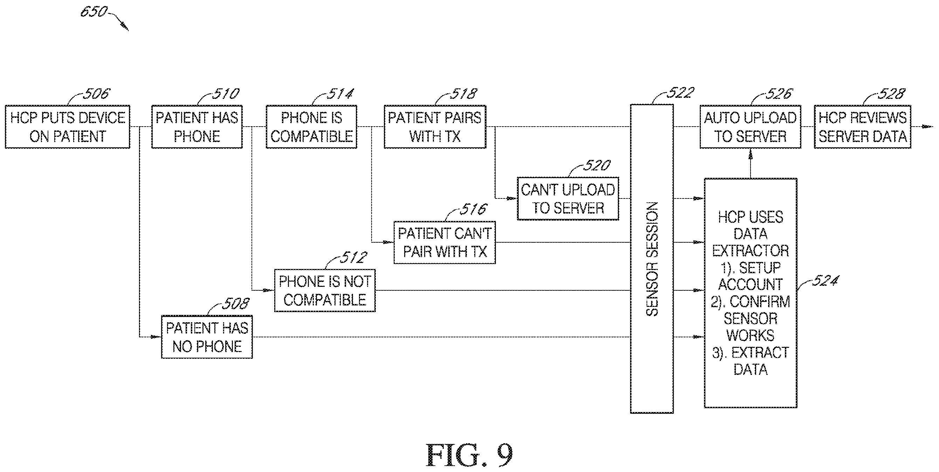

The flowchart 650 of Fig. 9 illustrates process flow within an HCP office in various use cases, with varying levels of user-provided technology. As may be seen from the flowchart, having a data extraction step, such as may be provided by an HCP reader or similar device, provides HCP reassurance of reimbursement regardless of scenario outcomes.

Instructions may be provided to the HCP on the HCP reader or other HCP device for performing this sequence of steps shown in this flowchart of those that follow. In this way the HCP may be led through the set-up process in a step by step manner. The instructions may be incorporated in the HCP app discussed above or they may be included in a separate, dedicated app. In addition, it should be noted that the HCP reader or other HCP device may include additional apps that are dedicated to tutorials regarding any of the various aspects of system usage and installation, and possibly even ancillary aspects such as reimbursement assistance and the like.

In a first step, the HCP provides a sensor and transmitter to the patient (step 506). If the patient has no phone (step 508), the sensor session may start (step 522), and following completion of the sensor session, the HCP may use the data extractor to perform steps including setting up an account, confirming that the sensor works, and extracting the data from the sensor (step 524).

If it is determined that the patient has a phone (step 510), and that the phone is compatible with the other components of the system (step 514), and that the phone may be appropriately paired with the transmitter (step 518), then the sensor session may simply proceed (step 522). In some cases the data may be automatically uploaded to the server (step 526), and the data reviewed by the HCP on the server (step 528).

If the patient does not have a smart phone (step 508), then the sensor session may still be conducted by collecting data on the transmitter and later extracting the same. Similarly, even if the phone is not compatible (step 512), the sensor session may still be conducted as data may still be collected by the transmitter, even if not transmitted to the user device right away. Even if the patient's phone cannot pair with the transmitter (step 516), such data may still be collected and later extracted. Finally, even if the pairing is successful but the data cannot be transmitted from the patient device for whatever reason (step 520), the data may still be stored for later extraction.

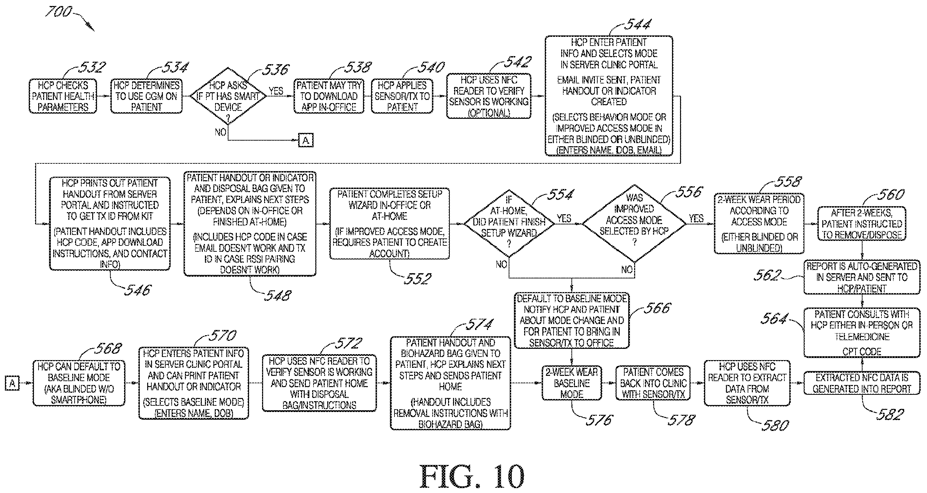

In any case, following conclusion of the sensor session, the patient may bring the transmitter back to the HCP office for data extraction (step 524).Fig. 10 is a flowchart 700 illustrating a more involved method according to present principles. In a first step, a patient who is considering CGM, or who has been told to consider it, may meet with an HCP and the same may perform various measurements (step 532), e.g., A1C and labs, so as to determine whether the patient is appropriate for and would benefit from CGM (step 534). To determine if the patient could monitor data from a CGM, the HCP may inquire as to whether the patient has a compatible smart device, e.g., smart watch, smart phone, tablet, and so on (step 536). The compatibility may be determined manually, but comparing the type of device, e.g., model number, with a list of compatible devices. Alternatively, the HCP may perform a compatibility check by, e.g., use of a test signal which provides a displayed result upon a successful (or unsuccessful) test of compatibility. In some cases the test signal may be generated by the HCP reader and transmitted to the user device via NFC or BLE. If the patient has a compatible device, the patient may try to download the monitoring app at the HCP office (step 538). The HCP may apply the sensor and transmitter to the patient (step 540). The HCP may then verify the sensor and transmitter are operational, e.g., using the HCP reader and, e.g., NFC (step 542) by, e.g., exchanging data with the transmitter over a wireless communications link, which may be implemented by way of NFC or BLE. Details of such verification are provided elsewhere. The HCP may then enter patient information and the HCP app or HCP server portal (step 544). As noted above, this step may include the sending of an email invitation using which the patient may download the monitoring app and/or set up a patient account. The HCP may also enter patient information by way of the HCP reader device.

The HCP may then print out a patient handout from the server portal and retrieve the transmitter ID from the kit. The transmitter ID may be embodied by the flexible electronics noted above, a barcode, a QR code, and so on (step 546). The patient handout and a disposal bag may be provided to the patient, and the HCP may explain subsequent steps (step 548). The patient may complete the setup wizard in the office or at home (step 552). If at home, a check may be provided as to whether the patient finished the setup wizard (step 554). If yes, the system may determine if a particular access mode was selected by the HCP (step 556). If the HCP did not select a particular access mode, or the patient did not finish the setup wizard at home, then the system may default to a baseline mode (step 566). In some cases, the baseline mode corresponds to a blinded mode. If the patient did finish the setup wizard, and the HCP did select an access mode, then the wear period may commence according to the access mode (step 558). For example, access modes may include mode such as blinded, unblinded, a combination (e.g., starting off in blinded but converting to unblinded at some point), and so on. In one implementation, the server can check if the setup wizard is not finished after a predetermined number of hours. A follow-up email can then be sent to the HCP or the patient.

Subsequent to the wear period, the patient may be instructed to remove the sensor and transmitter (step 560). Where a smart device has been paired with the sensor/transmitter, data transfer may have been occurring during the wear period, and thus a report may be auto generated by the server and sent to the HCP and/or the patient (step 562). The patient may then consult with the HCP either in person or via telemedicine (step 564).

Returning to step 536 noted above, if the patient does not have a compatible smart device, HCP may place the system in a default baseline mode, e.g., blinded without pairing with a smart phone (step 568). The HCP may enter the patient information in the HCP server clinic portal and again print out a patient handout (step 570). The HCP may employ the reader, e.g., with NFC, to verify that the sensor is working properly (step 572). Details of such verification steps are provided below with respect to the flowchart 800. The HCP may then send the patient home with a disposal bag and instructions (step 574). Again the wear period may commence (step 576). This step may also the default to baseline mode subsequent to step 566.

The patient may return with the sensor/transmitter in the disposal bag (step 578), and the HCP may use the reader to extract data from the sensor/transmitter (step 580). Subsequent to data extraction, the patient may consult with the HCP either in person or via telemedicine (step 564).

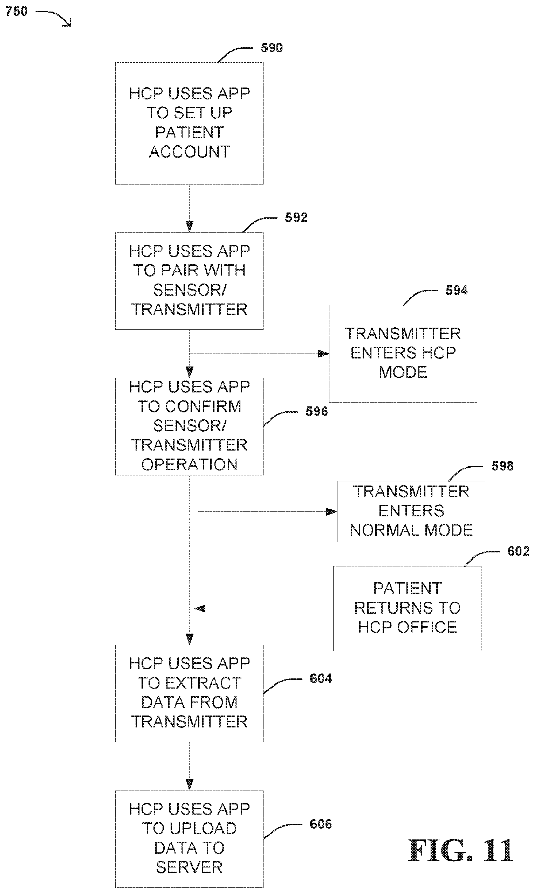

The flowchart 750 of Fig. 11 summarizes many of the actions of the HCP set-up procedure, particularly from the standpoint of the HCP application. In a first step, the HCP uses the HCP app to set up the patient account (step 590). The HCP may then use the app to provide a pairing step with the sensor transmitter (step 592), e.g., where the pairing occurs with the HCP device, with a professional product, or with a user smart device. The HCP app then may cause the transmitter to enter an HCP mode (step 594). In this mode the HCP may use the HCP app to confirm proper operation of the sensor and transmitter (step 596). This mode may be enabled by enabling a communications protocol that only allows one connection, or by blocking out other communication requests from other devices, and so on. In any case, in this mode, the HCP app may be provided with significantly greater permissions and privileges so as to allow configuration of the professional product or user smart device and yet subsequently allow the device to go back to a more typical mode, where such configuration details are protected from user alteration. Subsequent to confirmation of proper operation, the transmitter may be caused to enter a normal mode (step 598). The term "normal mode" is used to denote the mode in which the wear period occurs, and the same may be used to encompass several other modes, e.g., blinded, nonblinded, and so on.

Subsequent to the wear period, the patient returns to the HCP office (step 602), and the HCP uses the HCP app to extract data from the transmitter (step 604). In some cases the HCP may use the HCP app to upload data to a server (step 606).

Variations of the above will also be understood. For example, NFC on a mobile phone may be employed to scan the transmitter, which may then automatically cause the phone and transmitter to set up the system, such as by downloading a CGM monitoring app on the phone, as well as pairing the phone and transmitter and even initiating sensor startup.

For example, in one implementation the transmitter itself may store the application and, upon the pairing process, may transfer the application directly to the smart phone it was pairing with. A benefit of this arrangement is that there is no need to download the actual application in the professional setting, which often lacks appropriate or strong Wi-Fi or cellular connections, and which may implicate privacy concerns if the download occurs over a public network. Thus, the pairing may be initiated by an NFC connection and the application may be transferred via the NFC or via BLE. If the application stored on the transmitter is not the latest version, the same may be updated to the latest version when the user has a strong and secure connection. For example, in such cases, the app may communicate with the server to determine the latest version of the app. Alternatively, the server may push the latest version to the mobile device, upon determining the existing version is not the latest version.

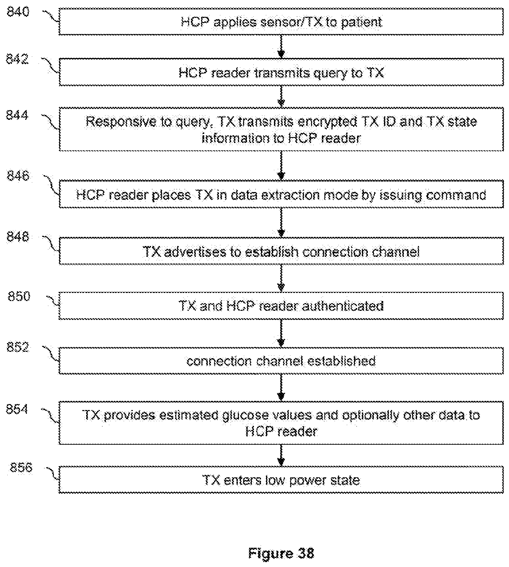

FIG. 38 shows a flowchart illustrating one example of the steps that may be involved in establishing communication between a sensor/transmitter with an HCP device such as an HCP reader (e.g., HCP reader 420 shown in FIG. 4) in an HCP office. For instance, these steps may be used as part of steps 592, 596 and; or 604 in FIG. 11 where an HCP app on an HCP device is used to pair the HCP device with the sensor/transmitter to verify proper sensor operation and extract data from the transmitter. In this example two different wireless protocols are employed. One of the wireless protocols is a short-range protocol such as NFC or RFID, for example. In some examples the short-range wireless protocol that is employed may be unencrypted at the protocol level (Of course, encryption may still be employed at the application level in these examples, if desired). The other protocol that is employed is an encrypted protocol that requires the use of an authentication procedure between the two devices. In one particular embodiment, which will be discussed for purposes of illustration, the short-range protocol that is employed is NFC and the encrypted protocol that is employed is BLE. Accordingly, in this example both the HCP reader and the transmitter are provisioned with an NFC transceiver circuit and a BLE transceiver circuit.

In a first step, a sensor/transmitter is applied to the patient and set up for use (step 840). To confirm that the system is set up and operating correctly, the HCP establishes communication between the HCP reader and the transmitter using the NFC. The HCP accomplishes this by bringing the HCP reader in proximity to the transmitter so that the two are within the NFC effective range. Once the HCP reader and transmitter are within range, the HCP reader transmits a query command to the transmitter using the NFC protocol (step 842). In response to receipt of the query command, the transmitter transmits its ID in an encrypted form using, e.g., public/private key encryption techniques (step 844). Moreover, to extract the transmitter ID from the encrypted form, the HCP reader may use a decryption key which may be obtained from the transmitter (based on a query) or from another computing entity (e.g., a server). In one example, the HCP reader upon providing a form of the transmitter ID or relevant information of the transmitter to the server, may receive a decryption key (e.g., a RSA key) to decrypt the transmitter ID.

In some embodiments the single query command may also cause the transmitter to transmit additional information such as its operational state, for example.

Examples of transmitter states that may be communicated by the transmitter to the HCP reader may include: (i) a storage state (indicating that the transmitter is still in its packaging or has not started a session that involves collecting glucose data, or not performing a sensor insertion verification), (ii) a sensor verification state indicating that the transmitter is performing a sensor insertion verification operation and has not yet collected glucose data from the sensor or waiting for data collection from the sensor to begin. Additionally, information such as, the total time to finish the verification and/or how long the verification will take, a result of the verification and when to check for the verification result, may also be provided when the transmitter is in the sensor verification state. (iii) An in-session state (indicating that the sensor is collecting data (e.g., glucose data) during its normal operation), and (iv) a session complete state (e.g., indicating that the transmitter has finished running a session; data is logged and available for download).

Additional information that may be transmitted to the HCP reader in response to the query command may include, for instance, how many attempts at sensor verification have been attempted. In one example, this information may be helpful to understand whether one or more false sensor startups have taken place (e.g., if more than one or more sensor verification inadvertently happened without the HCP performing or initiating the verification process).

Other additional information that may also be transmitted to the HCP reader in response to the query command include how much time remains until sensor verification is complete (as mentioned above). For example, if the HCP reader performs a query before the transmitter has finished the verification process that may take, for example, 30 seconds, the transmitter may indicate to the HCP reader to check or query again in the next few seconds, or may provide a particular time, to get the result of the verification.

Additional information in response to the query may also include how long has the current in-use session been running (e.g., when the sensor is in a running session). In one example, the result to this query is zero if the transmitter is in the storage state or in the sensor verification state.

Yet other additional information in response to the query may include whether or not the transmitter has been placed in a data extraction mode.

Additional information in response to the query may also include the type of BLE mode the transmitter is in and other information related to the mode. In one example, the additional information may include a type of BLE mode (e.g., normal BLE mode), the advertisement rate for the normal BLE mode, and the transmitter ID used for the normal BLE mode. In another example, the additional information may include another type of BLE mode (e.g., HCP data extraction BLE mode), the advertisement rate for the HCP data extraction BLE mode, and the transmitter ID used for the HCP data extraction BLE mode. It is contemplated that the advertisement rate during the HCP data extraction BLE mode will be higher than the normal BLE mode, and the transmitter ID or a form of the transmitter ID used for the HCP data extraction mode may be different than the one used for the normal BLE mode. It is contemplated that, during the 14-day session period, the transmitter may operate in the normal BLE mode.

If, based on the information received from the transmitter, the HCP confirms that the system is operating correctly and is in-session, the HCP completes the set up process by performing any additional steps that may be necessary, such as entering an operating mode (e.g., blinded or unblinded) of the system, for example. The patient may then use the device during a session.

When the patient returns to the HCP office after completion of the session (e.g., after 14 days), the HCP uses the HCP reader to place the transmitter in a data extraction mode. It is contemplated that, in some examples, after the 14-day session ends, one or more circuitry of the transmitter may transition into a low power mode automatically or upon receiving an indication from the user via a user device. Since patient data is now to be exchanged (e.g., after the completion of the session), an encrypted protocol that requires authentication such as BLE is employed. To establish the BLE connection, the NFC protocol is first used to place the transmitter in a data extraction mode. In particular, a BLE connection is established by once again bringing the HCP reader within proximity of the transmitter and issuing a NFC enter data extraction mode command using as a parameter a hashed version or an encrypted version of the transmitter ID(e.g., which the HCP reader obtained during the previous session (step 846)). If the transmitter confirms that the received hash is correct, an advertising and connection protocol is initiated by which the transmitter advertises for and connects to the HCP reader (step 848).

If the transmitter is currently communicating (or previously in communication) with a user/patient device when the enter data extraction mode command is invoked, the advertising scheme will begin after the user device disconnects. To ensure that any white listed user devices (e.g., a mobile device of the user that was in communication with the transmitter during the 14-day period) do not connect to the transmitter instead of the HCP reader, the transmitter may use a new BLE Generic Access Profile (GAP) address, and/or a new universally unique identifier (UUTD) to ensure that the HCP reader has priority on connection attempts. In some examples, during the extraction mode, the HCP reader may access information already stored in the memory of the HCP reader or another computing entity (e.g., a server) to identify and determine the identity the transmitter upon receiving initial communication request from the transmitter.

In one embodiment the transmitter uses continuous advertising to ensure that the HCP reader is made aware of the transmitter as soon as possible. That is, instead of advertising in a periodic mode in which advertising intervals are separated by quiescent periods, the transmitter may advertise without interruption. In some examples, the rate of advertisement during the data extraction mode (in order to establish connection with the HCP reader) is much faster than the rate of advertisement during a normal communication mode (e.g., when the transmitter communicates with the user's mobile device). It is contemplated that, a form of identification information of the transmitter may be included in the advertisement packet (e.g., a portion of the Transmitter ID or a hashed form of the same) in order to facilitate the HCP reader to quickly select and connect to the appropriate transmitter from amidst multiple transmitters that may be present in the HCP office (since the reader will know what transmitter ID it is looking for). It is further contemplated that, once the HCP data extraction mode is cleared, the transmitter may transition back to the normal mode of advertising where a previously connected user device may be able to communicate with the transmitter again normally.

Referring back to FIG. 38, once communication between the two devices (e.g., the transmitter and the HCP reader) has been established an authentication procedure can be performed as part of a data connection process (step 850). The authentication procedure may using existing standard and/or proprietary authentication techniques such as shown, for example, in U.S. Pat. App. Serial No. 14/968,695, which is hereby incorporated by reference in its entirety. The BLE connection is fully established upon completion of the authentication procedure involving exchanging secret keys and authenticating the same (step 852).

Once authenticated, the transmitter may provide data that has been obtained during the in-use session to the HCP reader (step 854). In particular, the transmitter can provide the estimated glucose values obtained by the analyte sensor and possibly additional data including, for example, private data (e.g., manufacturer proprietary information concerning the health and status of the system and data available from other sensors incorporated in the system (e.g., motion data from an accelerometer). It is noted that, the glucose data may be transmitted in a predetermined time (e.g., within 1 5 seconds) whereas, private data and motion data may be transmitted without a time budget.

As discussed above, during the data extraction mode, the transmitter retrieves all relevant details of a completed glucose session and exclusively communicates to the HCP reader as a data stream. Once data extraction is complete, the transmitter may be placed in a low power or sleep state (i.e., lowest possible storage mode) (step 856) which may last for a long time before exhausting the battery of the transmitter. It is noted that, after being placed in the storage mode, if the transmitter is used for any further purpose (e.g. failure analysis, additional data extraction, etc.) it would be returned to the manufacturer, where the transmitter may be woken up via NFC. In the various scenarios discussed above NFC is used as one of the communication protocols between the HCP reader and the transmitter. Since NFC communication is a very short-range protocol that requires the NFC antennas of the HCP reader and the transmitter to be virtually in contact with one another, the HCP is required to carefully align the two antennas when establishing communication. This can be difficult and awkward in a number of situations, particularly as sensor systems continue to be reduced in size, with a commensurate decrease in antenna size. Moreover, from the perspective of the HCP, it is important to be able to quickly and easily perform the data extraction process in order to minimize the time and effort that is needed.

Accordingly, in some embodiments it may be helpful to provide the HCP with an auditory or haptic feedback mechanism that facilitates the alignment process between the HCP reader and the transmitter antenna. For instance, in the case of auditory feedback, the HCP reader and/or the transmitter may be provided with an auditory transducer that emits a sound that increases in e.g., volume, pitch and/or in the frequency at which discrete pulses are emitted, as the proximity between the two antennas increases (and vice versa). Similarly, in the case of haptic feedback a vibrating transducer or the like may be used in the HCP reader to provide the feedback. Likewise, instead of or in addition to auditory or haptic feedback, visual feedback may be provided. For example, a light source on the HCP reader or on the transmitter such as an LED may emit optical pulses that increase in frequency as the proximity between the two antennas increases (and vice versa).

Besides the modes noted above, it is noted that the modes may be configured to be particularly interesting for use by the patient, allowing a degree of flexibility and "choose your own path" functionality. In this way the user may see the use of the sensor and transmitter and accompanying patient monitoring app as a "journey" or "adventure" to be pursued over time, including use of a transmitter with multiple sensors over multiple respective sensor sessions. As will be described in detail below, transmitters may incorporate hibernation or inactive state functionality so as to conserve battery life for such multiple sessions, and particularly so that, when data extraction occurs, sufficient power remains in the transmitter to allow signals to be sent from the transmitter to another device for data storage, e.g., using an HCP reader.

In the above described HCP set up procedures, it is important for the HCP to verify that the system is working, e.g., that the transmitter is properly receiving sensor counts from the sensor and is transmitting counts in cases where the transmitter is paired with a smart device including a HCP device. Such verifications may even bear on billing and reimbursement.

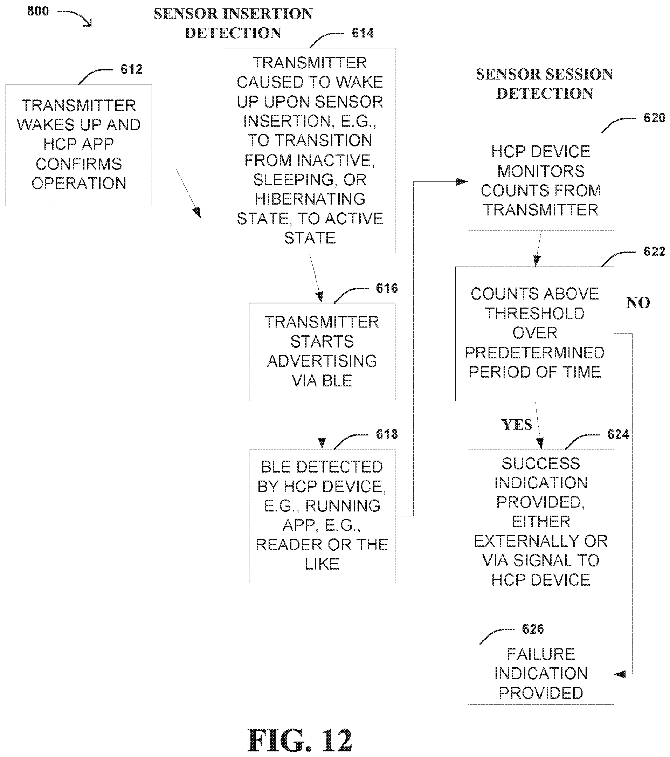

Referring to Fig. 12, the transmitter may ship and be provided in a kit in an inactive state, to conserve battery life, and thus the transmitter may require a "wake up" action caused by the HCP. Such action may be performed in various ways, including through the HCP app and/or a reader, or an HCP smart device. Details of the "wake up" procedure, in which the transmitter is transitioned from an inactive state to an active state, are described below. In the flowchart 800 of Fig. 12, a goal of the process is for the transmitter to have undergone a transition from an inactive state to an active state, and the proper operation is desired to be verified (step 612).

In one implementation, an HCP may verify two kinds of startup steps of the transmitter. Both kinds are required for proper operation. In this method, it is not just that the transmitter is caused to enter an active state, but also that the HCP app is caused to recognize the existence of the transmitter active state, and thereby provide HCP notification of proper operation.

Thus, in the flowchart 800, a first type of startup step includes detecting proper sensor insertion, and a second type includes detecting proper initiation of a sensor session. In the first type, the transmitter is caused to wake up upon sensor insertion and upon wake up, is caused to start communicating with the HCP device or HCP reader (step 614). For example, the transmitter can start advertising via BLE, which the HCP device can detect and thus determine transmitter activation (step 616). In this case, in some implementations the transmitter and HCP device will have been previously paired. In other implementations, step 614 or 616 may be employed to initiate a pairing process. The detection by the HCP device may be by way of prompting the HCP to conduct a visual inspection and subsequently confirm by way of a message back to the HCP device, detecting the beginning of receipt of sensor counts, as well as in other ways. Alternatively, the transmitter can be configured to send a separate notification message to the HCP device via NFC or BLE that the sensor insertion has happened. In either case, the HCP device detects the communication and detects that the first step has been performed (step 618).

In the next step, the HCP device monitors counts from the transmitter (step 620). The HCP device may further check as to whether the counts are within a predetermined range for a predetermined amount of time. In other words, the system automatically starts up, negating the need in prior systems for the user to start the sensor session using a display device.

If the counts are measured to be greater than a threshold level for the predetermined amount of time, then an indication of success is provided, either externally or via a signal on the HCP device (step 624). For example, if the threshold criterion is met, the HCP app may indicate so via a notification message. Alternatively, an external device may be cause to activate, e.g., an LED on the transmitter or the like, when the sensor count criterion has been met. Such may be particularly useful where there is no HCP device. In this case, the external device has to have some ability to know the sensor count criterion, and such may be implemented by way of an ASIC or other dedicated chip or electronic circuit, where the predetermined criterion is loaded into the external device at the time of manufacturing. Where an external device is employed, the wearable itself may provide notification, either visually or audibly, of proper operation. Such may be implemented by a small light that flashes, or a sound that beeps. The pairing with the smart phone may occur later for the user. In another implementation of an external device, the count monitoring may be performed on the transmitter. Where an HCP device is provided, the monitoring and determination of meeting the threshold criterion may be performed on the transmitter, the HCP device, the reader, a user smart device, or other connected device.

In the above steps, a wireless link including either NFC or BLE can be employed to perform the wireless communications noted. In the case of NFC, the NFC antenna can be anywhere on the phone, and thus use of an HCP reader device implemented as a dongle may be particularly useful, where such a dongle may include both NFC and BLE functionality.

A benefit to the method of Fig. 12, and similar methods as are described below, is that both steps have to occur for the transmitter to be properly verified and to enter into an active or working state for the duration of a sensor wear period. False startups are thus generally avoided. Other methods of avoiding false startups, which can deleteriously affect battery life, are described below.

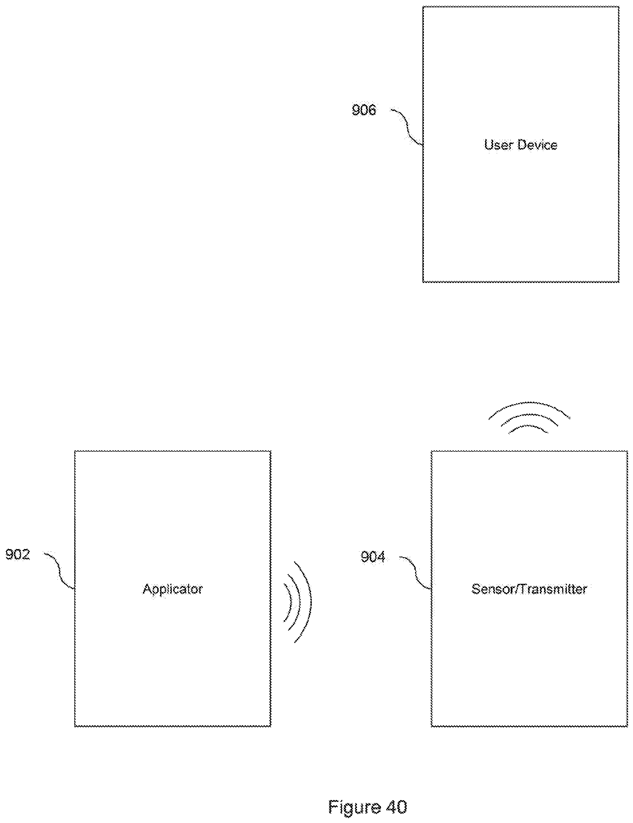

In the examples discussed above information obtained from the transmitter confirms that the sensor is working properly. In one alternative approach, the applicator that is used to insert the sensor into user's body may be provisioned with communication capabilities (e.g., NFC, BLE) that allow the applicator to verify that the sensor has been inserted correctly and is working properly. In one implementation the transmitter may send a signal (e.g., via NFC or BLE) to the applicator indicating whether or not the sensor is functioning properly. After the applicator has installed the sensor and the sensor has indicated that it is functioning properly, the applicator can send a wireless signal to the HCP app and/or the app on the user device indicating that the sensor is operational. Fig. 40 shows the applicator 902 in communication with the transmitter/sensor 904. The transmitter/sensor 904, in turn, is in communication with the user device 906.

Another issue often encountered is that users generally have very little time to spend with an HCP during a visit, and thus it is desired for the transmitter to be transitioned to an active state as soon as possible. Various solutions are proposed below.

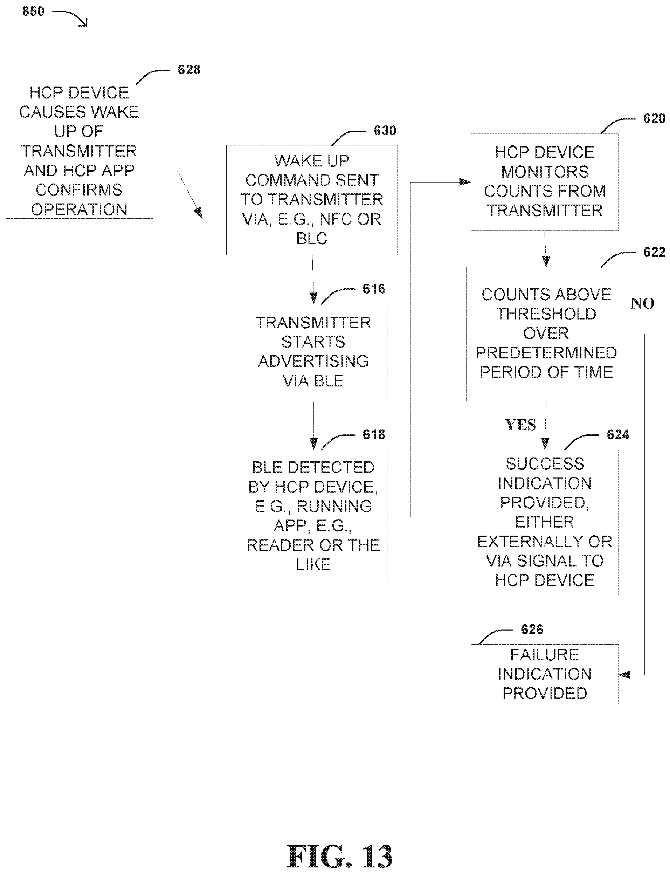

In one implementation, and referring to the flowchart 850 of Fig. 13, the HCP device may cause wake-up of the transmitter (step 628), and in particular may send a wake-up command to the transmitter via, e.g., a wireless link such as NFC or BLE (step 630). The remainder of Fig. 13 is as described above with respect to Fig. 12.

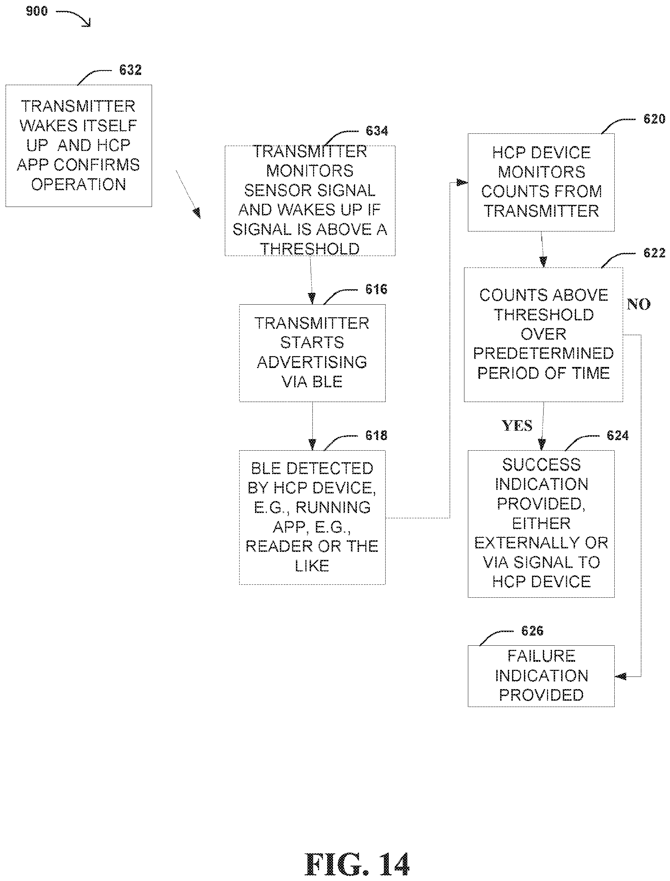

In another implementation, and referring to the flowchart 900 of Fig. 14, the transmitter wakes itself up (step 632) by monitoring the sensor signal and waking up if the signal is above the threshold (step 634). The remainder of Fig. 14 is as described above with respect to Fig. 12.

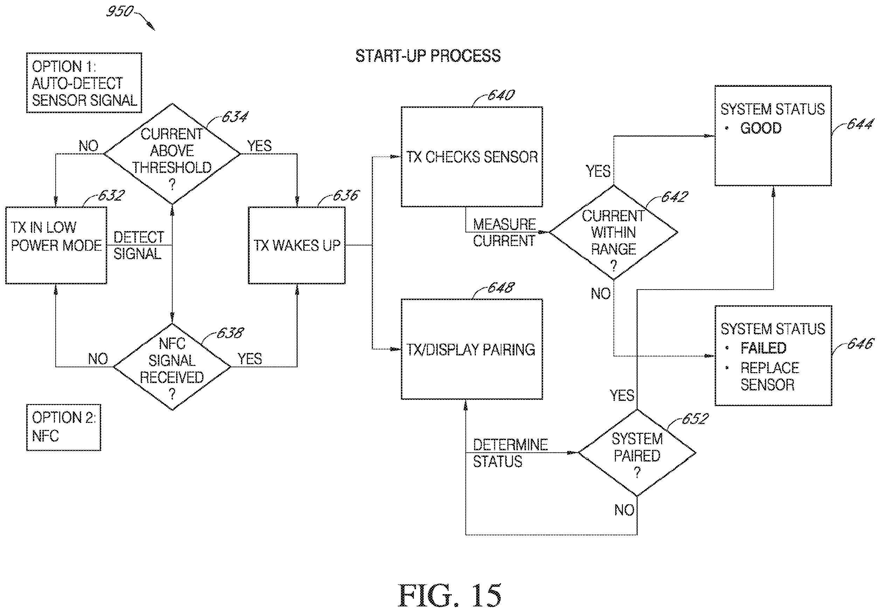

The above implementations are described as part of a combined process in the flowchart 950 of Fig. 15. It will be understood that other wake-up methods may be combined into the overall process. As can be seen, it is expected that such a startup process can be completed within a short time period, e.g., less than five minutes. The two options here include auto detection of a sensor signal and a forced wake-up via NFC. In this process, the transmitter detects whether the current/sensor signal is above a threshold, and if so, then the transmitter wakes up and starts a sensor session within a certain period of time for a predetermined amount of time, e.g., 2 minutes.

In a first step, a transmitter begins in a low-power mode (step 632). If a sensor signal is detected, a test may be performed as to whether the current corresponding to the signal is above a threshold (step 634). If not, the transmitter may be maintained in the low power mode (step 632). If so, the transmitter may be caused to wake up (step 636). If the signal is determined to correspond to an NFC signal (step 638), then the transmitter may again be caused to wake up (step 636). That is, an NFC signal may be employed to cause sensor wake-up even without sensor counts being measured. In some cases, both of the above may be employed and required for transmitter wake up. In other words, the measured current may be required to be above a threshold or within a target range and a wake-up signal may need to be required via NFC to cause transmitter wake up and to cause the transmitter to be maintained in this mode for the duration of a sensor session.

In some implementations, an accelerator that is included in the transmitter may facilitate the wake-up process. For example, if the accelerometer detects motion (e.g., caused by the user), the processor may wake up earlier than the predetermined time intervals, for example, every 2 or 5 mins (i.e., reduce the intervals at which it checks for current) at step 634. In one example, the transmitter may go back to the low power mode (e.g., step 632) if the current remains under the wake-up threshold and no movement is detected for a certain period of time.

Following wake-up, the transmitter may check the sensor for operation (step 640). Such steps may include checking if the detected current is within an appropriate range (step 642). If so, the system status may be judged as operational (step 644). If not, the system status may be determined to have failed (step 646). In some cases, the current may be required to be within a predetermined range for at least a predetermined period of time to result in transmitter wake up. Also following wake-up, where an external display device is used such as a professional product or user smart device, a step may be performed of pairing the transmitter with the display (step 648). If the system successfully pairs (step 652), then again the system status may be judged as operational (step 644). If the system does not successfully pair, the pairing operation may be performed again (step 648).

In some implementations, appropriate time measurements along with the measurement of counts/current may be utilized to determine sensor insertion time. In one example, the transmitter may be in a normal operating mode (e.g., after step 636). In such implementations, the transmitter may then measure counts/current within or above the sensor detection threshold level and may further record the time of the measurement (e.g., at time *a*). The transmitter may further monitor the measured counts/current for subsequent measurements. If the measured counts/current does not decline below the threshold in a subsequent measurement, the transmitter may determine and validate that the detected count/current measurement of the sensor detection threshold level did not result due to an error. Following that, the transmitter may receive a Start Session command from a display device at another time *(e.g., at time b)* - which provides the display device the opportunity to validate that time *a* is the legitimate sensor insertion time. The transmitter may then communicate to the display device that time *a* (and not time *b*) is the accurate sensor insertion/sensor session start time. As such, the signal processing algorithm in the transmitter and/or an appropriate entity may then adjust time dependent variables (e.g., delayed timing or start of displaying of EGV values based on the validated sensor insertion time (e.g., time a) and not based on time b). It is contemplated that, in some implementations, the transmitter may override time *b* (i.e., the initial indication of sensor session start time from the display device) with time *a*, upon initially receiving the sensor session command from the display device, and upon further validating that time *a* is the accurate sensor insertion/ sensor session start time.

In another example, the transmitter may validate a wakeup time (e.g., time *c*) of the transmitter as the sensor insertion time, when it is determined that the transmitter has indeed exited the low power state (e.g., from step 632). It is noted that the wakeup time c may be recorded prior to a marked wakeup time (e.g., a wakeup time marked at step 636) as the transmitter accurate wake up time, as the wakeup processing algorithm may introduce a delay time.

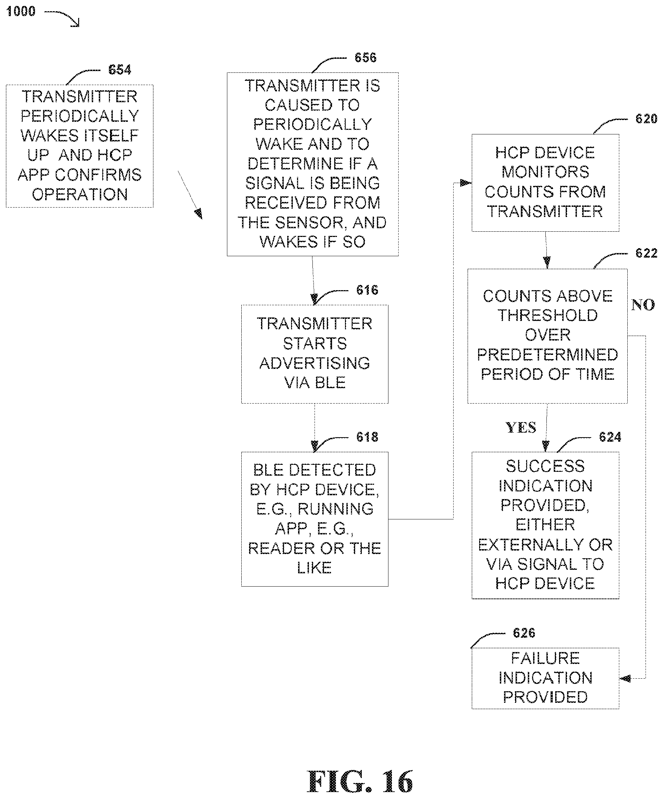

In yet another implementation, as illustrated by the flowchart 1000 of Fig. 16, the transmitter may again wake itself up (step 654), but in this case the transmitter may be caused to periodically wake-up and to determine if a signal is being received from the sensor (step 656). If so, the transmitter may enter the active state and start recording data. In some implementations, the system may communicate such an event, and in particular that it has successfully started up, e.g., via a transmitted signal to a connected device or via an external indication such as an LED or audible sound. The remainder of the method of Fig. 16 is as described above with respect to Fig. 12.

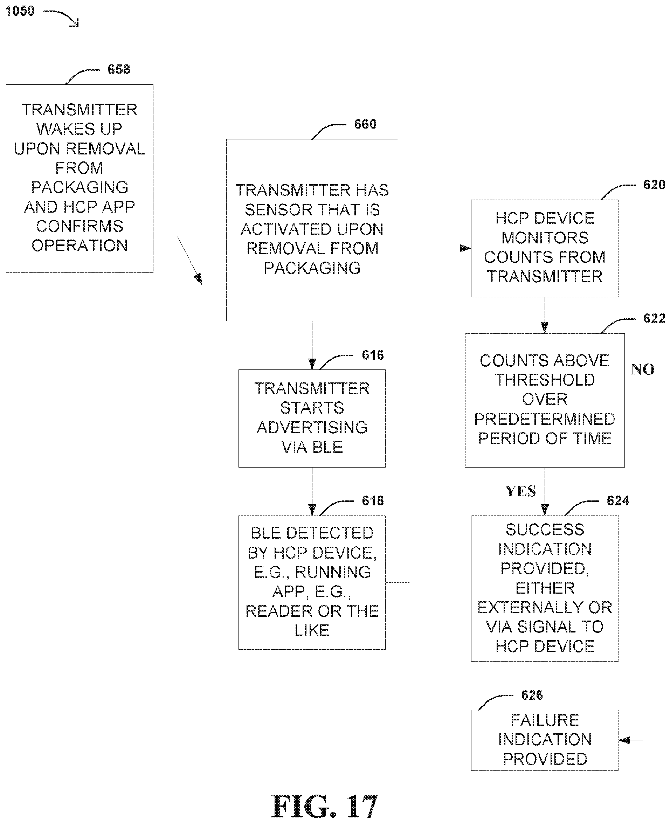

In yet another implementation, and as indicated by the flowchart 1050 of Fig. 17, the transmitter may be caused to wake up upon removal of the packaging (step 658). In more detail, the transmitter may be outfitted with a sensor that is activated (or is activatable) upon its removal from packaging (step 660). For example, the transmitter may be activated by a startup device, e.g., a NFC based device or via audio or visual cue. The HCP could then simply hand the patient instructions to download the app and to pair at a future time when data conductivity is achieved and/or the necessary support.

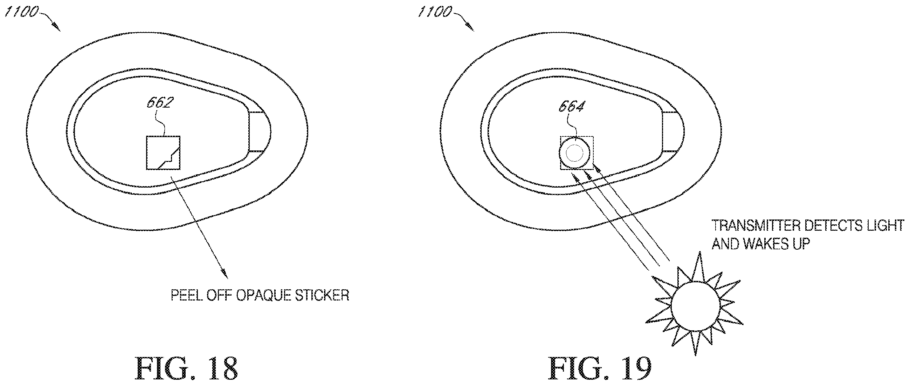

As one implementation, and as indicated by the device 1100 in Figs. 18 and 19, a wake-up triggering system and technique may be implemented by a light sensor 664 covered by an opaque sticker 662. The light sensor wakes the transmitter by closing the circuit when light falls on the sensor. Light is prevented from falling on the sensor when inside the packaging by maintaining the opaque sticker that covers the sensor until it is peeled off by the user when ready to pair.

The remainder of the description of the flowchart 1050 is as indicated above with respect to Fig. 12.

In another implementation, the sensor that wakes-up the transmitter when it is removed from the packaging in step 658 may be an accelerometer. The accelerometer may be included with the transmitter electronics or it may be incorporated in the packaging. In either case, upon detection of an acceleration event (such as caused by opening the packaging or deployment of the sensor or transmitter) by the accelerometer the accelerometer generates a signal that causes the transmitter to wake-up. In some cases the wake-up signal may only be generated if the magnitude of the acceleration is within a specified window. For instance, accelerations below a certain value may result from normal jostling of the packaging and not from opening it. Likewise, accelerations above a certain value may result from dropping the packaging and not from opening it. In one alternative, instead of an accelerometer, a one-time event circuit such as a shock sensor that breaks upon acceleration may be used.

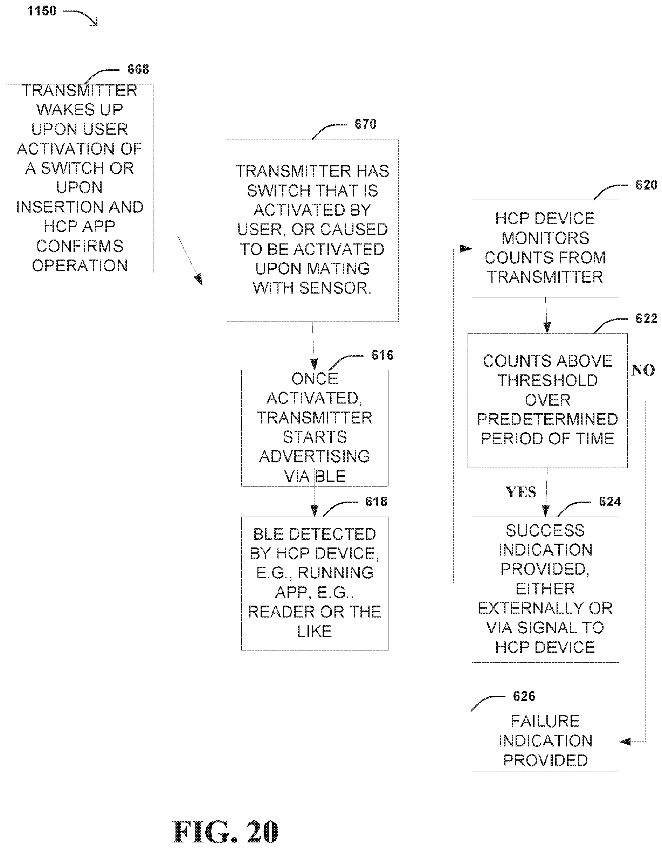

In yet another implementation, and referring to the flowchart 1150 of Fig. 20, the transmitter may wake up upon user activation of a switch or upon user insertion of the transmitter within the sensor housing (step 668). In this implementation, a physical activation switch is embedded into the transmitter that can be activated by instructing the user to press or otherwise activate the switch. Alternatively, the switch may be pressed by the applicator during deployment of the sensor into the user body. The embedded switch may be part of the flexible wearable and when the switch is pushed, a physical, e.g., metal to metal, contact is made, and the device may be caused to wake up from a low power or inactive or sleep mode. As before, at the end of the intended duration, the system would stop recording data and be ready to download data. Moreover, in some implementations the system may communicate to an app or another device that it has successfully started up.

The remainder of the flowchart 1150 is as described above with respect to Fig. 12.

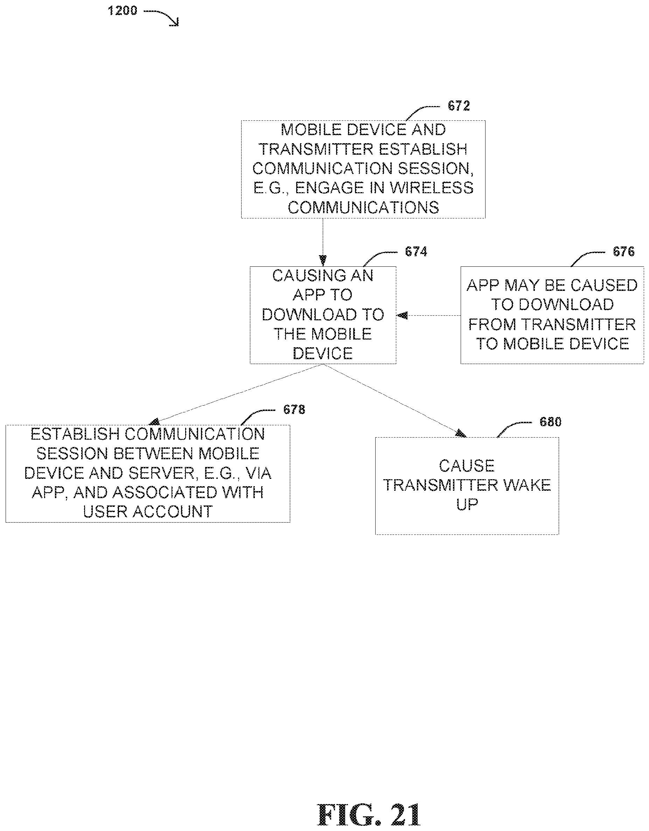

Other techniques may also be employed to cause wake-up of the transmitter. For example, referring to the flowchart 1200 of Fig. 21, a mobile device and a transmitter may establish a communication session, e.g., the same may engage in wireless communications as described above (step 672). An app for use by the patient, e.g., a CGM app, e.g., or another type of analyte monitoring app, may be caused to download to the mobile device (step 674). Alternatively, the app may be caused to download from the transmitter to the mobile device (step 676). Once the app is on the mobile device, a communication session may be formed in a secure way between the mobile device and a server, and associated with the user account (step 678). Also using the mobile app, the transmitter may be caused to wake up (step 680).

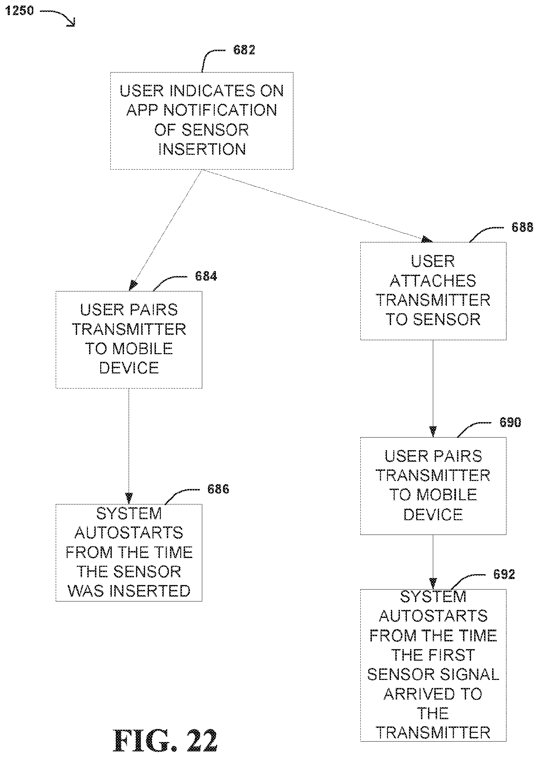

In this regard it is noted that starting a sensor session after a certain time has passed from the moment of insertion may negatively impact the accuracy of the measured data. Systems and methods according to present principles provide various ways to address this. In particular, and referring to the flowchart 1250 of Fig. 22, a user (or the HCP) may indicate on the downloaded app that sensor insertion has occurred (step 682). The user may then pair the transmitter to the mobile device (step 684), and the system may then auto start from the time the sensor was inserted (step 686).

Alternatively, the user may couple the transmitter to the sensor (step 688), and then pair the transmitter to the mobile device (step 690). The system may then auto start from the time the first sensor signal arrived at the transmitter (step 692).

In any case, in subsequent sessions, the user may attach the transmitter to the sensor, and the system may auto start the session from the time the first sensor signal arrives at the transmitter.

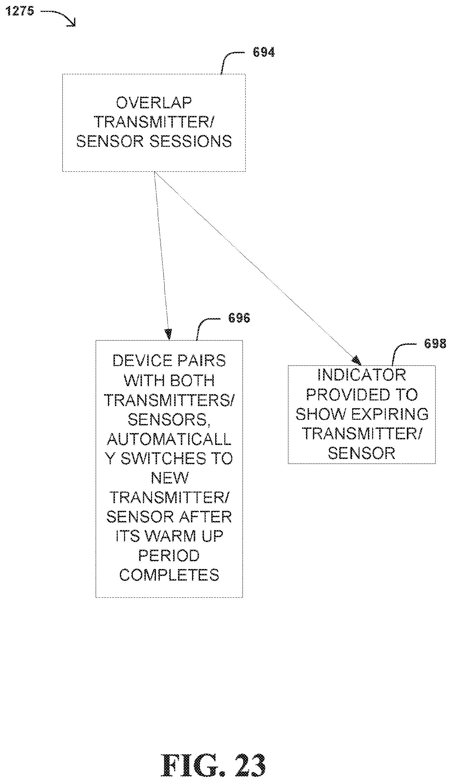

In a variation, and referring to the flowchart 1275 of Fig. 23, systems and methods according to present principles may address problems related to, during the warm-up period, a lack of glucose values being obtained. For example, using a low-cost or disposable transmitter, a patient could overlap the wearing of two transmitters/sensors (step 694). A display or displays could pair with both transmitters, and automatically switch to using the newer transmitter once the warm-up period is completed (step 696). In a variation, an indicator may be provided to show which is the old or expiring transmitter/sensor (step 698). In this way, the chances reduced of accidental removal of the newer sensor.

The indicator in step 698 may be provided by a visual indicator, e.g., an LED light, on the expiring transmitter or on the new transmitter. A physical indicator may also be employed, e.g., using vibrations. In another alternative, a display device, e.g., the professional product or the user's smart phone, could employ a proximity sensor to tell the user when the display device is near the expired or non-expired sensor. The resolution of the proximity sensor, i.e., its ability to distinguish one sensor from another, can be low if the sensors are widely separated, e.g., on opposite sides of the body, or if the sensors are required to be separated by way of user instruction or the like. In some cases such a system may require physically touching the display device to distinguish transmitters within inches of each other. Modes of communication for such purposes may include very short range electromagnetic signals, e.g., RF signals or magnetic fields, e.g., NFC. In another implementation, a custom USB or other cable may be employed that plugs into the display device and which has an adapter on the other end to read a transmitter. A device specifically configured for such purposes may be designed that has a port in which the transmitter may be placed or connect to, or which otherwise has a touch point for transmitter contact. In one example, a very low voltage power socket checking device may be employed, and the same may include, e.g., a small light that indicates if the touched transmitter is expired and should be removed.

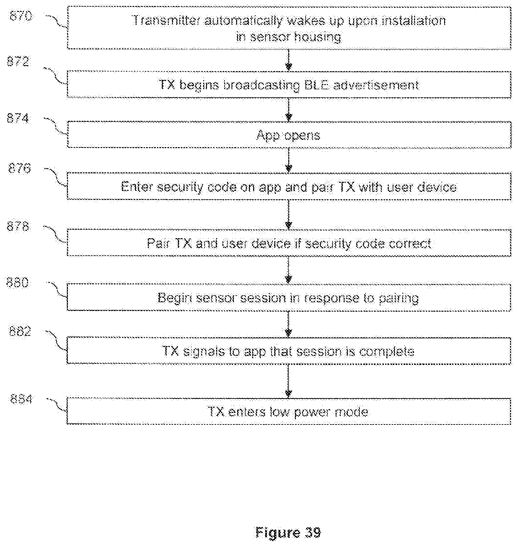

In a variation, and referring to the flowchart of Fig. 39, another technique that may be used to wake-up the transmitter when a user is to install a transmitter for the first time. In this variation, a mechanism is provided that automatically wakes-up the transmitter when the user installs it in the sensor bay/housing. This may be accomplished in any of a variety of ways. For instance, installation of the transmitter in the bay/housing may cause a dedicated wakeup circuit to be activated. As another example, a simple mechanical mechanism may be employed by which insertion of the transmitter into the bay/housing causes activation of a mechanical switch on the transmitter by engaging with a portion of the bay/housing, and thereby waking-up the transmitter.

Referring now to Fig. 39, after the user removes the transmitter from its packaging and installs it in the sensor bay/housing, the transmitter automatically wakes up without user intervention (step 870). Typically the transmitter can wake-up in a few seconds. Next, the transmitter begins broadcasting a BLE advertisement or beacon as part of an advertising and connection protocol (step 872). If the user has already installed the dedicated software app on their user device (e.g. smartphone), the app may automatically open and in response to the advertisement ask the user if he or she wishes to pair the devices (step 874). Assuming the user desires pairing, the user enters a transmitter ID or other code that may be used for security measures (step 876). In some cases this may require the user to take a picture of a barcode located on the transmitter. Alternatively, an NFC tag storing the appropriate transmitter ID or other code may be located on the transmitter, which the user device can read when the user brings the user device in contact with the transmitter. If the transmitter ID or other code is correct, the transmitter and the user device will be paired via BLE (step 878). The session may then start automatically after a warmup period, which may be indicated on the app with a countdown timer (step 880).

It should be noted that in the example shown in Fig. 39, the transmitter automatically drives the pairing and session start-up process once it is in installed in the sensor bay/housing without the need for any additional action on the user's part such as by initiating a session with a start button.

At the end of the session the transmitter sends a signal to the app on the user device indicating that the session has ended and the app in turn notifies the user that the session has been completed (step 882). The transmitter may then go into a low power or sleep state and periodically (e.g., every 5 minutes) wake-up to check if the app needs to be updated with data from the previous session (step 884).

When a subsequent session is started after the initial session as described in Fig. 39, the process is similar to that shown in Fig. 39, but with fewer steps since the transmitter and the user device are already paired. In this case when the user inserts the transmitter into a new sensor bay/housing, the transmitter automatically wakes-up from its sleep mode and broadcasts a BLE advertisement that automatically starts the dedicated app executing on the user device. The session then automatically starts after a warm-up period. The app may display a countdown timer that indicates when the warm-up period will be completed and the sensor session begin. If during the warm-up period the transmitter determines that the sensor is a used or expired sensor, the transmitter signals this condition to the app and then goes into a low power or sleep mode.

In some embodiments the countdown timer provided by the app may present a message such as "your sensor session will begin in three minutes." In general the timer may be configured to err on the side of overestimating the amount of time remaining rather than underestimating since it is better to exceed user expectations than fail to meet user expectations. In some cases it may be desirable to first ask the user at session start if they wish to be notified when the warm-up period is complete. If so, the notification may be provided directly by the app using any suitable means such as with a visual or audio indicator or a message could be provided through another app on the user device such as a calendar app, where it can be scheduled in a manner similar to any other event.

In another variation, a mechanism may be provided that allows the transmitter to remove itself. For example, the transmitter may eject itself from the sensor bay/housing, while the user may still be required to manually remove the adhesive patch. In another variation, the transmitter may trigger a device that causes the patch to loosen and/or to retract the sensor.

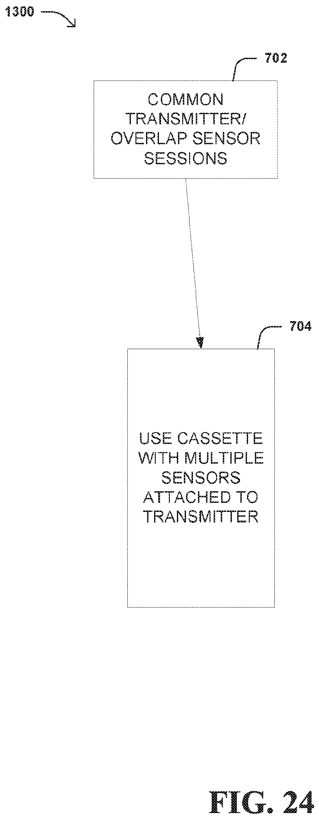

In another variation, and referring to the flowchart 1300 of Fig. 24, a common transmitter may be employed that is configured for use with overlapping sensor sessions (step 702). In one implementation a cassette is used with multiple sensors that can be placed in signal communication with the transmitter (step 704), with at least two sensors in signal communication with the transmitter at the same time, e.g., via NFC or BLE. In such a system employing a transmitter with multiple sensors, each sensor may be deployed in a serial fashion over time, with a degree of overlap between two sensors. A cassette or drum of sensors may be employed with an integrated deployment mechanism. Alternatively, a separate deployment mechanism may be employed, e.g., a tool that inserts the new sensor which is already in the cassette. The transmitter may have multiple spots, ports, or slots for sensors, but each sensor may be applied by a separate applicator tool to or through the transmitter at the beginning of each new session. In a variation of this implementation, a reusable applicator may be employed, which operates manually or automatically, and which can take an assembly consisting of a sensor, transmitter, and needle. Such a reusable applicator may be used repeatedly to install multiple serial sensors.

The above has described installation and, to a certain extent, pairing of transmitters with sensors and HCP or other mobile devices. It is noted in this regard that the transmitter has no actual source of absolute clock time. Even where a transmitter has been used before, if it has not been paired with a phone or like device, the transmitter has generally not communicated with a source of real-time clock data. Rather, the transmitter has only measured or clocked how long it has been operational since initiation. Such can be problematic, e.g., if a user sensor session ends after 14 days of use, but the user only brings the transmitter back to the HCP after 17 days. In such a scenario, the transmitter has no absolute data about how long it has been since initiation, particularly if it has been in a low-power mode for part of that time. Moreover, the HCP may desire to perform analysis on the data based on the actual day and time of the sensor data, e.g., either for individual analyses or for matching up with other event data, e.g., food or exercise.

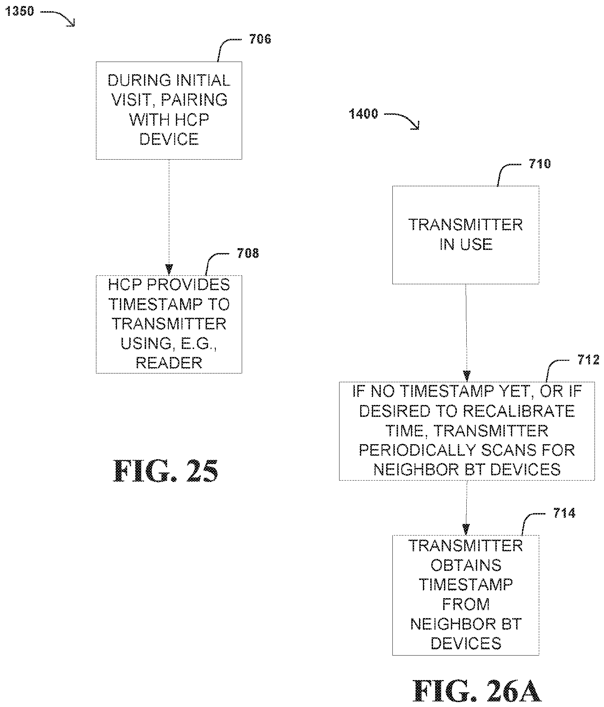

One solution is to actively provide data from the HCP device, e.g., from the HCP app, to the transmitter, e.g., using the real time of the clock on the HCP device. For example, and referring to the flowchart 1350 of Fig. 25, during the initial visit, the HCP device may be paired with the transmitter (step 706). Subsequently, the HCP device, e.g., HCP app, may provide a timestamp to the transmitter using, e.g., the reader in NFC mode (step 708). The transmitter may then use this time information to further timestamp the sensor data that will be collected by the transmitter over the course of the wear period. In that way, the transmitter will receive a correspondence or reference time (e.g., 0 sec matches to July 1st at 2pm), and such may be valid for both blinded/unblinded modes of the transmitter. Thus the HCP can provide the timestamp in one case, and the patient may go home and synchronize with their phone and get another time stamp. The transmitter then uses this time information to further timestamp the sensor data that will be collected by the transmitter over the course of the wear period (e.g., 14 days).

Alternatively, the transmitter may periodically look or scan for a timestamp from Bluetooth devices that may be in range of the transmitter. The transmitter may request for a "current" timestamp from one of the Bluetooth devices.

This implementation is shown by the flowchart 1400 of Fig. 26A. In this figure, a transmitter may currently be in use in a sensor session (step 710). If no timestamp has been obtained yet, or if it is desired to recalibrate according to a new timestamp, the transmitter may intermittently or periodically scan for neighbor devices, e.g., neighboring Bluetooth devices (step 712). The transmitter may request and subsequently obtain a timestamp from the neighboring Bluetooth devices in this fashion (step 714).

If there is a drift in time in the transmitter/sensor data during the sensor session, then the HCP may download the time drifted data after the sensor session and perform an adjustment of the data via a software application. In one implementation, a user may synchronize times at the beginning and end of the session to compensate for such drifts and time lags.

In variations, the user may pair to the transmitter when they get home, following the HCP visit, and the transmitter may receive a timestamp as part of this pairing process. In this case, the user professional product, or user's smart phone, provides the synchronization of time, where synchronization of time refers to providing a timestamp to the transmitter. In this way, the transmitter can synchronize to the real-world time regularly via the user's smart phone. In a related variation, if the transmitter is installed at the HCP office but the user does not synchronize the transmitter to their application until five hours later, the real-time would be obtained only at the five-hour point. However, if the transmitter has been employed to receive data during this time, such data may be time stamped retrospectively, so that the same can still be used as timestamp data in subsequent analyses.

As noted above, one of the mode choices the HCP has is to blind (or not) the display of measured analyte values to the patient. In this regard it is noted that the patient may act adversely, e.g., may alter their normal day-to-day activities or behavior if allowed to monitor the glucose data. This may mislead or hinder the HCP from accurately interpreting the patient's glucose data.

In cases where it is desired for the patient to be completely blinded with respect to the data, there is generally a reduced need to employ a professional product or user's smart phone, unless such is used for data backup. Where there is no smart device app involved, such is termed an "extreme blinded" mode.

In a particular embodiment regarding an extreme blinded mode, the blinded mode may be implemented such that the transmitter does not provide an EGV value to the phone/receiver because it turns off the BLE radio after a predetermined or a certain period of time. This embodiment may be particularly useful for patients without smart phones. Turning off the BLE radio can be done (by the transmitter) when the transmitter determines that it has not paired with any phone or device after a predetermined time (and after the HCP has put the transmitter on the user). In this example, the HCP does not provide a timestamp to the transmitter or verify sensor insertion at the setup stage. Instead, the HCP simply puts the transmitter on the user, and the user leaves the HCP office.

However, as the HCP does not provide a timestamp to the transmitter, the transmitter has no basis to determine an absolute starting time at which the sensor session started. The transmitter circuit can still keep time, however, by keeping a counter which would be initiated at the initial HCP set up stage. The counter could continue to keep track of "time" even after the sensor session ends (e.g., after 10 or 14 days), e.g., and even if the sensor is running in a low-power mode. By keeping the counter running, even after sensor session ends, the transmitter can provide information regarding how long it has been "awake" after being initialized. As such, when the user goes back to the HCP (e.g., after 14 days), the HCP reader or software can still determine out 'when' and 'for how long' the data was collected (based on the counter information).

In a particular implementation, a user may install the transmitter at any location, which may include an HCP's office, home, in their car, or elsewhere. In this case it is not necessary or possible to confirm (by the HCP) sensor insertion or sensor start. As noted no time reference, e.g., UTC time reference, is provided to the transmitter. The transmitter starts in a default mode, which may be a blinded mode, because the mode has not been changed via any means, e.g., has not been changed by the HCP using an HCP device, professional product, reader, dongle, or the like. As no Bluetooth pairing has occurred, the transmitter may automatically turn off its Bluetooth radio after a certain period of time, e.g., 1 to 12 hours.

In one implementation the session simply ends after a predetermined sensor duration, e.g., 15 days. The transmitter enters a very low-power "timekeeping mode". In this mode the transmitter may wake up periodically and update a record corresponding to the counter, e.g., every five minutes, every 30 minutes, and so on in some cases the transmitter may enter or avoid a "deep sleep" mode, in which NFC is required to wake the transmitter up.

In yet another variation of this system, a timekeeping mode ends after a predetermined period of time, e.g., after 15 days. This time may also be configured to be variable, and based upon battery power remaining. In this "timekeeping mode", energy savings is achieved by the lack of Bluetooth advertising in the blinded mode

In any case, the user returns to the HCP office and the HCP reader communicates with the device. For example, the HCP reader may wake the device via NFC, enable the Bluetooth low energy communications, download data over the same, and then end the session.

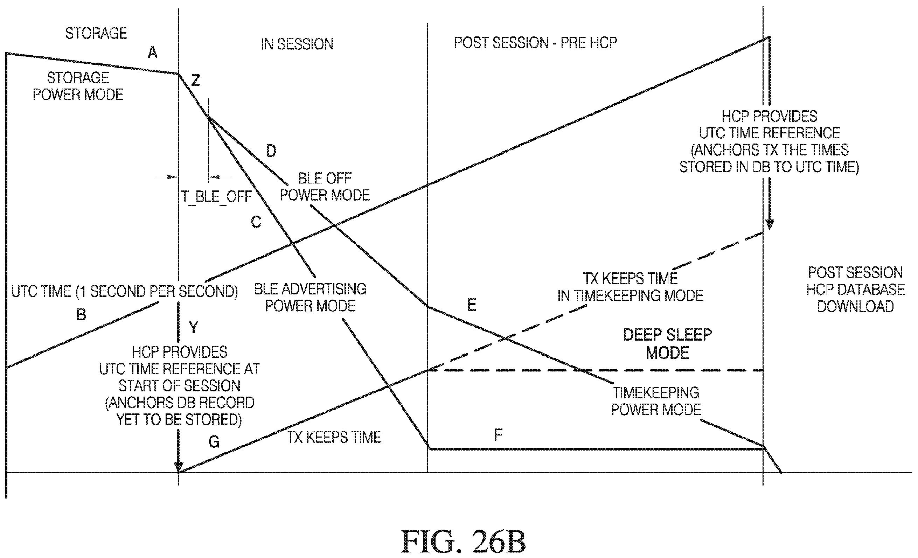

A graph of power left (or available) over time is illustrated in Fig. 26B. As may be seen, in an initial storage mode the power indicated by "A" slowly decreases while the time indicated by B increases; however, the measurement of this time has not yet begun in this mode.

At the beginning of a session various steps may occur. In one case the HCP provides a UTC time reference at the start of the session, which then "anchors" the session to a particular absolute starting time. This anchoring is indicated by Y, and the transmitter then keeps time according to line G.

In other cases, the HCP does not provide a time reference, e.g., where the user installs the transmitter themself at their home or in their car. For a period of time indicated by Z, BLE advertising may occur to allow potential communications to occur. In an unblinded mode, e.g., where data from the transmitter is transmitted to another device for display, then line C is followed, and the power decreases more rapidly. In the blinded mode, indicated by the line D, then following the initial BLE advertising, the BLE turns off and the power decreases more gradually.

The next segment indicates post-session activity. Line E indicates a timekeeping power mode, where the transmitter continues to monitor the passage of time. Line F indicates post-session activity while BLE advertising occurred during the session. As measured data was associated with absolute timestamps during this period, line F can indicate extremely low power consumption, i.e., a "deep sleep" mode, as even timekeeping is no longer required. In Fig. 26B, the line appears basically flat. In either of these cases, upon a visit to the HCP office, followed by download of the measured data, the data may be provided with a UTC time reference. In some cases, it will be the first time reference received, and the measured data may then be post associated with various time references, based on the provided UTC time reference. In some cases, where BLE advertising and data communications were conducted with, e.g., a smart phone, the UTC time reference received at the post-session HCP visit may provide a corroborating time reference, e.g., a second time reference that verifies the accuracy of the initial time reference. However, where a device is used that follows line F, a time reference provided at the HCP visit will not corroborate the initial time, as the time period indicated by the line F is unknown (no time data being recorded).

In particular implementations of the system described above, it is advantageous if Bluetooth advertising, and indeed all energy use in the way of communications, is turned off in the blinded mode. The blinded mode should generally be the default mode, although the same can be disabled by the HCP if the session is to be unblinded. In this case, the disabling can occur via Bluetooth low energy. Bluetooth low energy advertising may be configured to be activated via an NFC command at the HCP. In an unblinded case, it will generally already be on.

An advantage to such embodiments is that the BLE radio is off for the sensor session period, which significantly reduces battery power consumption. In addition, such implementations further significantly reduce the time required for the HCP to setup the transmitter. Data extraction may be as described before, e.g., via NFC/ Bluetooth.

To enable such modes, as noted above, during set up, the HCP programs the transmitter, or professional product or smart phone application, in various modes depending on the user's needs/condition and skill level. The HCP also programs the transmitter to be in blinded or unblinded mode.

In one exemplary way of accomplishing the programming, a transmitter may be provided with multiple serial number extensions, where each extension defines how an app or other connected device behaves when paired to the transmitter. For example, a first extension may correspond to a blinded professional mode; if this serial number extension is used, then when paired to a device, the device only displays sensor working status. A second extension may correspond to a semi blinded professional mode; if this serial number extension is used, then when paired to a device, the device only displays sensor working status and predefined alerts. A third extension may correspond to a real-time personal and professional mode; if this serial number extension is used, then when paired to a device, the device may be configured to display alerts, glucose values, trend graphs, and so on.

Generally the blinding mode choice can only be configured by the HCP; however, either the transmitter or the mobile display device may accomplish the blinding. The transmitter may accomplish the blinding by either not transmitting the data continuously during the sensor session or by transmitting the same with an appropriate flag indicating that the data is not to be displayed on the user interface of the smart device.

Where user downloads the app but does not see the analyte data, such is termed a "semi-blinded mode". In this mode, the user can receive alerts and calibration information, and in some cases can further enter data about medications ingested, meals eaten, and exercise performed. In this way, alerts can be employed to help mitigate severe adverse events. For example, the transmitter can change color, vibrate, heat up, or a connected transmitter can trigger alerts on a user's smart phone, without providing a glucose trend or number. Such facility allows the user to trigger action, for example, take a finger stick, to help address any potential concern that the sensor identified with the alert.

Where the user is enabled to view the analyte data, trend graphs, and so on, such is called an "unblinded mode".

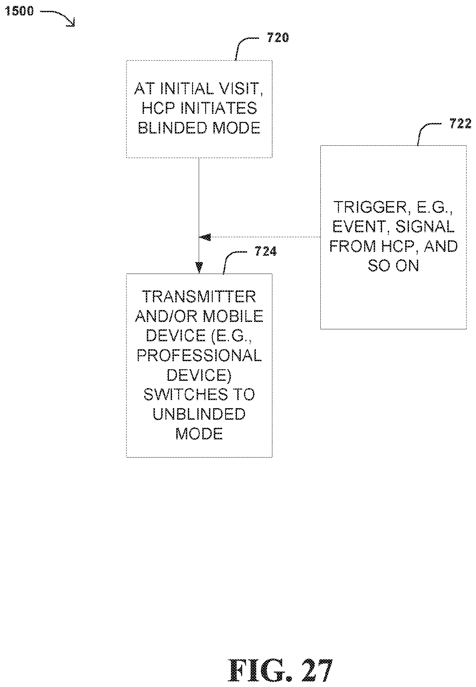

In some cases switching may occur between the blinded mode and the unblinded mode. Referring to the general overview flowchart 1500 shown in Fig. 27, at an initial visit, an HCP may initiate the blinded mode (step 720). A trigger event may occur (step 722), and the transmitter and/or mobile device may be caused to switch to the unblinded mode (step 724). In this way the transmitter may be made configurable for switching based on HCP preference as well on the occurrence of one or more events.

For example, one implementation may allow the physician to start the patient on a blinded CGM system, which will collect glucose data. The physician may set up a trigger, e.g., based on time or the occurrence of an event, that would automatically switch the system to an unblinded mode, giving the patient access to alerts and real time data. In one implementation, after 14 days the data becomes unblinded so the user can view their data. In another implementation, the data may become unblinded if the user attains an exercise or meal goal. In yet another implementation, the data may be blinded for seven days and then unblinded for the subsequent seven days. Other variations will also be understood.

In any case the physician can then compare the two data sets and provide the patient with actionable insights and therapy adjustments.

In another variation, "real-time blinding" may be employed to provide additional insights. In particular, it is noted that, with blinded mode, the user is not able to see the glucose data or get any of the glucose alerts. This is beneficial to prevent users from taking immediate actions based on looking at the glucose level changes, as doing so can impact the medical plan that an HCP may prescribe to the patient. On the other hand, patient viewing of data also helps the patient realize the impact of lifestyle events on the glucose value. Thus, "real-time blinding" provides a middle ground and accomplishes both goals. In particular, real-time blinding configures the system such that the patient can not see data immediately; however, the patient will be able to see older data - for example, data from the previous day or data older than several hours.

The amount of time can be made configurable, e.g., by the HCP, or ranges may be prescribed by the HCP, within which the user can set the time period. A corresponding UI of the app may in one implementation not include a usual trend screen, but only incorporate a reflection view, avoiding any confusion the user may get into by interpreting older data as current data.

As noted in Fig. 4, a diagnostic app 428 may be employed, and such may be particularly advantageously employed as a secure app for blinded mode diagnostics. In this regard, it is noted that a user cannot use a standard CGM app to view data during a diagnostic session. Accordingly, the diagnostic app will allow HCP's to set alerts without changing the course of therapy. For example, the user can view the diagnostic app to see the status of the sensor, e.g., time remaining, error messages, and so on, in addition to receiving real-time alerts to mitigate potential adverse events. In the use of the diagnostic app, the alert may be static with episodic data, i.e., not continuous, and not showing glucose profiles or history. The low glucose alert will simply show a number and a trend arrow, or just a number, or the threshold. In this way, users are still required to manage their diabetes in the same manner that was previously used with finger sticks alone. By hiding the glucose profile information, the user is not able to make dramatic therapeutic changes to their current therapy, therefore, such knowledge does not alter the outcome of a diagnostic session.

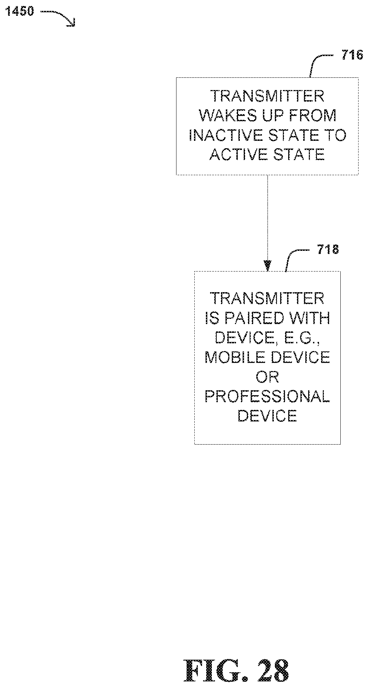

One aspect of the HCP set up process indicated above includes the pairing of the transmitter with a professional product or other smart device. For example, and referring to the flowchart 1450 of Fig. 28, subsequent to the transmitter waking up from the inactive state and entering an active state (step 716), the transmitter is paired with a device, e.g., a professional product, mobile device, HCP device, or the like (step 718). However, pairing the transmitter using traditional manual processes are sometimes cumbersome. Such processes include entering a transmitter ID or otherwise identifying the transmitter to the device to be paired. Such cumbersome and time-consuming processes may deter the HCP from setting up the transmitter for the patient.

Thus, in one implementation, a user friendly and quick method may be employed to identify the transmitter with the HCP device.

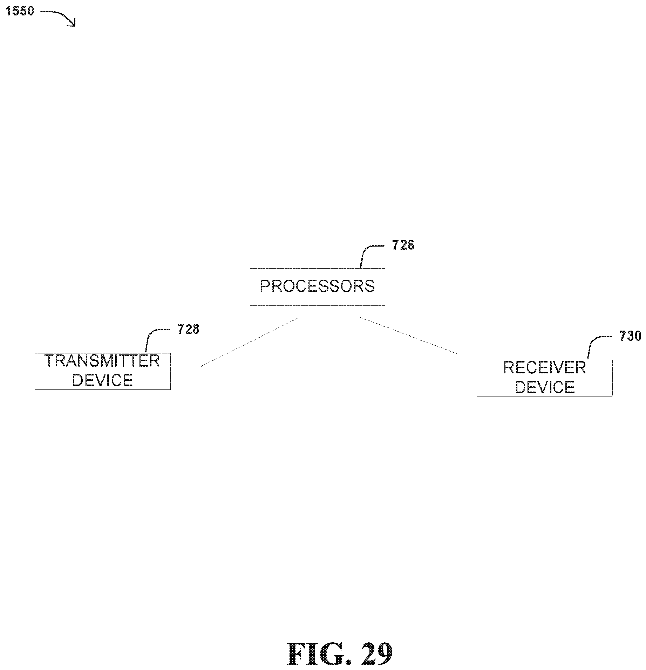

For example, and referring to the schematic diagram of Fig. 29, in one implementation, a system 1550 may be configured to include one or more processors 726, wherein at least one of the processors may be configured to execute a software application or commands to initiate communication between at least one receiver device 728 and at least one transmitter device 730. Here the term "receiver device" is used to refer to any device to which a transmitter may be paired and be in signal communication with, e.g., an HCP device, a professional product, a user device such as a smart phone, and so on.

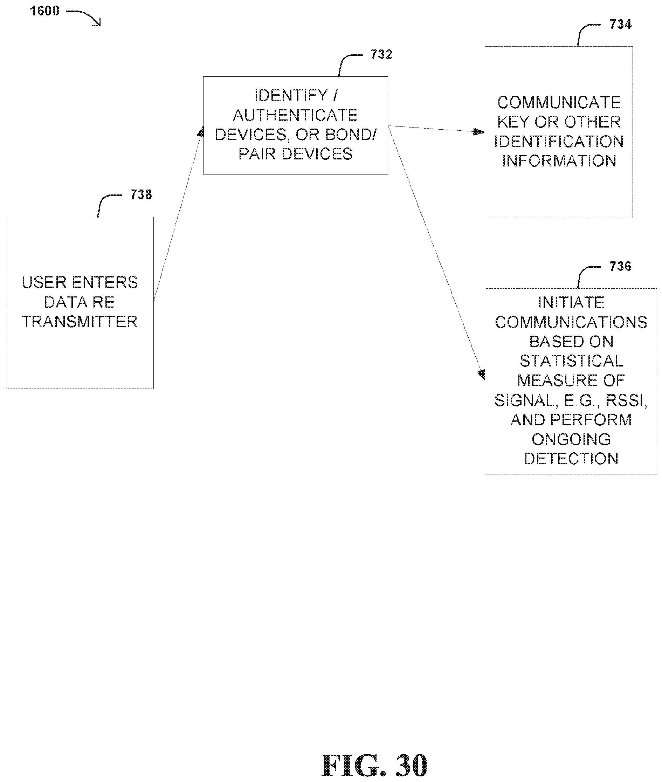

Referring to the flowchart 1600 of Fig. 30, the initiation of communication may include identifying at least one of the transmitter and a receiver device, authenticating at least one of the transmitter and a receiver device, or at least one of bonding and pairing of the devices (step 732). For example, and without limitation, bonding or pairing could include communicatively connecting receiver and transmitter devices including one or more of transmitting a communication key (step 734) such as an identity resolving key (IRK), transmitting transmitter identification information, secure simple pairing, barcode scanning, user input of a code, biometric authentication, etc. In some embodiments, communication may include transmitting data via at least one wireless radio frequency (RF) protocol.

In some implementations, an application key may be used to ensure secure communications between devices. The system may be configured to generate an application key, for instance by either the receiver or the transmitter, or by a central or cloud server associated with a software application. The system may further be configured to use the application key to encrypt information, e.g., data related to an analyte level. The system may further be configured to operate in at least one of a mode wherein the transmitter and receiver are continuously communicatively connected, and a mode where communication is initiated on an intermittent basis. The system may further be configured to operate a receiver device as a gateway to communicate with one or more other receiver devices.

One scenario in which it may be particularly important for pairing the transmitter and a receiver device in a rapid and user friendly manner can occur when a user has a medical emergency and hospital staff or first responders need to access the most recent analyte sensor data. In this situation it would be helpful to bypass the normal authentication techniques that may otherwise be used when pairing the transmitter to a receiving device. Of course, security and privacy concerns still need to be addressed to avoid any potential vulnerabilities. Thus, for instance, emergency responders may be equipped with receiver devices having an app that allows them to access a communication key or the like from a server that has a portal that is only made available to pre-authorized individuals or entities (e.g. hospitals or other emergency care facilities). The communication key or other credentials obtained from the server may allows secure communication to be established between the transmitter and the receiving devices. As another example, the transmitter may be configured to expose a dedicated wireless interface that is only made available to emergency responders.

In some embodiments, the system may be configured to operate in a mode where the transmitter and receiver are continuously communicatively connected. For example, a continuously connected mode may refer to a connection model wherein a connected receiver and transmitter will maintain a connection as long as possible until there is an error or an out-of-range condition.

In some embodiments, the system may be configured to operate in a mode wherein communication is initiated intermittently. For example, the system may transmit data on a periodic basis (where for instance the transmission period is one minute, five minutes, or ten minutes, etc.) and during a transmission window of duration less than the overall period, and to terminate communication for the remainder of the duration of the period. The system may further be configured to modify the periodicity or interval of intermittent communication based on factors such as time of day; an aspect of, or trend in, analyte data; remaining battery life; etc. In this way, for example, a single transmitter could be configured to communicate with multiple receivers during different transmission windows, or vice versa. Power savings could advantageously result from intermittent, rather than continuous, operation of at least one of the transmitter and receiver devices.

In some embodiments, the system may be configured to switch between a first mode where the receiver and transmitter device are continuously communicatively connected, and a second mode wherein communication between a transmitter device and a receiver device is initiated on an intermittent basis. Switching between the continuous and intermittent modes could be initiated, for example, in response to a command from either the transmitter or receiver device, responsive to a user input, and/or could be based on various criteria including type or class of device (e.g. phone, medical device, proprietary receiver or transmitter device, etc.), power supply capacity or constraints (e.g. battery life remaining), a measure of signal quality, time of day, aspects (including trends or statistical measures) of analyte data, etc.

In some embodiments, communication may be initiated based on information or a statistical measure related to a detected signal (step 736). For example, and without limitation, a receiver could be configured to identify a transmitter transmitting a signal based on at least one of information related to the strength of the signal, and information related to the quality of the signal such as at least one of bit error rate (BER) or signal-to-noise ratio (SNR).

By way of non-limiting example, received signal strength indication (RSSI) is a measure of information related to the strength of a detected signal. RSSI is determined from the power being received by an antenna. RSSI is a relative (percentage) measurement that is defined differently by different chip manufacturers, and as such its value for a given power level at an antenna cannot be precisely or uniquely specified. While it may be theoretically possible to determine distance from a transmitter in free space from RSSI values, reliable distance estimation is often impractical, particularly in indoor environments, due to the effects of objects, walls, reflections, multipath interference, etc. Within a given environment associated with a particular receiver/transmitter pair, however, the observed trend in RSSI is generally a reliable indicator of whether the distance between the receiver and the transmitter is increasing or decreasing.

In some embodiments, a receiver device may be configured to detect the presence of one or more transmitter device signals. The receiver device may be configured to monitor the detected transmitter device signals and identify a transmitter based on selection criteria such as at least one of the ongoing detection of a signal, information or statistical measure related to signal strength such as RSSI, and signal quality from the identified transmitter for a predetermined duration of time. In the event that the signal from the identified transmitter does not meet the selection criteria during or after the predetermined duration of time, the system may be configured to continue monitoring the detected transmitter device signals to select a transmitter for the initiation of communication or to employ an alternative method of transmitter identification or selection. The system may further be configured to initiate communication between the receiver device and the selected transmitter device.

In some embodiments, at least one system component, for instance, and without limitation, a receiver device or a display device, may be configured to display a list of detected transmitters to a user via a user interface. The system may be configured to prompt the user to select, from the displayed list of detected transmitters, a transmitter with which to initiate communication. The list may be sorted or prioritized, for example, by at least one of information related to signal strength such as RSSI, information related to signal quality, signal detection duration, and other prioritization criteria. The list may be filtered by threshold criteria, for example, to include signals for which at least one of the foregoing criteria meets predetermined threshold requirements. In some embodiments, the list may be restricted to a predetermined number of detected transmitters based on at least one prioritization criterion, for instance no more than ten transmitters associated with the highest RSSI values.

In some implementations, the user may be prompted to enter information identifying a transmitter (step 738), for instance via a user interface or using a camera or a bar code scanner. Information identifying the transmitter may be obtained, for example, from the transmitter or its packaging. In some implementations, the transmitter identification information may be encoded in a manner not visible to the naked eye, for instance an invisible code detectable by a bar code scanner.

The system may further be configured to confirm the identity or availability of a user-identified transmitter based on selection criteria such as the ongoing detection of at least one of information related to the strength of the signal detected from the selected transmitter (e.g. RSSI) for a predetermined period of time and information related to the quality of the signal detected from the selected transmitter for a predetermined period of time. In some embodiments, the selection criteria may include threshold values of at least one of information related to the detected signal strength and information related to the detected signal quality. In the event that the signal from the user-identified transmitter does not meet the selection criteria during or after the predetermined duration of time, the system may be configured to reiterate confirmation of the identity or availability of a user-selected transmitter based on the selection criteria and display an updated list of detected transmitters for user identification or selection. In some embodiments, the system may be configured to employ an alternative method of transmitter identification or selection before or after a predetermined number of iterations.

In some embodiments, a receiver device could be configured to identify or select a transmitter by detecting RSSI or other signal-related information from one or more transmitters. For example, and without limitation, a transmitter module of a sensor electronics device could be identified or selected based on at least one of: one or more extremal (maximum or minimum) threshold values of RSSI; one or more extremal threshold values of an average of more than one RSSI reading from the same device; a difference between the maximum, minimum or average RSSI values from each of a plurality of detected devices; selecting the maximum peak or average RSSI of all devices discovered within a predetermined time interval; a difference (e.g. delta, variance or trend), derivative, acceleration, or rate of change in RSSI values received from each of one or more discovered devices; a statistical measure such as standard deviation or mean average deviation (MAD) in RSSI values from each of one or more devices; and a filter based on type or class of device.

In some embodiments, one or more detected signals may originate from at least one of a sensor electronics device and a transmitter module configured to transmit at least one of an advertising signal, beacon, and signals related to an analyte level. In some implementations, the receiver device may be incorporated in a mobile phone or other electronic device. For instance, and without limitation, a transmitter module of a sensor electronics device may be configured to transmit an advertising signal when it is operatively coupled to an analyte sensor, when the analyte sensor is transcutaneously positioned, or when both of the foregoing conditions are met. Upon detection of the advertising signal, a receiver device may be configured to bond or pair with the transmitter, to prompt a user to move the receiver in close proximity to, or farther away from, the sensor electronics, or vice versa. The receiver may be configured to identify or select the transmitter based on a value derived from the RSSI associated with the advertising signal including the RSSI itself.

In some embodiments, the receiver may further be configured provide an indication to the user that a transmitter has been identified. The indication may for instance comprise feedback from the identified transmitter (for example a light, beep or other auditory signal, or haptic feedback) or a notification to the user via a user interface of a mobile phone or other electronic device, which may include information identifying the transmitter. The user may be prompted to select or verify the identity of the transmitter.

In some embodiments, if a detected RSSI or other signal-related related parameter or statistical measure exceeds a predetermined maximum threshold value, for instance when the transmitter and receiver are in close proximity, the receiver may be configured to prompt a user to move the receiver device farther away from the sensor electronics so that the sensor electronics can be identified based on a change in the RSSI or other information related to the signal strength or quality. The receiver may optionally further be configured to prompt the user to subsequently move the receiver in close proximity to the sensor electronics to further determine or verify the identity of the transmitter. For example, and without limitation, the receiver may be configured to first prompt the user to move the receiver device farther away from the sensor electronics when the RSSI exceeds a predetermined maximum threshold value, and subsequently prompt the user to move the receiver in close proximity to the sensor electronics after the RSSI falls below a predetermined minimum threshold value.

In some embodiments, a transmitter device may further comprise an accelerometer, optical or infrared detector, microphone or other sensor to aid in identifying the transmitter. For instance, and without limitation, a transmitter device of a sensor electronics module may be configured to begin to transmit an advertising signal when a user causes an accelerometer to generate a signal, for example by tapping the sensor electronics module. Alternatively, upon detection of an advertising signal, a receiver device may be configured to prompt a user to touch or tap a sensor electronics module. When the user touches or taps the sensor electronics in response to the prompt, a resulting accelerometer or other sensor signal may be transmitted to the receiver, which may be configured to receive the accelerometer or other sensor signal. The accelerometer or other sensor signal may be used for the purpose of identifying or selecting the transmitter. In some embodiments, touching or tapping of the transmitter could be used as a verification step, for example to confirm that a transmitter has been identified or selected, or that communication has been initiated.

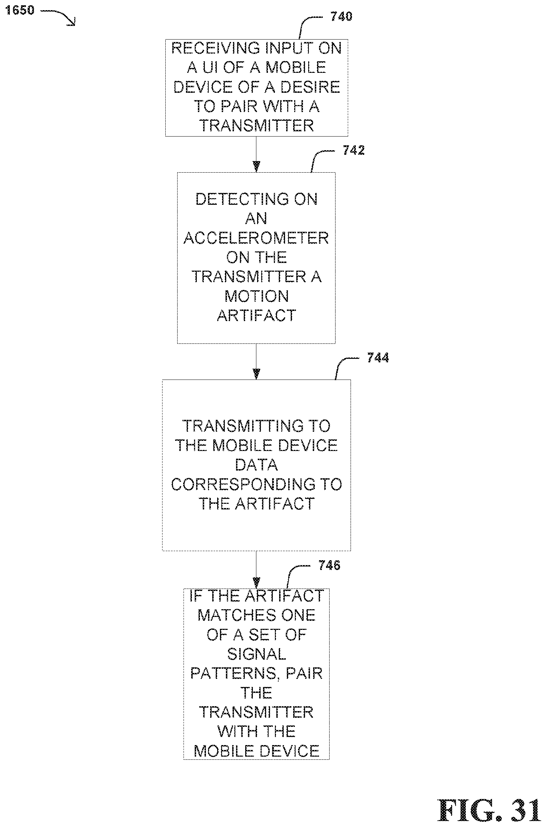

Similarly, and referring in addition to the flowchart 1650 of Fig. 31, input may be received on the UI of a mobile device indicating a desire to pair the mobile device with a transmitter (step 740). A motion change or artifact caused by the user may then be detected on an accelerometer on the transmitter (step 742). Data corresponding to the artifact may be transmitted to the mobile device (step 744). If the artifact matches one of a set of signal patterns, the transmitter may be subsequently paired with the mobile device (step 746). Such motion artifacts may include, e.g., tapping three times, shaking the transmitter for five seconds, jumping up and down three times, and so on. Alternatively, the motion artifact on the transmitter may automatically put the transmitter in the pairing mode, without the need to compare waveforms or signal patterns. In this implementation, the transmitter ID appears on the phone/receiver and automatically pairs, or the user confirms the intention to pair.

In some embodiments, a transmitter device may be configured to detect the presence of receiver device signals. The transmitter device may be configured to monitor the detected receiver device signals and identify a receiver based on selection criteria such as at least one of the ongoing detection of a signal, information or statistical measure related to signal strength such as RSSI, and signal quality from the identified receiver for a predetermined duration of time. In the event that the signal from the identified receiver does not meet the selection criteria during or after the predetermined duration of time, the system may be configured to continue monitoring the detected receiver device signals to select a receiver for the initiation of communication or to employ an alternative method of receiver identification or selection. The system may further be configured to initiate communication between the transmitter device and the selected receiver device.

By way of non-limiting example, the transmitter may be configured to transmit information related to the strength of the signal from the receiver, such as RSSI, to the receiver device. In some embodiments, at least one of a receiver and a transmitter may be identified or selected using RSSI or other signal-related information from either or both of the transmitter or receiver modules in accordance with the methods described herein.

In some embodiments, a transmitter device may be configured to identify or select a receiver by detecting RSSI or other signal-related information from one or more receivers. For example, and without limitation, a sensor electronics device may be identified or selected based on at least one of: one or more extremal (maximum or minimum) threshold values of RSSI; one or more extremal threshold values of an average of more than one RSSI reading from the same device; a difference between the maximum, minimum or average RSSI values from each of a plurality of detected devices; selecting the maximum peak or average RSSI of all devices discovered within a predetermined time interval; a difference, e.g. delta, variance or trend, derivative, acceleration, or rate of change in RSSI values received from each of one or more discovered devices; a statistical measure such as standard deviation or mean average deviation (MAD) in RSSI values from each of one or more devices; and a filter based on type or class of device.

In some embodiments, the system may be configured provide an indication to the user that a receiver has been identified. The indication may for example include feedback from the transmitter or identified receiver (for example a light, beep or other auditory signal, or haptic feedback) or a notification to the user via a user interface of a mobile phone or other electronic device, which may include information identifying at least one of the receiver device and the transmitter device. The user may be prompted to select a device for communication or verify the identification.

In some embodiments, the receiver may be configured to transmit information related to the RSSI of the transmitter, or other signal-related information, to the transmitter. The receiver may be identified or selected using RSSI or other signal-related information from either or both of the transmitter or receiver modules in accordance with the methods described herein.

The foregoing embodiments are included by way of illustration only. One of ordinary skill in the art will readily appreciate that the implementations of the systems and methods disclosed herein are not limited to the described embodiments. For instance, and without limitation, the various methods could be implemented by one or more of a receiver device, a transmitter device, sensor electronics, a display device, a mobile phone, a tablet, a computer, a wearable monitor (e.g. smart bracelet, smart watch, smart ring, smart necklace or pendant, workout monitor, fitness monitor, health or medical monitor, clip-on monitor, or the like), an adhesive sensor, smart textiles or clothing incorporating sensors, shoe inserts or insoles that include sensors, transdermal (i.e. transcutaneous) sensors, swallowed, inhaled or implantable sensors, or another electronic device, and the various systems could comprise any device or devices that could be configured to implement a given method described herein. Similarly one or more of any indications of signal strength or quality, or any information or statistical measure derived therefrom, could be substituted for RSSI.

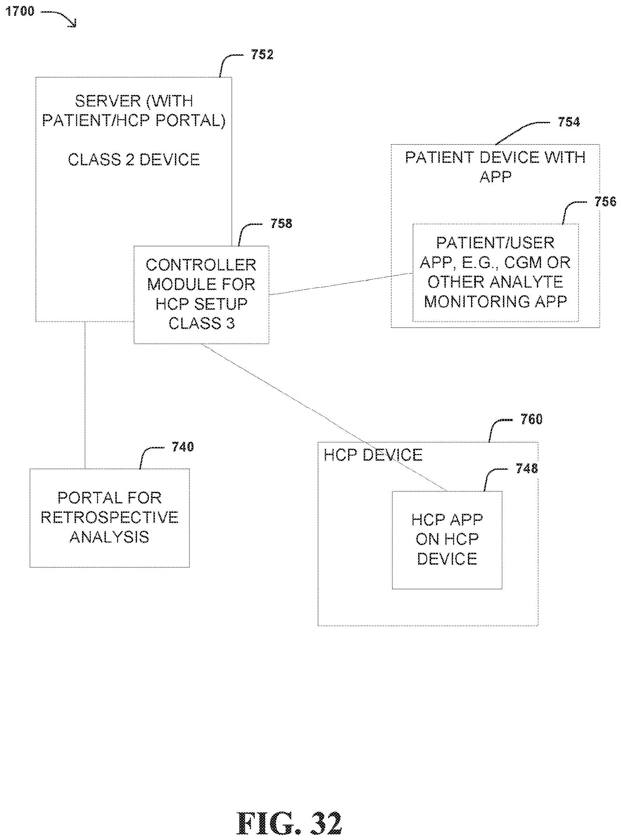

In systems and methods noted above, and referring to the system 1700 illustrated in Fig. 32, an HCP uses an HCP device 760 running an HCP app 748 to access a server 752 to which patient data is communicated. In addition, a patient device 754 may be in communication with the server 752 as well, where the patient device may be, e.g., a professional product, a patient smart device, and so on. The patient device 754 may be operating a patient/user app 756, where the app is, e.g., a CGM monitoring app or the like. The app 756 may further allow communications with the transmitter.

As the app 756 is closely related to patient health, it is generally a highly regulated class III medical device. The server 752, however, is usually employed for various types of retrospective analysis, e.g., using a portal 740, and thus the server may be less highly regulated, e.g., may be classified as a class II medical device. The HCP app 748, however, configures the patient app 756 and/or the patient device 754through the server 752. This configuration may consequently affect the classification of the server 752.

Thus, to address these issues, a controller module 758 may be implemented within the server 752. The controller module 758 is employed by the HCP app 748 to configure the patient app 756 (and any other needed functionality) of the patient device 754. In this way, the HCP set up portion, related to application configuration, is kept separate from the retrospective analysis portion of server functionality. In this case, the controller module may be classified as a class III medical device while the server maintains its classification as a class II medical device.

### Post-Session Battery Conservation and Transmitter Wake Up, Including for Data Extraction

As mentioned above, most HCPs lack significant time to either set up the transmitter for the patient at the beginning of a session or to extract the data from the transmitter at the conclusion of a session. Thus, rapid and convenient ways of performing such tasks are highly desired. Referring again to Fig. 4, a reader device 420 in combination with an HCP app 429 may provide a particularly convenient and uncomplicated way to perform the needed steps.

In one implementation, the HCP app is an application running on an HCP device such as the HCP computer. In another implementation, the HCP app may run on a mobile device and incorporate features of the HCP desktop app. Such features generally include, e.g., but are not limited to, supplying a timestamp to the transmitter, verifying sensor insertion and proper startup, providing an interface through which an HCP may enter patient information, as well as downloading data from the transmitter and uploading the same to a server. Appropriate devices may include, e.g., Android phones, iPhones, and other mobile devices.

For example, one category of devices that may be employed are smart phones, e.g., user smart devices or phones that are dedicated to this purpose, e.g., professional HCP products. Alternatively, a device with RF wireless communication capabilities could be connected to the HCP device, e.g., HCP computer, all the time. For example, the dongle 426 may be provided that is connected all the time and which may include multiple antennas, e.g., one antenna for NFC and one antenna for BLE. The two antennas may be provided in a unitary housing or in separated housing.

Advantages of BLE communications include that the transmitter need not be very close (unlike NFC) to the computer or dongle to perform the various steps, these various steps including transmitter set up during the initial stage after the sensor has been inserted, and which includes further waking up the transmitter.

However, to upload data to the dongle after the sensor session, an NFC enabled dongle may be employed which also serves the purpose of waking up the transmitter prior to downloading the data from the same. Advantages of the use of a dongle include that the same can generally include large NFC antennas, which are easier to use than the smaller NFC antennas found in mobile devices. The dongle can also be an active NFC device, rather than a passive NFC device. Some communications can be performed via BLE, while others can be performed via NFC. Factors determining which wireless protocol is desired may include, e.g., how long the data download or upload takes, the distance between the devices, and so on.

In addition to using the dongle, the transmitter may function in one or more different modes. For example, in one implementation, the dongle (using, e.g., NFC) operated by commands from the HCP app causes the transmitter to function in two modes. A first mode is an HCP mode. In the HCP mode, the HCP may be enabled to perform functionality such as transferring the timestamp, verifying operation, and so on. The HCP mode may last for a predetermined amount of time, e.g., 10 minutes, after which a default "normal" mode is automatically entered, e.g., for regular wear during a sensor session. A benefit of HCP mode is security, e.g., in HCP mode the transmitter may be configured such that no other communications can take place. In this way, information cannot be obtained from the transmitter other than through the dongle. BLE communications can also be enabled during this predetermined time period of the HCP mode. Once back in the normal mode, ordinary and normal communications can take place.

In one implementation, particularly for data extraction, wake up of the transmitter can occur through NFC, but data transfer can occur through BLE, as BLE is generally considerably faster than NFC. In some implementations, the dongle can be provided with an appropriate ASIC or programmed processor to provide some intelligence in the decisions and determinations it is configured to calculate, e.g., the dongle may determine how best to transfer the data, given other factors in the receiver and transmitter, e.g., whether it is better to transfer data via NFC or BLE. And as noted in Fig. 4, the dongle may have a target 422 disposed thereon so the user is aware of where to put the transmitter for NFC communications.

After a sensor session is over, in order to save battery power in the transmitter so that the transmitter may be powered up for data extraction, various steps may be taken. For example, the transmitter battery may be temporarily disconnected. In another implementation, the transmitter may enter an inactive mode, which may also be termed "hibernation", sleep mode, or just generally an "inactive mode". The transmitter may be caused to enter such a mode following the temporal end of the sensor session, e.g., following 7 days, 14 days, and so on. Various technologies disclosed below may be employed to wake the transmitter from such a mode, so that data can be extracted from the same.

For example, in one implementation, a wake-up circuit can be employed in the transmitter electronics that can wake up the transmitter immediately and speed the startup process. In more detail, the wake-up circuit includes functionality to wake the wireless transmitter up after the same has been turned off or put into a low-power state to conserve battery life after one or more measurements have been taken over a period of time. The wake-up circuit wakes the transmitter, to wirelessly transmit the one or more measurements to a dedicated display or other smart device, e.g., a professional product. When using the wake up circuit, the sensor electronics module or transmitter can be configured such that it remains in a low power or storage mode until just before completion of the manufacturing process, e.g., prior to shipment by the manufacturer or distributor, for example, so that the sensor electronics module does not consume power while sitting in storage at the manufacturer or distributor. In the same way, the sensor electronics module may also enter "low power" or "no power" modes during certain periods of inactivity, e.g., T_{inactive} periods, e.g., in between wireless communication sessions. It should be noted that in some embodiments, one or more components making up the sensor electronics module, such as the transceiver, may wake up or power down, while one or more other components may remain in low power/sleep mode. In other embodiments, it may be that all the components making up the sensor electronics module wake up or enter a low power/sleep mode. Such components within the sensor electronics module or transmitter may include a power down module, which using hardware, software, or firmware, can enable the transmitter to transition from an active state to an inactive state.

Waking up and powering on the sensor electronics module (e.g., causing the sensor electronics module to enter an active or operational mode, such as during T_{inactive} periods) can occur, in one embodiment, when a sensor reading above a predetermined threshold is detected, e.g., where the predetermined threshold can be specified in terms of counts or via other means e.g., by use of a potentiostat, A/D converter, or the like. In some implementations, the processor of the sensor electronics module (or other controller chip or device of the sensor electronics module) may wake up periodically, e.g., every five minutes, to monitor counts. If the number of counts received is below a predetermined count threshold, the processor or controller may return to a low power mode. If the number of counts received exceeds the predetermined count threshold, the processor or controller wakes up and processes and/or forwards sensor information to the telemetry module to be delivered to one or more of the display devices. Also, in some implementations, the processor or other controller of the sensor electronics module may remain in operational mode to monitor counts to determine whether the sensor electronics module is to begin receiving/obtaining and/or processing sensor information from the continuous analyte sensor, and may perform such continually occurring periodic monitoring checks several or more times.

Systems and methods according to present principles may incorporate functionality so as to avoid potential issues of the above wake-up circuit, in particular, false or missed wakeups. for example, some embodiments rely on a benchmarked count threshold of approximately X counts (e.g., 9000 counts) which would generally be received over the application of current for approximately Y seconds or minutes (e.g., 300 seconds or 5 minutes). In still other embodiments, the benchmarked count threshold can be monitored in the context of a persistent condition, e.g., where the persistent condition can include a consistent frequency of counts over a subset of the Y time duration.

In implementations of the above method, an initial determination may be made that the number of received counts meets or exceeds the benchmarked count threshold, and then another determination can be made to determine whether the number of received counts meets or exceeds a second benchmarked count threshold (U) for a second period of time (V). For example, the processor, or in general, the sensor electronics module, will not wake up unless the second benchmarked count threshold U is met or exceeded for the second period of time V.

The operation can further include determining that the second benchmarked count threshold U is met or exceeded for the second period of time V over multiple intervals (n). This method may be used to check persistence, i.e., a verification step to ensure the sensor electronics module is really meant to wake up from a low power storage mode due to insertion of the continuous analyte sensor as opposed to a false wake up due to inadvertent contact by the user with the sensor electronics module, for example. The implementation can ensure that counts are generated indicative of real sensor data and not counts from electrostatic discharge (ESI) based data, which tends to come in bursts.

Various embodiments described above can address scenarios to conserve battery power in the context of, e.g., the sensor electronics module, being in a low power or storage mode. For example, the sensor electronics module may enter low power or no power modes during certain periods of inactivity, e.g., T_{inactive} periods, e.g., in between wireless communication sessions. However, in a scenario where the sensor electronics module should wake up in order to receive and transmit sensor information, it would be problematic if the sensor electronics module failed to wake up.

Accordingly, some embodiments implement a mechanism to ensure that the sensor electronics module wakes up when warranted. In particular, some embodiments utilize a watchdog timer. A watchdog timer is a hardware feature for an electronic component or circuit that has a countdown timer. A watchdog timer can be used to detect system anomalies or malfunctions and recover from them. For example, if the countdown timer reaches 0, the chip will reset. Since the watchdog is hardware and not software controlled, software errors will not interfere with its operation.

Additional details of such a wake-up circuit are described in US Patent Application Serial Number 62/270,485, filed December 21, 2015, entitled "Continuous Analyte Monitoring System Power Conservation", owned by the assignee of the present application and herein incorporated by reference in its entirety.

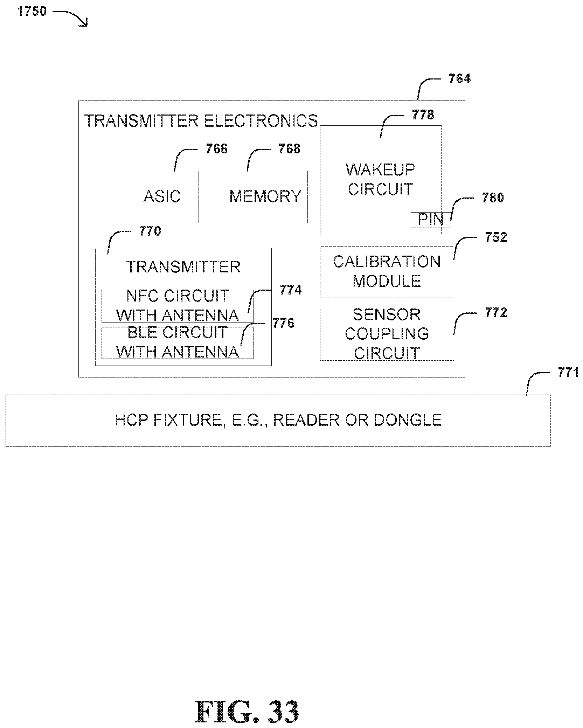

In another embodiment, the system 1750 of Fig. 33 illustrates an exemplary wakeup circuit that can be used within transmitter electronics to wake the transmitter immediately and to speed up the startup process. For example, the wake-up circuit can be used to bring the transmitter out of storage mode, and data extraction can even be initiated by the wake-up circuit. For example, if the operator/HCP removes the transmitter from the sensor, and connects the transmitter to a fixture, e.g., the reader, the transmitter can be configured to begin advertising and the pairing and extraction process can begin. Such can include Bluetooth pairing involving hashtags and challenge values to create a secure link.

In more detail, Fig. 33 illustrates transmitter electronics 764 resting adjacent an HCP fixture such as a reader 771, which may be implemented as a dongle attached to the HCP device. The transmitter electronics 764 includes an ASIC 766 and memory 768. A transmitter 770 is configured within the transmitter electronics to communicate with external devices, and the same may include an NFC circuit 774 and a BLE circuit 776. A calibration module 752 is shown, which may perform calibration of the sensor within the confines of transmitter electronics. Otherwise, such calibration can be performed on a smart device in signal communication with the transmitter. A sensor coupling circuit 772 allows conductive communications with wires from the indwelling analyte sensor. A wake-up circuit 778 is provided to accomplish the functionality of the wakeup circuit noted above. As shown, the wakeup circuit 778 may include a wake-up pin 780 which allows convenient access during debugging, i.e., so that wake-up can be quickly performed. For example, in an appropriately-configured wake up circuit, a 'high' level of potential may be applied to such a wake up pin 780 and the circuit may be such that the same initiates a routine that results in a waking up of the transmitter. Alternatively, the logical low level of potential may be applied. During the remainder of the time, the pin may be left floating.

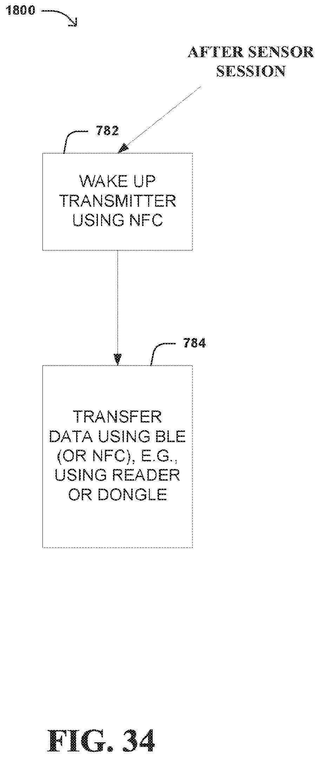

Referring in addition to the flowchart 1800 of Fig. 34, following conclusion of a sensor session, one way of waking up the transmitter is by interrogating the same with an NFC signal (step 782), e.g., to initiate a query to the transmitter for information on demand. Data may then be transferred using NFC or another means, e.g., BLE (step 784). It is noted in this connection that NFC may also be used to transfer data, and BLE could also be used for the wakeup signal. Generally NFC is preferred for the wakeup due to its speed and lower power usage; however, security for NFC is generally only by way of proximity.

An issue encountered with the above devices, however, includes false startups. For example, energy captured by the NFC antenna may lead to false startups and data degradation. Where a wake-up circuit is provided with a wake-up pin, electromagnetic energy may couple to the pin and cause similar deleterious startups, data degradation, and battery drain (it is generally desired to keep, e.g., 10% of battery power remaining for data extraction). In a particular example, energy captured by the NFC antenna may couple to the sensor circuitry or to the power circuit, e.g., power rails connected to the ASIC or to the rectifier. The coupled energy may give rise to noise in the sensor data and adversely affect transmitter electronics. Accordingly, it is important to know when to consider the sensor data and when to disregard the sensor data during various intentional and unintentional NFC operations.

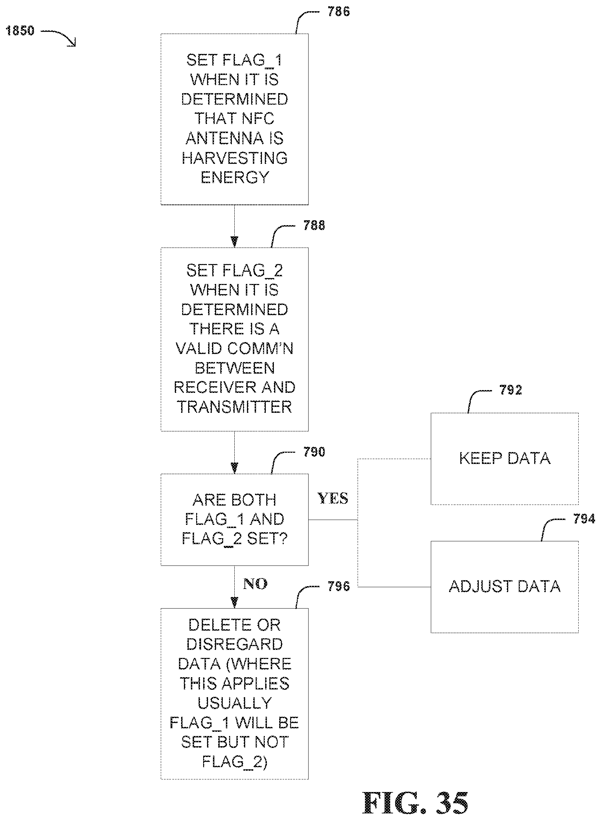

An exemplary method of doing so is provided by the flowchart 1850 of Fig. 35. In this method, interrupt flags are employed to identify certain relevant key points of data transfer. A feedback mechanism is used to identify when the circuit is harvesting energy and flag the timecorresponding sensor data appropriately. For example, interrupt flags may be employed in the following way. A first flag, FLAG 1, may be set when it is determined that energy harvesting is occurring by the NFC antenna/circuity (step 786). Similarly, a second flag, FLAG_--2, may be set when it is determined that there is a valid NFC communication occurring between the transmitter and a receiver, e.g., a smart device (step 788). For example, FLAG 2 is set during a valid data exchange between the transmitter and a receiver.

A test is performed as to whether both FLAG_1 and FLAG_2 are set (step 790). If the test is positive, then it may be determined that intentional data is being exchanged via NFC. In this case, in one implementation, sample data corresponding to such a time period may be processed so as to compensate accordingly. For example, the processor may initially flag sensor data that are measured or sampled during an NFC operation, the data may then be kept (step 792) or adjusted (step 794). Other options include to weight the data differently, filter the data, and so on.

On the other hand, if only FLAG_1 is set but not FLAG_2, then it may be determined that unintentional data is being captured via NFC, e.g., random noise is being captured from other RFID sources. While various steps may be taken, in one implementation, such data may be removed, deleted, or otherwise disregarded from future calculations (step 796).

In another implementation, an adjustable sampling rate may be employed when using NFC. For example, adjustable sampling can be used to discriminate whether NFC is affecting the signal. In this case, the sampling rate may be changed based on whether, e.g., FLAG 1 is set.

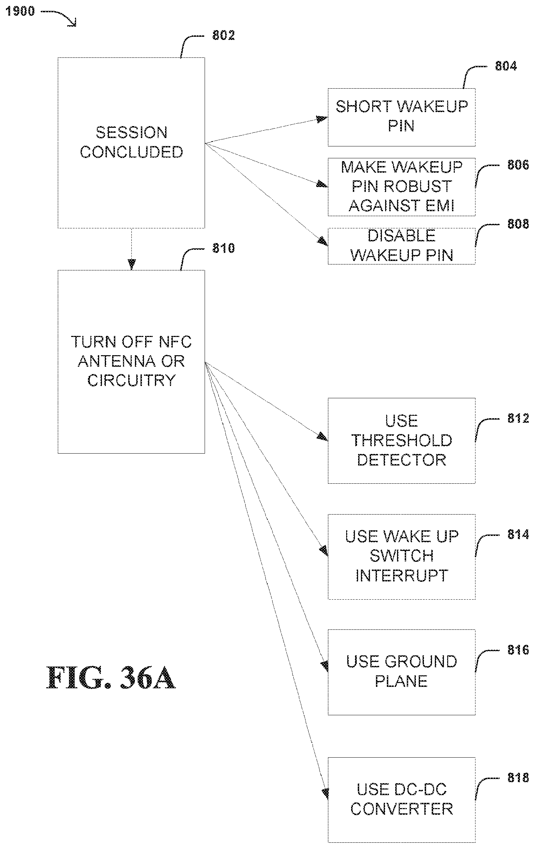

Referring to the flowchart 1900 of Fig. 36A, hardware approaches may also be employed to address this issue. In particular, following conclusion of a session (step 802), such hardware approaches may involve acts taken to disrupt to the action of the wake-up pin or turning off the NFC antenna or circuitry (step 810).

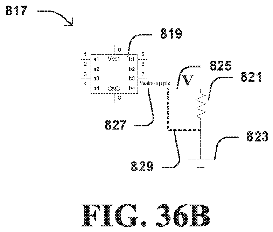

In the former approach, it is noted that the wake-up pin is generally floating with potentially a pull up/down resistor, e.g., which may be a resistor extending between the wake-up pin and ground. See, e.g., Fig. 36B, in which a transmitter system 817 includes a transmitter chip 819 having a wake-up pin 827 which is connected through a resistor 821 to ground 823. If no current is flowing, then the wake-up pin is at the same voltage as ground, e.g., the voltage V at point 825 is the same as ground. However, with enough electromagnetic induction (EMI), it is possible that false transitions on this pin can be generated, causing the voltage at point 825 to be different from ground, and resulting in erroneous wake ups. Typically EMI is something that occurs intermittently. However, if EMI triggers multiple erroneous wake up, such may begin to significantly and deleteriously impact battery life. During a wake-up event, the battery is automatically connected to the processor, drawing additional current as compared to the storage mode current draw. This results in a reduced use life.

Thus, in one implementation, the wake-up pin may be shorted (step 804). For example, a jumper 829 may be employed to connect the wake-up pin to ground (see Fig. 36B). Alternatively the wake-up pin may be hardened or otherwise made more robust against EMI (step 806). For example, a strong pull up/down resistor may be employed, and if needed, a capacitor as well. Finally, the wake-up pin may be effectively disabled prior to entering storage mode (step 808), e.g., by providing an instruction to the wake-up pin through the ASIC.

In the latter approach, one implementation is to use a threshold detector (step 812). For example, a detector mechanism may be implemented to identify energy captured by the NFC antenna, and such captured energy may be monitored and compared against a threshold. If the threshold is met or exceeded, the NFC circuitry and/or the NFC antenna may be shut off by way of a switch.

In another implementation, a wake-up switch interrupt may be employed (step 814). For example, if it is determined that the NFC antenna or circuitry is being used only to wake up the transmitter from the sleep mode, then the NFC antenna/circuitry can be disabled after the wakeup process is completed. In this way, the NFC antenna will no longer pick up random signals.

In yet another implementation, a ground plane may be employed (step 816). In this implementation, an additional ground plane may be employed to dump the excess energy captured by the NFC antenna. In yet another implementation, excess NFC energy captured may be used as a flag to operate a DC to DC converter (step 818).

Wake up circuits as noted above can be significantly beneficial by providing a convenient way to activate the transmitter. However, the same are not without disadvantages. For example, some use cases call for a patient to remove the entire sensor/transmitter/adhesive patch assembly following a sensor session, and to place the same in a disposable bag; however, in such cases errant currents may pass between the sensor leads, causing false start ups and draining the transmitter battery, particularly if the environment in which the transmitter is placed is such that material may periodically cause a conductive path between the sensor leads. Accordingly, battery power may be saved on the transmitter by disabling the wake-up circuit. Ways of doing so are described below.

One way of effectively disabling the wake-up circuit is to increase the threshold potential required to be measured (using the techniques noted above) for processor verification of sensor operation, e.g., by increasing the benchmarked count threshold noted above. In this way, an accidental wake-up is likely to be immediately noticed as false, at least by the time of the next threshold measurement, e.g., as noted above where such are measured over multiple intervals, and thus the system will reenter storage mode more quickly.

Another approach to increasing consumer health is not just to extend sensor session life by considerations of battery drain but also to render sensors and transmitters even smaller and potentially disposable. For example, some users may desire to purchase such a device over the counter and use the same over a period of two weeks to obtain an idea of their glycemic variability, even where such may not yield real time data. Such a disposable CGM may simply collect data and periodically allow for data transfers. For reduced cost, and efficiency of data transfer, the disposable CGM may collect the data and periodically downsample the data so only those few points required to accurately illustrate glycemic exposure in response are kept. For example, the CGM and transmitter may only keep data about maxima, minima, and certain inflection points.

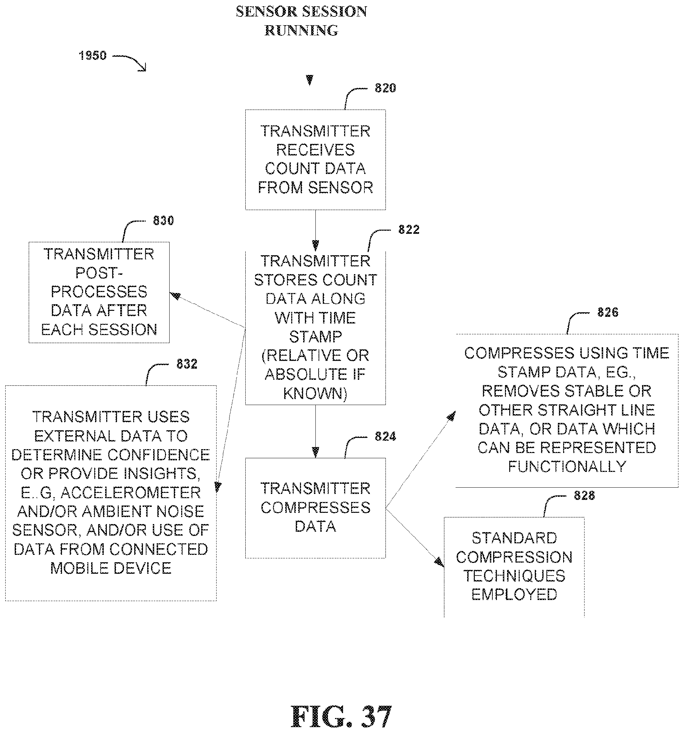

In more detail, and referring to the flowchart 1950 of Fig. 37, during a sensor session a transmitter may receive count data from a sensor (step 820). The transmitter may store the count data along with a timestamp (step 822). For example, the timestamp may be an absolute time stamp indicating a date and time, or the timestamp may be relative, indicating that the transmitter has been in use, e.g., for 1000 minutes during this sensor session.

The transmitter may include various ways of compressing the data (step 824). As noted, the transmitter may compress the data (step 826), and such compression may include using the timestamp data. Stable or other straight-line data, or data which can be represented functionally, may be removed and replaced with just end point data or data about the function. For example, stable data can be eliminated by only saving and transmitting data points between inflection points. For example, if glucose is stable at 90 mg per DL for two hours and then slowly reaches 95 mg per DL, the data between can be eliminated and only the endpoints stored and/or transmitted. Alternatively, only data that changes more than a predefined amount, e.g., 5 or 10 mg/dl, may be recorded and/or transmitted, and the data in between interpolated.

Other standard data compression techniques may also be employed (step 828). Such data compression techniques may include Lempel-Ziv compression, Huffman encoding, or algorithmic encoding. It is expected that using such techniques, data transfer rates may be high enough to transfer an entire sensor session worth of data in just a few seconds.

As another aspect, a CGM that is disposable in this way need not necessarily contain or include a calibration or conversion algorithm. Such processing may be provided elsewhere, e.g., on a display device or in the cloud.

As the use is retrospective, certain artifacts can be removed altogether so as to avoid the possibility of misinterpretation. These include artifacts such as "dip and recover" faults and PSD type artifacts. Such data segments may be blocked out on the display as "glucose data not available" or the like. If artifacts are short enough in duration, the simplified glucose trace need not be impacted at all.

Data downloading or extraction may be as noted above, or in a particularly simple implementation, a Wi-Fi hub or the like could be provided. Such a data hub could be used for all remote health data capture/transfer, not only CGM, but rather as part of a healthcare subscription including data such as heart rate, weight, blood pressure, and the like.

Advantages include cost and data transfer time. In particular, cost is reduced by avoiding data transmission, and by choosing passive communications technologies. Time and cost of memory storage is reduced by intelligently down sampling the hundreds of data points each day to a much fewer number, e.g., 10 - 20 points, and further incorporating lossless data compression methods.

To further increase accuracy, data may be postprocessed in the transmitter, e.g., may undergo steps of smoothing or the like to improve accuracy (step 830 of Fig. G5). The postprocessing may occur, e.g., after each CGM session, e.g., which may be one week long, 10 days long, 14 days long, and so on. In this way, a need may be eliminated for external software applications in which to perform such post processing of raw data. In another implementation, data may be postprocessed at the end of each 24 hour period. Any smoothing, and lessons learned or insights gained, can be applied proactively while the user is still wearing the device, potentially maximizing the overall accuracy potential after the entire CGM sensor session. As a result of such retrospective processing, range bars of glucose data on trend graphs may narrow over time as additional data is gained by retrospective processing.

Contextual awareness can also be employed to provide additional insights, and to provide a degree of "calibration". For example, during meal time, rapid rates of change are expected. Such contextual data can be gleaned by time of day data, including analysis of historic patterns, e.g., the time that the user usually eats lunch. Contextual data can also be gleaned from geolocation data, e.g., if the user is in the same location as a restaurant known to be frequented, and so on. Sensitivity of key algorithms can be increased or decreased during these meal time periods based on the contextual information and reference time frames, in addition to historical patterns of user activity. Timestamp data may also be employed in this context.

As a further way of increasing accuracy and confidence of the data, the transmitter may use available external data to provide one or more insights (step 832). For example, if the transmitter uses an accelerometer or other method of measuring activity, or if the transmitter is in signal communication with a display device that includes an accelerometer, accelerometer data may be employed to determine various insights. For example, such insights may include reasons for particularly noisy data, and if such noise is due to user exercise or a signal fault. For example, long periods of activity and/or short periods of strenuous activity as measured by the accelerometer would increase the sensitivity of algorithms used to predict or measure glucose. Confidence levels of rates of change may then be increased if sustained exercise activity is measured. In other words, if the transmitter or app knows the user is exercising, then data will be maintained, as confidence may increase that noisy data is not caused by noise in the data.

Ambient noise sensors may be employed in the same way. For example, such ambient noise sensors may be employed to identify sleep or other times when prolonged periods of significant ambient noise reduction is identified. Such may also be correlated with time of day to even more accurately identify sleeping intervals.

Using all such gathered data, statistical analyses may be performed at the end of a diagnostic session, such may provide an indication for the user of their performance, and such may further provide a convenient overall "score" for the clinician use after the CGM period. Key statistics may include "% time in target range" or "% time in hypoglycemia", hypoglycemic risk indicators, and so on. This overall "score" can be used to "triage" patients that are at higher risk to trigger work flow for the clinician to focus on these individuals first when reviewing data. This may work as a precursor to any detailed statistical report or more formal clinical decision support software.

For example, a large diabetes center or a large diagnostic lab may process diagnostic CGM data from 100 patients in a day, thus generating 100 individual reports. The results and corresponding patients can be triaged based on the overall "risk score" provided so the clinicians can reach out to the high risk patients first and address any ongoing concerns identified with CGM data.

### Examples

In one specific exemplary implementation, a small single use disposable sensor product may be provided with a "smart" transmitter, i.e., transmitter that provides one or more processing functions such as the above-noted smoothing, and the same may be configured to push data to a receiver such as a professional product or a user smart device. After use, the entire system, i.e., transmitter and sensor and adhesive patch, may be returned to the HCP for data extraction and analysis. In some cases, data extraction will have already occurred by a connection between the transmitter and a receiver. Such a device may be calibrated a single time or be provided with factory calibration. Existing applicator technology may be employed to install the sensor into the user and in some cases to also snap a transmitter onto the sensor housing. Such technology is described in, e.g., US Patent Application Serial Number 15/298,721, filed October 20, 2016, entitled "Transcutaneous Analyte Sensors, Applicators Therefore, and Associated Methods", owned by the assignee of the present application and herein incorporated by reference in its entirety.

In another specific exemplary implementation, a small single use disposable sensor product may be provided with a "smart" transmitter as noted above, and the same may be used primarily for non-adjunctive purposes. Data from such a smart transmitter may be shared with a third party, even during a sensor session. Such third parties include, e.g., payors. Such a device may be factory-calibrated and in some cases even available without a prescription. A deployment system may be provided to install the same in a patient.

Implementations of systems and methods according to present principles may include the provision of features such as a target range, the creation of soft alarms or notifications that provide education to the user after the fact, e.g., not necessarily in real time, providing event entries to allow entry of target medication compliance, provision of an appropriate API to configure event entry from meal apps or directly from a user interface by the user, provision of an appropriate API to configure event entry from Health Kit and the like, and so on.

Other implementations include that an HCP office may use their encrypted email system to communicate back and forth with patients. Such information may be provided "all in one place", e.g., as part of an electronic medical record. A file corresponding to the 14-day (or other sensor session duration) report may be uploaded directly to the EMR and provided in a printable format.

In some implementations, a home screen may be provided on the receiver device that indicates trends, blood glucose numbers, an indication of whether the patient is "in range" or "out of range", and such thresholds for such ranges may be set by the HCP as part of HCP set up. Events may be indicated using a date/time stamp. Statistics may be provided in which a user or HCP could compare one day against another. Patients may be enabled to use such data to learn "cause-and-effect" based on what they see on their home screen, and in this way be enabled or feel empowered to learn such aspects themselves.

The patient app may provide color coding to show when blood sugar levels are high, low, or in target. Smart phone notifications may be configured as well, indicating to the user if they are high or low based on alerts that have been set.

In some implementations, during a sensor session, if a connected receiver is employed, the HCP may be informed if the patient is staying high or is having a hypoglycemic event.

The HCP app may be configured to provide the HCP with a report including data such as average blood glucose, average A1c, frequency of hypoglycemic events, percentage of time in, above, and below a target range. The app may overlay results to illustrate trends. The app may post events including those pertaining to nutrition, stress, activity, illness, infections, sleep patterns, and the like. Such an app may be available at least shortly before a patient visit to allow HCP review to better inform patient consultation, and the app may highlight certain critical information to allow the HCP to better formulate a therapy plan for the patient. The highlighting of certain critical information may include by comparing detected or determined patterns to criteria stored in a database, with the criteria particularly detecting dangerous patterns. In many cases the HCP app may be configured such that an HCP can break a desired change into smaller steps that tend to work best for patients, and encourage positive reinforcement loops.

In one implementation, patients may be enabled to learn which variables impact their health by performing daily tasks and learning, from the app, e.g., app 403, how their body reacts to different activities. In this way, patients may gain a greater understanding of cause-and-effect, removing some of the guesswork of the treatment of their disease. For example, an exemplary task may be to eat their favorite meal at their favorite restaurant, or to drink orange juice at breakfast, or to take a 30 minute walk. The patient app may provide a summary of how these activities impacted their health, at least from the standpoint of glycemic response. Where such sensors are also available, the patient may learn how such activities impacted their blood sugar levels, cholesterol levels, blood pressure, and so on. The patient app may also be configured to remind the patient to take medications, as well as what medications to take. Smart phone reminders and notifications may also be set for vitamin intake, blood pressure measurements, and so on.

The app may be configured to allow the user to choose goals that matter to them, and provide education, action, and milestones customized to that goal or challenge. For example, the app may be configured such that the user can enter goals they wish to master in a certain priority order. Challenges may include, e.g., "I want to wear my favorite high school jeans again", or "I want to make healthy eating choices near work.". A challenge may last for, e.g., two weeks. Once accomplished, the patient may move on to the next step they have selected in the priority order. The app may provide a daily progress report that can be shared with others. The app may also show others who have succeeded and those who have struggled with the same goal. For example, the app may indicate that "The goal you selected was also selected by 85% of other people your age. They reported that portion control and exercise were the secret to their success."

In one exemplary implementation, a certified diabetic educator (CDE) may assist a new patient using the following steps. The CDE may inquire as to whether the patient has a smart phone, and if they do, the CDE may assist the patient in downloading the patient app to the smart phone. The CDE may then insert the sensor into the abdomen of the patient, and may then snap the transmitter into the sensor pod. At this point the app could be set up. Various user accounts could be set up, and such may include appropriate disclosures and verifications to ensure compliance with HIPAA and other legal requirements. Patient information may be entered, including their name, email address, username, password, and password confirmation. The patient may then be asked or prompted to read and comply with various safety statements, e.g., to not wear the device while undergoing an MRI, and so forth. The patient may then confirm their understanding and agreement with the various statements.

The CDE may then set up the patient's glucose thresholds, and thus set up a target range for the patient. The CDE may explain that when the patient's glucose concentration rises above or below the thresholds, a notification will be provided on their smart phone. The CDE may then assist the patient in pairing the sensor and transmitter to the app. Various ways of performing such pairing have been described above. The CDE may then explain to the patient how the same may mark events such as eating carbs or performing physical activity. Such event entry techniques are then provided to the patient. The CDE may then provide a booklet or other information about how the user can obtain additional information, if needed or desired. The CDE may explain to the patient that the sensor has been confirmed and verified to be working, e.g., receiving an appropriate number of counts, but that usable glucose concentration data may not be available for a couple of hours.

Following the conclusion of the sensor session, e.g., 14 days later, a notification may appear on the user's phone such as "Congratulations, your session is over. Swipe for sensor removal instructions." If the data has been uploaded during the session, the patient may throw away the sensor and transmitter in an appropriate way. If not, the user can keep the same, or just the transmitter, and provide the same to the doctor for data extraction at the next visit. Upon review of the data using the HCP app, the doctor can provide various insights and suggestions for the patient.

In some of the examples discussed above the patient sets up the user account and associates it with the app on the patient's mobile device. In other examples, such as shown in Fig. 8, for example, the HCP assists the patient in this task. In one alternative implementation, the user account may be set up on behalf of the patient before the patient is provided with the kit that includes the sensor, transmitter and applicator. In one particular implementation, the kit may also include a mobile device on which the app has been pre-installed for the patient (e.g., Android phones, iPhones, etc.). In another implementation the patient may use his or her own mobile device on which the app may be installed.

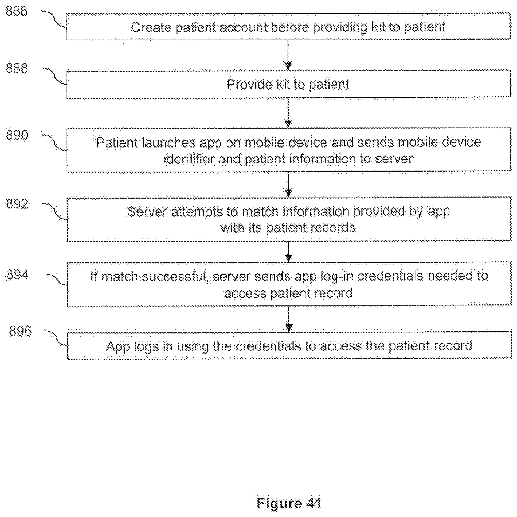

Fig. 41 shows one example of a method in which a patient's user account is set up on a server so that the patient is able to automatically log-in using the app on their mobile device while only entering a minimal amount of information.

In step 886, prior to providing the kit to the patient, the patient account is set up on the patient's behalf in a database that is maintained on a server. Among other things that are provisioned in the patient account such as the patient name, data of birth (DOB), and so on, the patient account is provisioned with a unique identifier of the mobile device that is included with the kit that is to be provided to the patient. For example, the identifier may be the international Mobile Equipment Identity (IMEI) of the mobile device. If the mobile device that is used is provided by the user and is not part of the kit, a different mobile device identifier may be assigned and used as the unique identifier.

In step 888, the patient is provided with the kit, including the mobile device. In step 890, the user launches the app, which establishes communication with the server and automatically sends the unique identifier of the mobile device. The app will also send patient information that has also been pre-provisioned in the patient record, which is used to associate the patient with the mobile device. This information may include, for example, one or more of the following: the patient's email address, DOB, phone number, and so on.

In step 892 the server attempts to match the unique identifier of the mobile device and the patient information to a patient record that is stored in its database. If the match is successful, then in step 894 the server sends the app the credentials needed to log in or otherwise access the matching patient record in the database. The app then logs in using the credentials to access the patient record in step 896.

The automatic log-in procedure described above has been illustrated for a new patient for which a user record needs to be created. The procedure may be also be used for existing patients. For instance, if in step 888 instead of providing a kit to a new patient, an existing patient is provided with a new transmitter, then in step 886 instead of creating a new patient account, the identifier of the new transmitter will be entered into the patient's existing record. Thus, in this way the process shown in Fig. 41 allows both new and existing patients to automatically log-in and access their patient account record with minimal effort.

It should be noted that the functionality of the server employed in the automatic log-in procedure discussed above in some cases may be distributed among multiple servers which may or may not be controlled by the same entity. For instance, in one particular example one or more of the servers may be controlled and operated by a device manufacturer and one or more additional servers may be controlled and operated by a database provider.

For ease of explanation and illustration, in some instances the detailed description describes exemplary systems and methods in terms of a continuous glucose monitoring environment; however it should be understood that the scope of the invention is not limited to that particular environment, and that one skilled in the art will appreciate that the systems and methods described herein can be embodied in various forms. Accordingly any structural and/or functional details disclosed herein are not to be interpreted as limiting the systems and methods, but rather are provided as attributes of a representative embodiment and/or arrangement for teaching one skilled in the art one or more ways to implement the systems and methods, which may be advantageous in other contexts.

For example, and without limitation, described monitoring systems and methods may include sensors that measure the concentration of one or more analytes (for instance glucose, lactate, potassium, pH, cholesterol, isoprene, and/or hemoglobin) and/or other blood or bodily fluid constituents of or relevant to a host and/or another party.

By way of example, and without limitation, monitoring system and method embodiments described herein may include finger-stick blood sampling, blood analyte test strips, non-invasive sensors, wearable monitors (e.g. smart bracelets, smart watches, smart rings, smart necklaces or pendants, workout monitors, fitness monitors, health and/or medical monitors, clip-on monitors, and the like), adhesive sensors, smart textiles and/or clothing incorporating sensors, shoe inserts and/or insoles that include sensors, transdermal (i.e. transcutaneous) sensors, and/or swallowed, inhaled or implantable sensors.

In some embodiments, and without limitation, monitoring systems and methods may comprise other sensors instead of or in additional to the sensors described herein, such as inertial measurement units including accelerometers, gyroscopes, magnetometers and/or barometers; motion, altitude, position, and/or location sensors; biometric sensors; optical sensors including for instance optical heart rate monitors, photoplethysmogram (PPG)/pulse oximeters, fluorescence monitors, and cameras; wearable electrodes; electrocardiogram (EKG or ECG), electroencephalography (EEG), and/or electroinyography (EMG) sensors; chemical sensors; flexible sensors for instance for measuring stretch, displacement, pressure, weight, or impact; galvanometric sensors, capacitive sensors, electric field sensors, temperature/thermal sensors, microphones, vibration sensors, ultrasound sensors, piezoelectric/piezoresistive sensors, and/or transducers for measuring information of or relevant to a host and/or another party.

In this document, the terms "computer program medium" and "computer usable medium" and "computer readable medium", as well as variations thereof, are used to generally refer to transitory or non-transitory media such as, for example, main memory, storage unit interface, removable storage media, and/or channel. These and other various forms of computer program media or computer usable/readable media may be involved in carrying one or more sequences of one or more instructions to a processing device for execution. Such instructions embodied on the medium, may generally be referred to as "computer program code" or a "computer program product" or "instructions" (which may be grouped in the form of computer programs or other groupings). When executed, such instructions may enable the computing module or a processor thereof or connected thereto to perform features or functions of the present disclosure as discussed herein.

Various embodiments have been described with reference to specific example features thereof. It will, however, be evident that various modifications and changes may be made thereto without departing from the broader spirit and scope of the various embodiments as set forth in the appended claims. The specification and figures are, accordingly, to be regarded in an illustrative rather than a restrictive sense.

Although described above in terms of various example embodiments and implementations, it should be understood that the various features, aspects and functionality described in one or more of the individual embodiments are not limited in their applicability to the particular embodiment with which they are described, but instead may be applied, alone or in various combinations, to one or more of the other embodiments of the present application, whether or not such embodiments are described and whether or not such features are presented as being a part of a described embodiment. Thus, the breadth and scope of the present application should not be limited by any of the above-described example embodiments.

Terms and phrases used in the present application, and variations thereof, unless otherwise expressly stated, should be construed as open ended as opposed to limiting. As examples of the foregoing: the term "including" should be read as meaning "including, without limitation" or the like; the term "example" is used to provide illustrative instances of the item in discussion, not an exhaustive or limiting list thereof, the terms "a" or "an" should be read as meaning "at least one," "one or more" or the like; and adjectives such as "conventional," "traditional," "normal," "standard," "known" and terms of similar meaning should not be construed as limiting the item described to a given time period or to an item available as of a given time, but instead should be read to encompass conventional, traditional, normal, or standard technologies that may be available or known now or at any time in the future. Likewise, where this document refers to technologies that would be apparent or known to one of ordinary skill in the art, such technologies encompass those apparent or known to the skilled artisan now or at any time in the future.

The presence of broadening words and phrases such as "one or more," "at least," "but not limited to" or other like phrases in some instances shall not be read to mean that the narrower case is intended or required in instances where such broadening phrases may be absent. The use of the term "module" does not imply that the components or functionality described or claimed as part of the module are all configured in a common package. Indeed, any or all of the various components of a module, whether control logic or other components, may be combined in a single package or separately maintained and may further be distributed in multiple groupings or packages or across multiple locations.

All numbers expressing quantities of ingredients, reaction conditions, and so forth used in the specification are to be understood as being modified in all instances by the term 'about.' Accordingly, unless indicated to the contrary, the numerical parameters set forth herein are approximations that may vary depending upon the desired properties sought to be obtained. At the very least, and not as an attempt to limit the application of the doctrine of equivalents to the scope of any claims in any application claiming priority to the present application, each numerical parameter should be construed in light of the number of significant digits and ordinary rounding approaches.

All references cited herein are incorporated herein by reference in their entirety. To the extent publications and patents or patent applications incorporated by reference contradict the disclosure contained in the specification, the specification is intended to supersede and/or take precedence over any such contradictory material.

Unless otherwise defined, all terms (including technical and scientific terms) are to be given their ordinary and customary meaning to a person of ordinary skill in the art, and are not to be limited to a special or customized meaning unless expressly so defined herein. It should be noted that the use of particular terminology when describing certain features or aspects of the disclosure should not be taken to imply that the terminology is being re-defined herein to be restricted to include any specific characteristics of the features or aspects of the disclosure with which that terminology is associated.

Additionally, the various embodiments set forth herein are described in terms of example block diagrams, flow charts, and other illustrations. As will become apparent to one of ordinary skill in the art after reading this document, the illustrated embodiments and their various alternatives may be implemented without confinement to the illustrated examples. For example, block diagrams and their accompanying description should not be construed as mandating a particular architecture or configuration.

Further description is found in the following clauses:
1. A continuous glucose monitoring device configured for use by a Health Care Professional (HCP), comprising:
   a housing;
   a circuit configured to receive a signal from a transmitter coupled to an indwelling glucose sensor,
   a calibration module configured to convert the received signal into clinical units;
   a user interface configured to display a measured glucose concentration in the clinical units;
   wherein the user interface is further configured to receive input data about a patient level; and
   wherein the input data about the patient level causes the device to operate in a mode based at least in part on one or more patient characteristics..
2. The device of clause 1, wherein the one or more patient characteristics includes a user's technical skill level.
3. The device of any of the previous clauses, wherein the one or more patient characteristics includes a user's type of diabetes.
4. The device of clause 3, wherein the user interface is further configured to prompt an HCP to enter data about whether the user is a T-1 diabetic, a T-2 diabetic, or prediabetic.
5. The device of any of the previous clauses, further comprising a memory to store glucose concentration values in clinical units and further configured to store the input data.
6. The device of clause 5, further comprising an output circuit configured to transmit the stored glucose concentration values.
7. The device of clause 6, wherein the transmission is configured to occur over a duration of less than five seconds.
8. The device of clause 6, wherein the transmission is configured to occur using near field communications or Bluetooth low-energy.
9. The device of any of the previous clauses, wherein the transmitter is configured to store measured glucose concentration values.
10. The device of clause 9, further comprising an output circuit configured to transmit the stored glucose concentration values.
11. The device of clause 10, wherein the transmission is configured to occur using near field communications or Bluetooth low-energy.
12. A method of configuring a continuous glucose monitoring device, comprising:
   displaying a user interface on an HCP device;
   displaying a prompt on the user interface for an HCP to enter data about a patient; and
   causing a continuous glucose monitoring device, including an indwelling sensor and a signally-coupled transmitter, the transmitter in signal communication with the HCP device, to operate in a mode based on the entered data.
13. The method of clause 12, wherein the data is about whether a patient is a T-1 diabetic, a T-2 diabetic, or is prediabetic.
14. The method of any of clauses 12 - 13, wherein the data is about a user's technical skill level.
15. The method of any of clauses 12 - 14, further comprising storing glucose concentration values in clinical units and the input data.
16. The method of clause 15, further comprising transmitting the stored glucose concentration values from the transmitter to the HCP device upon receipt of or triggering by an interrogation signal.
17. The method of clause 16, wherein the interrogation signal is received by the transmitter from a near field communications device or a Bluetooth low-energy device coupled to the HCP device.
18. The method of any of clauses 12 - 17, wherein the continuous glucose monitoring device is configured to download an app, the app configured to control the continuous glucose monitoring.
19. The method of clause 18, wherein the mode is a blinded mode, such that the app running on the continuous glucose monitoring device is configured to receive and store but not display glucose concentration data.
20. The method of clause 19, wherein the app is further configured to receive entered data corresponding to event data, where in the event data corresponds to medication data, meal data, or exercise data.
21. The method of clause 18, wherein the mode is an unblinded mode, such that the continuous glucose monitoring device is configured to receive and store and display glucose concentration data.
22. The method of clause 18, wherein the mode is configured to begin in a blinded mode and to switch to an unblinded mode at a predetermined time after a sensor session has begun.
23. The method of clause 18, wherein the mode is configured to begin in a blinded mode and to switch to an unblinded mode upon the occurrence of a trigger event.
24. The method of clause 23, wherein the trigger event corresponds to a patient parameter meeting a predetermined threshold criterion.
25. The method of clause 18, wherein the mode is configured to begin in a blinded mode and to switch to an unblinded mode upon the receiving of a trigger signal from an external device.
26. The method of any of clauses 12 - 25, wherein the patient data includes a transmitter serial number, the transmitter serial number having a plurality of extensions selectable by the HCP, the extension selected to be appropriate for the patient, wherein the serial number extension identifies the mode in which the continuous glucose monitoring device is to operate.
27. The method of clause 18, wherein the mode is a real-time blinded mode, such that the app running on the continuous glucose monitoring device is configured to receive and store but not display real time glucose concentration data, but is configured to display non-real-time historic glucose concentration data.
28. The method of any of clauses 12 - 27, further comprising operating a diagnostic application on the HCP device, the diagnostic application allowing HCP's to view and set CGM parameters without changing the course of therapy.
29. The method of clause 28, wherein the parameters include the sensor time remaining, sensor status, and current time in the session,
30. The method of any of clauses 12 - 29, wherein the mode is a blinded mode, and wherein the transmitter is configured to store data measured by the indwelling sensor.
31. A method of configuring a continuous glucose monitoring device for use by a patient, the configuring performed by an HCP, comprising:
   establishing a communication session associated with an HCP account between an HCP client device and a server;
   on a user interface associated with the HCP client device, prompting an HCP to enter patient data;
   also on the user interface associated with the client device, prompting an HCP to enter identifying data corresponding to a transmitter and/or sensor, the transmitter and/or sensor associated with the continuous glucose monitoring device;
   receiving the entered patient data and the sensor identifying data, and transmitting the entered patient data and the identifying data to the server for storage and association with a patient account.
32. The method of clause 31, wherein the identitying data is identifying data about a transmitter.
33. The method of any of clauses 3 1 - 32, further comprising receiving an output from the server in response to the transmitting entered patient data and the identifying data to the server, wherein the received output includes a code configured for use by a patient smart device to download an app for use in continuous glucose monitoring.
34. The method of clause 33, wherein the code is received by way of email or text.
35. The method of any of clauses 31 - 34, further comprising transmitting set up information to the continuous glucose monitoring device.
36. The method of clause 35, wherein the transmitting occurs by way of near field communications or Bluetooth low-energy.
37. A method of configuring a continuous glucose monitoring device for use by a patient, the configuring performed by an HCP, comprising:
   establishing a first communication session associated with an HCP account between an HCP client device and a server;
   on a user interface associated with the HCP client device, prompting an HCP to enter patient data;
   establishing a second communication session between the HCP client device and a transmitter associated with the glucose monitoring device, whereby the transmitter and/or a sensor associated with the continuous glucose monitoring device may be identified to the HCP client device;
   receiving the entered patient data and the identifying data, and transmitting the entered patient data and the identifying data to the server for storage and association with a patient account.
38. The method of clause 37, wherein the second communication session transmits transmitter identification data to the HCP client device.
39. The method of any of clauses 37 - 38, further comprising receiving an output from the server in response to the transmitting entered patient data and the sensor identifying data to the server, wherein the received output includes a code configured for use by a patient smart device to download an app for use in continuous glucose monitoring.
40. The method of clause 39, wherein the code is received by way of email or text.
41. The method of any of clauses 37 - 40, further comprising transmitting set up information to the continuous glucose monitoring device.
42. The method of clause 41, wherein the transmitting occurs by way of near field communications or Bluetooth low-energy.
43. A reader device configured for use by an HCP, comprising:
   a housing;
   a first circuit configured to receive a first signal from a transmitter associated with an indwelling glucose sensor;
   a second circuit configured to transmit a second signal to a computing environment.
44. The device of clause 43, wherein the circuit configured to receive a first signal is configured to receive the first signal using a wired or wireless communications protocol.
45. The device of clause 44, wherein the circuit configured to receive a first signal is activated by an interrogation signal from the transmitter.
46. The device of clause 44, wherein the circuit configured to receive a first signal activates the transmitter using an interrogation signal.
47. The device of clause 46, wherein following activation, the circuit extracts stored glucose concentration data from the transmitter.
48. The device of clause 44, wherein the communications protocol is wireless and is selected from the group consisting of: Bluetooth low-energy or near field communications.
49. The device of any of clauses 43 - 48, further comprising a third circuit configured to measure one or more operating parameters of a transmitter and to provide an output based on the measured one or more operating parameters, whereby a status of operation of the transmitter may be determined.
50. A reader device configured for use by an HCP, the reader device configured to determine proper functioning of a transmitter associated with a continuous glucose monitor, comprising a circuit configured to measure one or more operating parameters of a transmitter and to provide an output based on the measured one or more operating parameters, whereby a status of operation of the transmitter is determined.
51. A method of determining proper startup of a transmitter, the transmitter associated with a continuous glucose monitor, comprising:
   on a transmitter, detecting insertion of a sensor;
   upon the detecting, causing the transmitter to transition from an inactive state to an active state, wherein in the active state the transmitter transmits signals encoded with data corresponding to readings received from the sensor.
52. The method of clause 51, wherein in the active state the transmitter transmits signals using Bluetooth low energy.
53. The method of any of clauses 51 - 52, further comprising receiving the transmitted signals on an HCP device.
54. The method of any of clauses 51 - 53, further comprising receiving the transmitted signals on a patient device.
55. The method of clause 54, wherein the patient device is a smart phone or a smart watch.
56. The method of clause 55, further comprising, upon the receipt of an acknowledgment signal by the transmitter from the patient device, transmitting a signal from the transmitter to an HCP device, whereby the HCP device is provided verification that a patient device is properly working with a patient sensor and transmitter.
57. The method of clause 55, further comprising collecting data from the transmitter over a duration of time from the transmitter as measured by the sensor.
58. The method of clause 57, wherein the extracting includes interrogating the transmitter using the HCP device.
59. The method of clause 58, wherein the interrogating is performed using near field communications or Bluetooth low-energy.
60. The method of any of clauses 51 - 59, further comprising transmitting a signal from the transmitter, the signal encoded with data indicating that sensor insertion has occurred.
61. a method of activating a transmitter in a rapid fashion, the transmitter having been stored for a duration of time, the transmitter configured for physical engagement with an indwelling glucose sensor, the transmitter configured for transmitting signals representative of measured glucose concentration values to a mobile device, comprising:
   transmitting a wake-up command from an HCP device to the transmitter,
   wherein the wake-up command causes the transmitter to transition from an inactive state to an active state.
62. The method of clause 61, wherein the transmitting a wake-up command is performed using near field communications or Bluetooth low-energy.
63. The method of any of clause 61 - 62, wherein the transmitting a wake-up command is performed by communicating a signal to a wake-up pin on a processor at least in part operating the transmitter.
64. The method of any of clause 61 - 63, wherein the transmitting a wake-up command is performed in response to the detection of a signal measured by the indwelling glucose sensor.
65. A method of activating a transmitter in a rapid fashion, the transmitter having been stored for a duration of time, the transmitter configured for physical engagement with an indwelling glucose sensor, the transmitter configured for transmitting signals representative of measured glucose concentration values to a mobile device, comprising:
   on the transmitter, detecting a signal from a connected sensor;
   if the detected signal is determined to have an amplitude that exceeds a predetermined threshold, causing the transmitter to transition from an inactive state to an active state.
66. A method of activating a transmitter in a rapid fashion, the transmitter having been stored for a duration of time, the transmitter configured for physical engagement with an indwelling glucose sensor, the transmitter configured for transmitting signals representative of measured glucose concentration values to a mobile device, comprising:
   causing a transmitter to be activated on a periodic basis, the transmitter when activated configured to receive signals from a glucose sensor;
   such that if the transmitter is activated and does not receive signals from a glucose sensor within a predetermined period of time, the transmitter is caused to be inactivated; and
   such that if the transmitter is activated and does receive signals from a glucose sensor within a predetermined period of time, the transmitter is caused to be permanently activated, whereby the transmitter may be periodically woken up to determine if the connection has been made to the indwelling glucose sensor.
67. The method of clause 65, wherein periodic basis is between every five minutes and every 15 minutes.
68. A method of activating a transmitter in a rapid fashion, the transmitter configured for physical engagement with an indwelling glucose sensor, the transmitter configured for transmitting signals representative of measured glucose concentration values to a mobile device, comprising, upon activation of a transmitter, where a transmitter is coupled to a sensor and adhered to a patient by a patch, the transmitter receiving measurement signals from the sensor, the activation causing the transmitter to emit signals representative of and based on the measurement signals, rendering an indication of the activation on the transmitter, the sensor, or the patch, whereby a user may be notified of the activation without having to use other devices.
69. The method of clause 67, wherein the rendered indication is in visual or audible form.
70. A method of activating a transmitter in a rapid fashion, the transmitter configured for physical engagement with an indwelling glucose sensor, the transmitter configured for transmitting signals representative of measured glucose concentration values to a mobile device, comprising, upon activation of a transmitter, where a transmitter is coupled to a sensor and adhered to a patient by a patch, the transmitter receiving measurement signals from the sensor, the activation causing the transmitter to emit signals representative of and based on the measurement signals, transmitting a signal from the transmitter to an external device, the external device rendering an indication of the activation, whereby a user may be notified of the activation.
71. A method of activating a CGM system in a rapid fashion, the CGM system including a transmitter configured for physical engagement with an indwelling glucose sensor, the transmitter configured for transmitting signals representative of measured glucose concentration values to a patient mobile device, comprising:
   upon a wireless engagement of a patient mobile device and a transmitter, causing an app to download to the patient mobile device; and
   further upon the wireless engagement, and the download of the app, establishing a communication session between the patient mobile device and a server, the communication session associated with a user account.
72. The method of clause 71, wherein the wireless engagement includes an engagement using near field communications or Bluetooth low-energy.
73. The method of any of clauses 71 - 72, wherein the app is downloaded to the patient mobile device from the transmitter.
74. The method of any of clauses 71 - 73, wherein the app is downloaded to the patient mobile device from a server.
75. The method of any of clauses 71 74, further comprising causing the transmitter to begin transmitting signals from the sensor, the signals indicative of measured glucose values measured by the sensor.
76. A system for activating a transmitter in a rapid fashion, the transmitter configured for physical engagement with an indwelling glucose sensor, the transmitter configured for transmitting signals representative of measured glucose concentration values to a mobile device, comprising:
   an applicator configured to install an indwelling sensor;
   wherein the applicator is further configured to install a transmitter in physical engagement with the indwelling sensor, wherein the transmitter has a switch which when activated causes the transmitter to enter an active state, wherein in the active state the transmitter receives signals from the sensor and transmits signals representative of measured glucose concentration values to a mobile device; and
   such that the switch is configured to be activated when the transmitter is caused to physically engage with the indwelling sensor.
77. A transmitter configured to be activated in a rapid fashion, the transmitter further configured for physical engagement with an indwelling glucose sensor, the transmitter configured for transmitting signals representative of measured glucose concentration values to a mobile device, comprising:
   a housing, the housing including means to physically engage a glucose sensor, the glucose sensor configured to at least partially indwell within a patient;
   a light sensor disposed within the housing, the light sensor optically exposed to an exterior of the housing through a window;
   a cover applied to the window, the cover configured to block exposure of the light sensor to light prior to use of the transmitter,
   such that, before use, a user removes the cover to expose the light sensor to light, the light sensor when activated causing the transmitter to enter an active state, whereby the transmitter may be woken up by removal of the cover.
78. The transmitter of clause 77, wherein the cover adheres to the window.
79. A transmitter configured for physical engagement with an indwelling glucose sensor, the transmitter configured for transmitting signals representative of measured glucose concentration values to a mobile device, comprising:
   a housing, the housing including means to physically engage a glucose sensor, the glucose sensor configured to at least partially indwell within a patient;
   a circuit configured to receive timestamp information from an external source; and
   a memory configured to store the timestamp information from the external source.
80. The transmitter of clause 79, wherein the memory is further configured to associate the timestamp information with one or more received data packets, the received data packets associated with signals from the sensor,
81. The transmitter of any of clauses 79 - 80, wherein the circuit is a near field communication circuit or a Bluetooth low-energy circuit.
82. The transmitter of clause 81, wherein the circuit is a Bluetooth low-energy circuit, and wherein the circuit is configured to periodically poll nearby Bluetooth devices for timestamp information.
83. The transmitter of clause 81, wherein the circuit is configured to receive timestamp information from an HCP device.
84. The transmitter of clause 81, wherein the circuit is configured to receive timestamp information from a patient mobile device.
85. The transmitter of any of clauses 79 --- 84, further comprising a processor in signal communication with the memory, the processor configured to compensate for time drifts or time lags.
86. The transmitter of clause 85, wherein the processor is configured to compensate for time drifts or time lags by causing a periodic or occasional synchronization.
87. A kit for pairing a patient mobile device including a continuous glucose monitoring app with a transmitter, the transmitter configured for transmitting signals representative of measured glucose concentration values to a mobile device, comprising:
   a transmitter, the transmitter including means to physically engage a glucose sensor; and
   an identifying component including flexible electronics, the identifying component configured to be scanned by a patient mobile device, the patient mobile device receiving identification information about the transmitter, the identification information stored on the flexible electronics;
   such that upon receipt of the identification information the patient mobile device is configured to be paired with the transmitter, wherein the pairing allows the patient mobile device to receive signals transmitted by the transmitter, the signals representative of measured glucose concentration values.
88. The kit of clause 87, wherein the identifying component comprises a sticker.
89. The kit of any of clauses 87 - 88, wherein the identifying information includes calibration information.
90. The kit of any of clauses 87 - 89, wherein the pairing is further between the glucose monitoring app and the transmitter.
91. The kit of clause 90, wherein the pairing is performed on the basis of identified RSSI signal strength.
92. The kit of any of clauses 87 - 91, wherein the identifying component is integrated as part of a sensor adhesive patch.
93. The kit of any of clauses 87 - 92, wherein the app is configured to verify that the sensor is within a proper operating range by determining whether received counts are within a predetermined range over a predetermined period of time.
94. A method for pairing a patient mobile device including a continuous glucose monitoring app with a transmitter, the transmitter configured for transmitting signals representative of measured glucose concentration values to a mobile device, comprising:
   receiving input on a user interface of a mobile device a desire an indication that the mobile device should be paired with a transmitter;
   detecting on an accelerometer within the mobile device a motion artifact;
   upon the detecting, transmitting a signal to cause the transmitter to be placed in a pairing mode; pairing the transmitter with the mobile device.
95. The method of clause 94, further comprising indicating on the user-interface a desired motion artifact.
96. The method of any of clauses 94 - 95, wherein the motion artifact comprises a predetermined number of taps.
97. The method of any of clauses 94 - 96, wherein the motion artifact comprises a shaking motion for a predetermined threshold period of time.
98. A method for pairing a patient mobile device including a continuous glucose monitoring app with a transmitter, the transmitter configured for transmitting signals representative of measured glucose concentration values to a mobile device, comprising:
   receiving input on a user interface of a mobile device an indication that the mobile device is to be paired with a transmitter;
   detecting a signal from the transmitter;
   determining if an RSSI measurement of the detected signal exceeds a predetermined threshold; and
   causing a pairing to occur between the mobile device and the transmitter based on the determination that detected signal exceeded the predetermined threshold.
99. The method of clause 98, wherein the determining step includes confirming an identity or availability of a user-identified transmitter based on selection criteria including ongoing detection of at least one of information related to the strength of the signal detected from the selected transmitter for a predetermined period of time and information related to the quality of the signal detected from the selected transmitter for a predetermined period of time,
100. A reader device configured for use by an HCP, the reader device configured to set up a transmitter associated with a continuous glucose monitor, comprising a circuit configured to receive and transmit one or more operating parameters of a transmitter for display on a user interface of an HCP device, the reader device further configured to receive modified transmitter parameters from the user interface and to store the modified transmitter parameters on the transmitter.
101. A method of activating a transmitter, the transmitter having been employed in a sensor session, the transmitter configured for physical engagement with an indwelling glucose sensor, the transmitter configured for transmitting signals representative of measured glucose concentration values to a mobile device, the transmitter configured to, following a predetermined duration of a sensor session, transition from an active state to an inactive state, comprising:
   transmitting a wake-up command from an external device to the transmitter,
   wherein the wake-up command causes the transmitter to transition from an inactive state to an active state.
102. The method of clause 101, wherein the transmitting a wake-up command is performed using near field communications or Bluetooth low-energy.
103. The method of any of clauses 101 - 102, further comprising downloading data stored in the transmitter to the external device.
104. The method of clause 103, wherein the wake-up command is transmitted using a near field communications protocol.
105. The method of clause 104, wherein the downloading is performed using a near field communications protocol.
106. The method of clause 104, wherein the downloading is performed using a Bluetooth low-energy protocol.
107. The method of clause 103, wherein the external device is the HCP device.
108. The method of clause 107, wherein the HCP device is an HCP reader or controller or an HCP smart phone.
109. The method of clause 103, wherein the external device is a patient device.
110. The method of clause 109, wherein the patient device is a patient's smart phone.
111. A method of reducing noise in the signal transmissions between a transmitter and a mobile device, the transmitter configured for physical engagement with an indwelling glucose sensor, the transmitter configured for transmitting signals representative of measured glucose concentration values to the mobile device using a near field communications protocol, comprising:
   upon detection of a signal with energy greater than a predetermined threshold level on a near field communication circuit within a transmitter, setting a first flag and associating the first flag with corresponding data;
   on the detection of near field communications signaling between the transmitter and an external device, setting a second flag and associating the second flag with corresponding data;
   transmitting glucose concentration data from the transmitter to an external device;
   on the external device, storing the transmitted data;
   in calculations involving the transmitted data, adjusting, compensating for, or disregarding data for which both the first and second flags are set.
112. A method of reducing noise in the signal transmissions between a transmitter and a mobile device, the transmitter configured for physical engagement with an indwelling glucose sensor, the transmitter configured for transmitting signals representative of measured glucose concentration values to the mobile device using a near field communications protocol, comprising:
   upon detection of a signal with energy greater than a predetermined threshold level on a near field communication circuit with then a transmitter, setting a first flag and associating the first flag with corresponding data;
   on the detection of near field communications signaling between the transmitter and an external device, setting a second flag and associating the second flag with corresponding data;
   transmitting glucose concentration data from the transmitter to an external device,
   on the external device, storing the transmitted data;
   in calculations involving the transmitted data, associating data for which both the first and second flags are set with a lower weighting than data for which neither flag is set.
113. A method of reducing noise in the signal transmissions between a transmitter and a mobile device, the transmitter configured for physical engagement with an indwelling glucose sensor, the transmitter configured for transmitting signals representative of measured glucose concentration values to the mobile device using a near field communications protocol, compri sing:
   upon detection of a signal with energy greater than a predetermined threshold level on a near field communication circuit with then a transmitter, setting a first flag and associating the first flag with corresponding data;
   on the detection of near field communications signaling between the transmitter and an external device, setting a second flag and associating the second flag with corresponding data;
   transmitting glucose concentration data from the transmitter to an external device;
   on the external device, storing the transmitted data;
   in calculations involving the transmitted data, disregarding data for which the first flag is set.
114. A transmitter configured for reducing noise in signal transmissions to a mobile device, comprising:
   a housing, the housing including means to physically engage a glucose sensor, the glucose sensor configured to at least partially indwell within a patient;
   a near field communication circuit disposed within the housing, the near field communication circuit configured for transmitting signals to an external device;
   a threshold detector coupled to the near field communication circuit, the threshold detector configured to detect a signal energy of energy captured by the near field communications circuit,
   such that if the threshold detector detects a signal of energy greater than a predetermined threshold, the threshold detector causes the near field communication circuit to become inactive.
115. The transmitter of clause 114, wherein the threshold detector causes an antenna associated with the near field communication circuit to be disabled.
116. A transmitter configured for reducing noise in signal transmissions to a mobile device, comprising:
   a housing, the housing including means to physically engage a glucose sensor, the glucose sensor configured to at least partially indwell within a patient;
   a wireless communication circuit disposed within the housing, the wireless communication circuit configured for transmitting signals to an external device;
   a detector coupled to the wireless communication circuit, the detector configured to detect if a signal corresponding to a wake-up command corresponding to a wake-up process is being captured by the wireless communications circuit,
   such that if the detector detects a wake-up signal, the detector causes the wireless communication circuit to become inactive following completion of the wake-up process.
117. The transmitter of clause 116, wherein the wireless communication circuit is a near field communications circuit.
118. A transmitter configured for enhanced battery life, the transmitter further configured for signal transmission to a mobile device, comprising:
   a housing, the housing including means to physically engage a glucose sensor, the glucose sensor configured to at least partially indwell within a patient;
   a wireless communication circuit disposed within the housing, the wireless communication circuit configured for transmitting signals to an external device;
   a battery, the battery configured to power the wireless communication circuit and to enable measurement by the glucose sensor;
   a power down circuit configured to cause the transmitter to transition from an active state to an inactive state following completion of a sensor session;
   a wake-up circuit configured to cause the transmitter to transition from an inactive state to an active state following completion of the sensor session such that data stored on the transmitter may be transmitted to the external device, the wake-up circuit in part activated by a wake-up pin, the wake-up circuit further configured to connect the battery to the wireless communications circuit in the active state, and such that the wake-up circuit is configured to, when transitioning to the inactive state, disable the wake-up pin.
119. A transmitter configured for enhanced battery life, the transmitter further configured for signal transmission to a mobile device, comprising:
   a housing, the housing including means to physically engage a glucose sensor, the glucose sensor configured to at least partially indwell within a patient;
   a wireless communication circuit disposed within the housing, the wireless communication circuit configured for transmitting signals to an external device;
   a battery, the battery configured to power the wireless communication circuit and to enable measurement by the glucose sensor;
   a power down circuit configured to cause the transmitter to transition from an active state to an inactive state following completion of a sensor session;
   a wake-up circuit configured to cause the transmitter to transition from an inactive state to an active state following completion of the sensor session such that data stored on the transmitter may be transmitted to the external device, the wake-up circuit in part activated by a wake-up pin, the wake-up circuit further configured to connect the battery to the wireless communications circuit in the active state, and such that the wake-up pin is hardened against EMI.
120. The transmitter of clause 1 19, wherein the hardening is by use of a strong pull up/down resistor.
121. The transmitter of clause 120, wherein the hardening is further by use of a capacitor.
122. The transmitter of any of clauses 119 - 121, wherein the hardening is by mechanically shorting the wake-up pin to a disabled polarity.
123. A transmitter configured for enhanced battery life, the transmitter further configured for signal transmission to a mobile device, comprising:
   a housing, the housing including means to physically engage a glucose sensor, the glucose sensor configured to at least partially indwell within a patient;
   a wireless communication circuit disposed within the housing, the wireless communication circuit configured for transmitting signals to an external device;
   a battery, the battery configured to power the wireless communication circuit and to enable measurement by the glucose sensor;
   a power down circuit configured to cause the transmitter to transition from an active state to an inactive state following completion of a sensor session;
   a wake-up circuit configured to cause the transmitter to transition from an inactive state to an active state following completion of the sensor session such that data stored on the transmitter may be transmitted to the external device, the wake-up circuit in part activated by a wake-up pin, the wake-up circuit further configured to connect the battery to the wireless communications circuit in the active state, and such that the wake-up circuit is configured to, when transitioning to the inactive state, disable the wake-up circuit.
124. A transmitter configured for enhanced data storage, the transmitter further configured for signal transmission to a mobile device, comprising:
   a housing, the housing including means to physically engage a glucose sensor, the glucose sensor configured to at least partially indwell within a patient;
   a wireless communication circuit disposed within the housing, the wireless communication circuit configured for transmitting signals to an external device;
   a battery, the battery configured to power the wireless communication circuit and to enable measurement by the glucose sensor;
   a memory, the memory configured to store data representative of glucose values measured by the glucose sensor; and
   a processor configured to perform data processing on data stored in the memory, wherein the processor is configured to periodically compress the data stored in the memory, such that the data occupies less memory than before the compressing.
125. The transmitter of clause 124, wherein the processor is configured to compress the data to a minimum number of data points necessary to accurately illustrate a glycemic exposure of the patient.
126. The transmitter of clause 125, wherein the data points include those corresponding to maximums, minimums, and inflection points, along with corresponding abscissa time values.
127. The transmitter of any of clauses 124 - 126, wherein the processor is configured to compress the data by eliminating data points representing stable values, where stable values are those within a range of stability.
128. The transmitter of any of clauses 124 - 127, wherein the processor is configured to compress the data using lossy or lossless compression techniques.
129. The transmitter of clause 128, wherein the lossy or lossless compression techniques include one or more selected from the group consisting of Lempel-Ziv compression, Huffman encoding, or algorithmic encoding.
130. The transmitter of any of clauses 124 - 129, wherein the processor is configured to compress the data by only storing data that change is more than a defined amount, wherein data in between such points is interpolated.
131. The transmitter of any of clauses 124 - 130, wherein the processor is configured to compress the data by removing artifacts.
132. A method of operating a transmitter for enhanced data storage, the transmitter configured for transmitting signals from an indwelling glucose sensor to a mobile device, comprising:
   receiving signals over time from an indwelling glucose sensor;
   storing data representing the received signals in a memory;
   performing data processing on the stored data, wherein the data processing includes periodically or aperiodically compressing the data stored in the memory, such that the data occupies less memory than before the compressing.
133. The method of clause 132, wherein the compressing compresses the data to a minimum number of data points necessary to accurately illustrate a glycemic exposure of the patient.
134. The method of clause 133, wherein the data points include those corresponding to maximums, minimums, and inflection points, along with corresponding abscissa time values.
135. The method of any of clauses 132 - 134, wherein the compressing compresses the data by eliminating data points representing stable values, where stable values are those within a range of stability.
136. The method of any of clauses 132 - 135, wherein the compressing compresses the data using lossy or lossless compression techniques.
137. The method of clause 136, wherein the lossy or lossless compression techniques include one or more selected from the group consisting of Lempel-Ziv compression, Huffman encoding, or algorithmic encoding,
138. The method of any of clauses 132 - 137, wherein the compressing compresses the data by only storing data that change is more than a defined amount, wherein data in between such points is interpolated.
139. The method of any of clauses 132 - 138, wherein the compressing compresses the data by removing artifacts.
140. A method of operating a transmitter for enhanced data accuracy, the transmitter configured for transmitting signals from an indwelling glucose sensor to a mobile device, comprising:
   receiving signals over time from an indwelling glucose sensor;
   storing data representing the received signals in a memory;
   performing data processing on the stored data, wherein the data processing includes periodically or aperiodically post-processing the data stored in the memory, such that the accuracy of the data, or accuracy of calculations based on the data, is enhanced.
141. The method of clause 140, wherein the periodically post -processing the data includes post
   -processing the data every 24 hours or every 48 hours.
142. The method of any of clauses 140 - 141, wherein the periodically post -processing the data includes periodically smoothing the data.
143. A method of operating a continuous glucose monitor, comprising:
   receiving signals at a transmitter from an indwelling glucose sensor;
   receiving external signals at the transmitter from an external sensor,
   wherein the sensor signals and the external signals are stored based on an absolute or relative time of receipt.
144. The method of clause 143, wherein the external sensor is an ambient noise sensor.
145. The method of clause 144, further comprising calculating a sleep or exercise event based on the signals from the noise sensor.
146. The method of any of clauses 143 - 145, wherein the external sensor is an accelerometer.
147. The method of clause 146, further comprising calculating a sleep or exercise event based on the signals from the accelerometer.
148. The method of any of clauses 143 - 147, wherein the external sensor is a GPS receiver.
149. The method of clause 148, further comprising calculating a sleep or exercise or meal event based on the signals from the GPS receiver.
150. A method of operating a continuous glucose monitor, comprising:
   receiving signals at a mobile device from a transmitter as measured by an indwelling glucose sensor;
   receiving external signals at the mobile device from an external sensor,
   storing the sensor signals and the external signals at the mobile device based on an absolute or relative time of receipt.
151. A transmitter configured for physical engagement with an indwelling glucose sensor, the transmitter configured for transmitting signals representative of measured glucose concentration values to a mobile device, comprising:
   a housing configured to physically engage a glucose sensor, the glucose sensor configured to at least partially indwell within a patient;
   a first communication circuit configured to communicate with the mobile device using a short-range wireless protocol;
   a second communication circuit configured to communicate with the mobile device using an encrypted wireless protocol; and
   a memory for storing information received from the glucose sensor.
152. The transmitter of clause 151, wherein the first communication circuit is a near field communication (NFC) circuit.
153. The transmitter of clause 152, wherein the second communication circuit is a Bluetooth low energy circuit.
154. The transmitter of clause 151, wherein the second communication circuit is a Bluetooth low energy circuit.
155. The transmitter of clause 151, wherein the mobile device is an HCP device.
156. The transmitter of clause 152, further comprising a feedback indicator that facilitates physical alignment by a user between an antenna of het NFC circuit and an antenna of the mobile device.
157. The transmitter of clause 156, wherein the feedback indicator includes an auditory, haptic or visual indicator.
158. A method for communication between a mobile device and a transmitter configured for physical engagement with a continuous glucose sensor, the transmitter configured for transmitting signals representative of measured glucose concentration values to the mobile device, comprising:
   receiving, at the transmitter, a data extraction command from the mobile device using a short-range wireless protocol;
   responsive to the data extraction command, causing the transmitter to be placed in a data extraction mode of operation;
   transmitting an advertising message in accordance with an encrypted wireless protocol to initiate a connection with the mobile device over the encrypted wireless protocol; and
   upon establishing a connection with the mobile device over the encrypted wireless protocol, transmitting estimated glucose values to the mobile device over the connection.
159. The method of clause 158, further comprising, prior to receiving the data extraction command, receiving, at the transmitter, a query command using the short-range wireless protocol and, responsive to the query command, transmitting an encrypted identifier of the transmitter using the short-range wireless protocol.
160. The method of clause 159, wherein the query command is issued after the glucose sensor is inserted in the patient.
161. The method of clause 160, wherein the query command is issued while in an HCP office.
162. The method of clause 160, wherein, further responsive to the query command, transmitting operational state information specifying one or more operational states of the transmitter.
163. The method of clause 162, wherein the operational state information is selected from the group consisting of a transmitter storage state, sensor verification state, an in-session state, and a session complete state.
164. The method of clause 158, wherein establishing the connection includes causing the transmitter to be authenticated by the mobile device,
165. The method of clause 158, wherein the advertising message includes information identifying the transmitter.
166. The method of clause 158, wherein transmitting the advertising message includes periodically transmitting advertising messages.
167. The method of clause 158, wherein transmitting the advertising message includes continuously transmitting advertising messages.
168. The method of clause 158, further comprising communicating only with the mobile device to which the connection is established while in the data extraction mode and not any other mobile device.
169. The method of clause 158, further comprising obtaining a decryption key to decrypt the encrypted identifier of the transmitter from a server over a communication network.
170. The method of clause 158, further comprising powering down the transmitter after transmission of the estimated glucose values is completed.
171. The method of clause 158, wherein transmitting the estimated glucose values includes transmitting the estimated glucose values within a specified period of time.
172. The method of clause 171, wherein the specified period of time is 15 seconds.
173. The method of clause 158, wherein the encrypted wireless protocol is a BLE protocol having at least two modes of operation and further comprising, prior to transmitting the advertising message, switching from a first BLE mode of operation to a data extraction mode of operation.
174. The method of clause 173, wherein the data extraction mode of operation has a greater rate at which advertising messages are transmitted in comparison to the first BLE mode of operation,
175. The method of clause 174, further comprising using different identifiers for the transmitter when operating in the first BLE mode and the data extraction mode.
176. The method of clause 162, wherein the operational state information is selected from the group consisting of a transmitter storage state indicating that the transmitter is still in packaging, a sensor verification state indicating that the transmitter is performing a sensor insertion verification operation, an in-session state indicating that the sensor is collecting data, and a session complete state indicating that a session is complete and that the data is available for downloading.
177. The method of clause 176, wherein the operational state information for the sensor verification state further includes information indicating a time remaining to complete sensor insertion verification and a confirmation of sensor verification.
178. The method of clause 177, wherein the operational state information for the sensor verification state further includes information indicating a number of sensor insertion verification attempts that have been performed.
179. The method of clause 176, wherein the operational state information further includes a duration of a current in-use session.
180. A continuous glucose monitoring device configured for use by a Health Care Professional (HCP), comprising:
   a housing;
   a first communication circuit configured to communicate with a continuous glucose monitoring device that includes a transmitter coupled to an indwelling glucose sensor using a short-range wireless protocol;
   a second communication circuit configured to communicate with the transmitter using an encrypted wireless protocol; and
   a user interface for entering an operational mode in which the continuous glucose monitoring device is to operate.
181. A method for pairing a patient mobile device including a continuous glucose monitoring app with a transmitter, the transmitter configured for transmitting signals representative of measured glucose concentration values to the patient mobile device, comprising:
   detecting insertion of the transmitter in a glucose sensor housing;
   responsive to the detecting, automatically causing the transmitter to transition from an inactive state to an active state in which the transmitter is able to transmit signals encoded with data corresponding to readings received from the sensor;
   broadcasting an advertisement message in accordance with a first wireless protocol;
   responsive to the broadcasting, opening an app on the patient mobile device;
   causing a security code to be entered on the patient mobile device to pair the transmitter with the patient mobile device using the first wireless protocol;
   pairing the transmitter with the patient mobile device using the first wireless protocol if the security code is correct; and
   responsive to the pairing, causing a sensor session to begin.
182. The method of clause 181, wherein the app opens automatically without user intervention in response to the broadcasting.
183. The method of clause 181, wherein causing the security code to be entered includes manual entry of the security code through a user interface of the patient mobile device.
184. The method of clause 181, wherein causing the security code to be entered includes receiving on the patient mobile device a signal that includes the security code, the signal being sent by the transmitter using a second wireless protocol.
185. The method of clause 184, wherein the second wireless protocol is NFC.
186. The method of clause 181, wherein the first wireless protocol is BLE.
187. The method of clause 185, wherein the first wireless protocol is BLE.
188. The method of clause 181, wherein the session begins after a sensor warmup period, and further comprising causing the app to indicate through a user interface on the patient mobile device approximately when the sensor session will begin after the warmup period.
189. a method of activating a transmitter in a rapid fashion, the transmitter having been stored for a duration of time, the transmitter configured for physical engagement with an indwelling glucose sensor, the transmitter configured for transmitting signals representative of measured glucose concentration values to a mobile device, comprising:
   on a transmitter that is stored in packaging, detecting a signal indicative of an acceleration event;
   if the detected signal is determined to have indicate that the acceleration event resulted from acceleration within a specified range, causing the transmitter to transition from an inactive state to an active state.
190. A method for communication between a mobile device and a transmitter configured for physical engagement with an indwelling glucose sensor, the transmitter configured for transmitting signals representative of measured glucose concentration values to the mobile device, comprising:
   receiving, at the transmitter, a data extraction command from the mobile device using a short-range wireless protocol;
   responsive to the data extraction command, causing the transmitter to be placed in a data extraction mode of operation;
   transmitting an advertising message in accordance with an encrypted wireless protocol to initiate a connection with the mobile device over the encrypted wireless protocol, and
   upon establishing a connection with the mobile device over the encrypted wireless protocol, transmitting operational state information specifying at least one operational state of the transmitter, the at least one operational state of the transmitter including a sensor session completion state indicating that a session is complete and the data is available for downloading.
191. The method of clause 190, further comprising, upon receiving the data extraction command, transmitting an encrypted identifier of the transmitter using the short-range wireless protocol.
192. The method of clause 191, wherein the query command is issued while in an HCP office.
193. The method of clause 190, wherein establishing the connection includes causing the transmitter to be authenticated by the mobile device.
194. The method of clause 190, wherein the advertising message includes information identifying the transmitter.
195. The method of clause 194, wherein transmitting the advertising message includes periodically transmitting advertising messages.
196. The method of clause 194, wherein transmitting the advertising message includes continuously transmitting advertising messages.
197. The method of clause 194, wherein the short-range wireless protocol is NFC and the encrypted wireless protocol is BLE.
198. A method for communication between an HCP reader and a transmitter configured for physical engagement with an indwelling glucose sensor, the transmitter configured for transmitting signals representative of measured glucose concentration values to the HCP reader, comprising:
   receiving, at the transmitter, a data extraction command from the HCP reader using a short-range wireless protocol, the data extraction command including an encrypted version of an identifier of the transmitter,
   responsive to the data extraction command, causing the transmitter to be placed in a data extraction mode of operation if the transmitter determines that the identifier included in the data extraction command is correct;
   transmitting an advertising message in accordance with an encrypted wireless protocol to initiate a connection with the HCP reader over the encrypted wireless protocol, the advertising message including information that causes the HCP reader to have priority of connection over other devices; and
   upon establishing a connection with the HCP reader over the encrypted wireless protocol, transmitting sensor data to the HCP reader over the connection.
199. The method of clause 198, wherein the encrypted wireless protocol is BLE.
200. The method of clause 198, wherein the information that causes the HCP reader to have priority of connection over other devices includes a BLE GAP address.
201. The method of clause 198, wherein the advertising message includes at least a portion of an identifier of the transmitter.
202. The method of clause 198, wherein transmitting the advertising message includes periodically transmitting advertising messages.
203. The method of clause 198, wherein transmitting the advertising message includes continuously transmitting advertising messages.
204. The method of clause 198, wherein the short-range wireless protocol is NFC.
205. The method of clause 199, wherein the sensor data transmitted to the HCP reader includes estimated glucose values.

## Claims

1. A method of configuring a continuous glucose monitoring device for use by a patient, the configuring performed by a Health Care Professional, HCP, comprising:
establishing a communication session associated with an HCP account between an HCP client device and a server;
on a user interface associated with the HCP client device, prompting an HCP to enter patient data;
on the user interface associated with the client device, prompting an HCP to enter identifying data corresponding to a transmitter and/or sensor, the transmitter and/or sensor associated with the continuous glucose monitoring device;
receiving the entered patient data and the sensor identifying data, and transmitting the entered patient data and the identifying data to the server for storage and association with a patient account.

2. The method of claim 1, wherein the identifying data is identifying data about a transmitter.

3. The method of any of claims 1- 2, further comprising receiving an output from the server in response to the transmitting entered patient data and the identifying data to the server, wherein the received output includes a code configured for use by a patient smart device to download an app for use in continuous glucose monitoring.

4. The method of claim 3, wherein the code is received by way of email or text.

5. The method of any of claims 1- 4, further comprising transmitting set up information to the continuous glucose monitoring device.

6. The method of claim 5, wherein the transmitting occurs by way of near field communications or Bluetooth low-energy.

7. A method of configuring a continuous glucose monitoring device for use by a patient, the configuring performed by a Health Care Provider, HCP, comprising:
establishing a first communication session associated with an HCP account between an HCP client device and a server;
on a user interface associated with the HCP client device, prompting an HCP to enter patient data;
establishing a second communication session between the HCP client device and a transmitter associated with the glucose monitoring device, whereby the transmitter and/or a sensor associated with the continuous glucose monitoring device may be identified to the HCP client device;
receiving the entered patient data and the identifying data, and transmitting the entered patient data and the identifying data to the server for storage and association with a patient account.

8. The method of claim 7, wherein the second communication session transmits transmitter identification data to the HCP client device.

9. The method of any of claims 7-8, further comprising receiving an output from the server in response to the transmitting entered patient data and the sensor identifying data to the server, wherein the received output includes a code configured for use by a patient smart device to download an app for use in continuous glucose monitoring.

10. The method of claim 9, wherein the code is received by way of email or text.

11. The method of any of claims 7 - 10, further comprising transmitting set up information to the continuous glucose monitoring device.

12. The method of claim 11, wherein the transmitting occurs by way of near field communications or Bluetooth low-energy.

13. A system for configuring a continuous glucose monitoring device for use by a patient, the configuring performed by a Health Care Professional, HCP, the system comprising an HCP client device having a user interface, a server, a transmitter and/or a sensor, the system configured to carry out the method of any of claims 1 to 6.

14. A system for configuring a continuous glucose monitoring device for use by a patient, the configuring performed by a Health Care Professional, HCP, the system comprising an HCP client device having a user interface, a server, a transmitter and/or a sensor, the system configured to carry out the method of any of claims 7 to 12.

15. One or more computer-readable media comprising instructions which, when executed by one or more processors, perform the method of any of claims 1 to 12.
